# EUROPEAN PATENT APPLICATION

(11) **EP 4 026 860 A1**
(43) Date of publication of application: **13.07.2022**
(21) Application number: 21216988.2
(22) Date of filing: 22.12.2021
(51) Int. Cl.: C08F 220/38, C08L 33/14, C08L 33/16

(54) **BIO-ELECTRODE COMPOSITION, BIO-ELECTRODE, AND METHOD FOR MANUFACTURING BIO-ELECTRODE**

(30) Priority: 06.01.2021 JP 2021001104
(71) Applicant: Shin-Etsu Chemical Co., Ltd., Tokyo 100-0005 (JP)
(72) Inventor: Ikeda, Joe, Tokyo, 100-0005 (JP); Iwabuchi, Motoaki, Niigata (JP); Hatakeyama, Jun, Niigata (JP)
(74) Representative: Wibbelmann, Jobst

(57) **Abstract**

A bio-electrode composition contains a polymer compound (A), which contains a repeating unit-a having a structure selected from the group consisting of salts of ammonium, sodium, potassium, and silver formed with any of fluorosulfonic acid, fluorosulfonimide, and N-carbonyl-fluorosulfonamide; and filler particles (B), which are one or more selected from the group consisting of silica particles, alumina particles, titania particles, and zirconia particles. Thus, the present invention provides: a bio-electrode composition capable of forming a living body contact layer for a bio-electrode which enables quick signal collection after attachment to skin and does not leave residue on the skin; a bio-electrode including a living body contact layer formed of the bio-electrode composition; and a method for manufacturing the bio-electrode.

## Description

### TECHNICAL FIELD

The present invention relates to: a bio-electrode that is used in contact with the skin of a living body and capable of detecting physical conditions such as heart rate by an electric signal transmitted from the skin; a method for manufacturing the bio-electrode; and a bio-electrode composition desirably used for a bio-electrode.

### BACKGROUND ART

A recent growing popularity of Internet of Things (IoT) has accelerated the development of wearable devices, such as watches and eye-glasses that allow for Internet access. Even in the fields of medicine and sports, wearable devices for constantly monitoring the user's physical state are increasingly demanded, and such technological development is expected to be further encouraged.

In the field of medicine, use of wearable devices has been examined for monitoring the state of human organs by sensing extremely weak current, such as an electrocardiogram which detects an electric signal to measure the motion of the heart. The electrocardiogram measurement is conducted by attaching an electrode coated with an electro-conductive paste to a body, but this is a single (not continuous), short-time measurement. On the other hand, development of the above medical wearable device is aimed at device for continuously monitoring the health condition for a few weeks. Accordingly, a bio-electrode used in a medical wearable device is required to make no changes in electric conductivity even in long-time use and cause no skin allergy. In addition to these, it is also required that a bio-electrode is light-weight and can be produced at low cost.

Medical wearable devices are classified into two types: a type which is directly attached to body and a type which is incorporated into clothes. As the type which is attached to a body, it has been proposed a bio-electrode using water-soluble gel containing water and electrolyte, which are materials of the foregoing electro-conductive paste (Patent Document 1). The water-soluble gel contains sodium, potassium, or calcium as the electrolyte in a water-soluble polymer for retaining water, and converts changes of ion concentration from skin into electricity. On the other hand, as the type which is incorporated into clothes, it has been proposed a means to use cloth in which an electro-conductive polymer such as PEDOT-PSS (poly-3,4-ethylenedioxythiophene-polystyrenesulfonate) or silver paste is incorporated into the fibers for electrodes (Patent Document 2).

However, the use of the hydrophilic gel containing water and electrolytes unfortunately brings about loss of electric conductivity due to water evaporation in drying process. Meanwhile, the use of a higher-ionization-tendency metal such as copper can cause some users to suffer from skin allergy. The use of an electro-conductive polymer such as PEDOT-PSS can also cause skin allergy due to the strong acidity of the electro-conductive polymer, and further cause peeling of the electro-conductive polymer from fibers during washing.

By taking advantage of excellent electric conductivity, the use of metal nanowire, carbon black, carbon nanotube, and the like as electrode materials has been examined (Patent Documents 3, 4, and 5). With higher contact probability among metal nanowires, the wires can conduct electricity even when added in small quantities. The metal nanowire, however, can cause skin allergies since they are thin material with sharp tips. The carbon nanotubes can stimulate (irritate) a living body by the same reason. Although the carbon black is not as poisonous as carbon nanotube, it also stimulates the skin to a certain degree. Even if these electrode materials themselves cause no allergic reaction in the manners described above, the biocompatibility may be degraded depending on the shape of a material and its inherent stimulation, thereby making it hard to satisfy both electric conductivity and biocompatibility.

Although metal films seem to function as excellent bio-electrodes thanks to extremely high electric conductivity, this is not always the case. Upon heartbeat, the human skin releases not only extremely weak current, but also sodium ion, potassium ion, and calcium ion. It is thus necessary to convert changes in ion concentration into current. Noble metals, however, are difficult to ionize and are inefficient in converting ions from skin to current. Therefore, the resulting bio-electrode using a noble metal is characterized by high impedance and high resistance to the skin during electrical conduction.

There has been proposed a lithium ion-conductive composite for solid electrolyte, the composite using a silicon atom-containing compound having a lithium ion-containing segment. It is known that because the composite has favorable electric conductivity and favorable lithium ion transport number at the use temperature of battery as well as excellent mechanical properties, moldability, and favorable adhesion to electrode, the resulting battery reduces or eliminates the risks of fire, electrolyte leakage, and so forth and is highly safe (Patent Document 6).

There have been proposed bio-electrodes in each of which an ionic polymer is added (Patent Documents 7, 8, 9). A bio-electrode obtained by mixing a silicone adhesive with an ionic polymer and a carbon powder added thereto has adhesion and high water repellency so that biological signals can be stably collected even when the bio-electrode is attached to the skin for a long time during which the user takes a shower and sweat. Ion polymers do not permeate the skin and hence do not stimulate the skin, and the biocompatibility is high. From these aspects, the bio-electrode enables long-time attachment.

Although silicones are inherently insulators, the ionic conductivity is improved by the combination with an ion polymer and a carbon powder, and thus the function as a bio-electrode is obtained. Nevertheless, it has been desired to improve the performance by further improving the ionic conductivity.

Patent Documents 7, 8, and 9 mentioned above state that a silicone compound having a polyether chain is effective as an additive to improve the ionic conductivity. Polyether chains are also used to improve the ionic conductivity of lithium ion polymer batteries, and are effective to improve the conductivity of ions. However, the ionic conductivity by such polyether chain is lower than that in a water-containing gel of a hydrophilic gel, and further improvement of the ionic conductivity is demanded.

Bio-electrodes are required to collect signals immediately after attached to skin. A gel electrode has ion concentrations equivalent to those of skin, and ions move in and out smoothly. In a water-containing gel, ions move so fast that signals can be detected immediately after attachment to the skin. Meanwhile, it takes long time for a dry electrode to detect signals after attachment to skin. This is presumably because no signal is found until the dry electrode surface is saturated with ions released from skin.

As described above, bio-electrodes are required to collect signals immediately after attachment to skin, but the dry electrode development faces many problems. Hence, it has been desired to develop a bio-electrode which does not cause residue on skin or skin irritation, and which collects signals immediately after attachment to skin.

### CITATION LIST

### PATENT LITERATURE

Patent Document 1: WO 2013/039151 A1
Patent Document 2: JP 2015-100673 A
Patent Document 3: JP H05-095924 A
Patent Document 4: JP 2003-225217 A
Patent Document 5: JP 2015-019806 A
Patent Document 6: JP 2007-059092 A
Patent Document 7: JP 2018-099504 A
Patent Document 8: JP 2018-126496 A
Patent Document 9: JP 2018-130533 A

### SUMMARY OF INVENTION

### TECHNICAL PROBLEM

The present invention has been made to solve the above problems. An object of the present invention is to provide: a bio-electrode composition capable of forming a living body contact layer for a bio-electrode which enables quick signal collection after attachment to skin and does not leave residue on the skin; a bio-electrode including a living body contact layer formed of the bio-electrode composition; and a method for manufacturing the bio-electrode.

### SOLUTION TO PROBLEM

To achieve the object, the present invention provides a bio-electrode composition, a bio-electrode, and a method for manufacturing the bio-electrode as follows.

The present invention provides a bio-electrode composition comprising:
a polymer compound (A), which comprises a repeating unit-a having a structure selected from the group consisting of salts of ammonium, sodium, potassium, and silver formed with any of fluorosulfonic acid, fluorosulfonimide, and N-carbonyl-fluorosulfonamide; and
filler particles (B), which are one or more selected from the group consisting of silica particles, alumina particles, titania particles, and zirconia particles.

Such a bio-electrode composition is capable of forming a living body contact layer for a bio-electrode which enables quick signal collection after attachment to skin, and which does not cause residue on the skin.

The repeating unit-a preferably has a structure shown by any of the following general formulae (1)-1 to (1)-4, 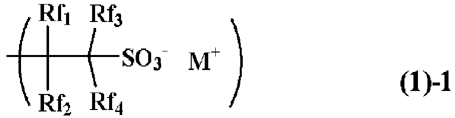 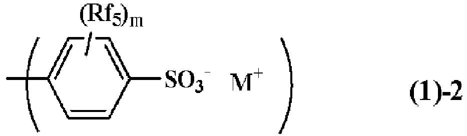 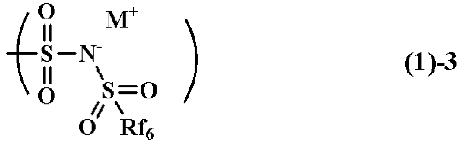 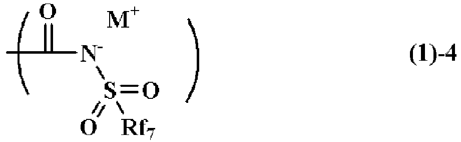 wherein Rf₁ and Rf₂ each represent a hydrogen atom, a fluorine atom, an oxygen atom, a methyl group, or a trifluoromethyl group, provided that when Rf₁ and Rf₂ represent an oxygen atom, the single oxygen atom represented by Rf₁ and Rf₂ bonds to a single carbon atom to form a carbonyl group; Rf₃ and Rf₄ each represent a hydrogen atom, a fluorine atom, or a trifluoromethyl group, provided that at least one of Rf₁ to Rf₄ is a fluorine atom or a trifluoromethyl group; Rf₅, Rf₆, and Rf₇ each represent a fluorine atom, or a linear or branched alkyl group having 1 to 4 carbon atoms, and have at least one fluorine atom; M⁺ represents an ion selected from the group consisting of an ammonium ion, a sodium ion, a potassium ion, and a silver ion; and "m" represents an integer of 1 to 4.

The repeating unit-a having such structures enables the bio-electrode composition to form a living body contact layer for a bio-electrode that is more excellent in electric conductivity and biocompatibility.

The repeating unit-a further preferably comprises at least one repeating unit selected from the group consisting of repeating units-al to -a7 shown by the following general formula (2), 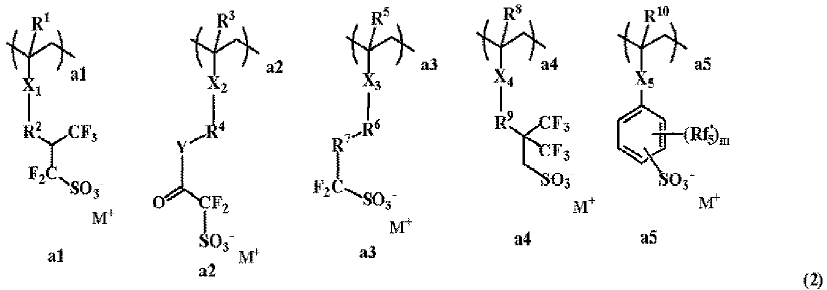 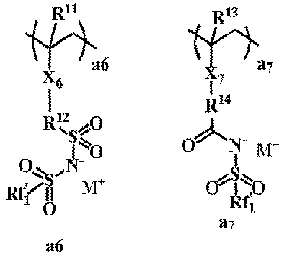 wherein R¹, R³, R⁵, R⁸, R¹⁰, R¹¹, and R¹³ each independently represent a hydrogen atom or a methyl group; R², R⁴, R⁶, R⁹, R¹², and R¹⁴ each independently represent a single bond, or a linear, branched, or cyclic hydrocarbon group having 1 to 13 carbon atoms, the hydrocarbon group optionally having either or both of an ester group and an ether group; R⁷ represents a linear or branched alkylene group having 1 to 4 carbon atoms, and one or two hydrogen atoms in R⁷ are optionally substituted with a fluorine atom; X₁, X₂, X₃, X₄, X₆, and X₇ each independently represent any of a single bond, a phenylene group, a naphthylene group, an ether group, an ester group, and an amide group; X₅ represents any of a single bond, an ether group, and an ester group; Y represents an oxygen atom or a -NR¹⁹-group; R¹⁹ represents a hydrogen atom, or a linear or branched alkyl group having 1 to 4 carbon atoms, and optionally forms a ring together with R⁴; Rf₁' and Rf₅' each represent a fluorine atom, a trifluoromethyl group, or a linear or branched alkyl group having 1 to 4 carbon atoms, and have at least one fluorine atom; "m" represents an integer of 1 to 4; a1, a2, a3, a4, a5, a6, and a7 satisfy 0≤a1≤1.0, 0≤a2≤1.0, 0≤a3≤1.0, 0≤a4≤1.0, 0≤a5≤1.0, 0≤a6≤1.0, 0≤a7≤1.0, and 0<a1+a2+a3+a4+a5+a6+a7≤1.0; and M⁺ represents an ion selected from the group consisting of an ammonium ion, a sodium ion, a potassium ion, and a silver ion.

The repeating unit-a having such structures enables the bio-electrode composition to form a living body contact layer for a bio-electrode that is further excellent in electric conductivity and biocompatibility.

The polymer compound (A) preferably comprises an ammonium ion shown by the following general formula (3) as an ammonium ion for forming the ammonium salts, 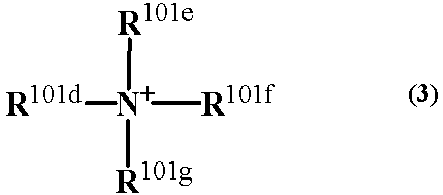 wherein R^{101d}, R^{101e}, R^{101f}, and R^{101g} each represent a hydrogen atom, a linear, branched, or cyclic alkyl group having 1 to 14 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and R^{101d} and R^{101e}, or R^{101d}, R^{101e}, and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (3) within the ring.

Incorporating the polymer compound (A) containing such an ammonium ion enables the bio-electrode composition to form a living body contact layer for a bio-electrode that is further excellent in electric conductivity and biocompatibility.

The bio-electrode composition preferably further comprises a compound (C) having a polyglycerin structure.

The bio-electrode composition containing the compound (C) having a polyglycerin structure is capable of forming a living body contact layer for a bio-electrode that can quickly collect signals after attached to skin.

The compound (C) having a polyglycerin structure is preferably a silicone compound having a polyglycerin structure.

The bio-electrode composition containing a silicone compound having a polyglycerin structure is capable of forming a living body contact layer for a bio-electrode that can more quickly collect signals after attached to skin.

The silicone compound having a polyglycerin structure is preferably shown by any of the following general formulae (4) and (5), 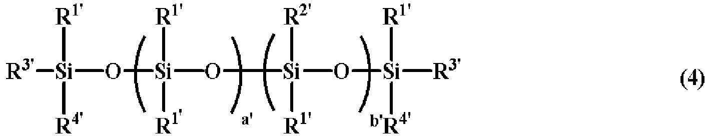 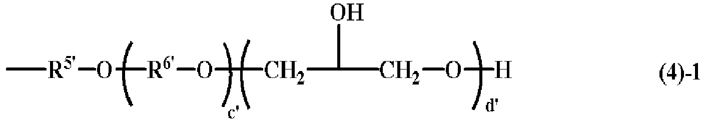 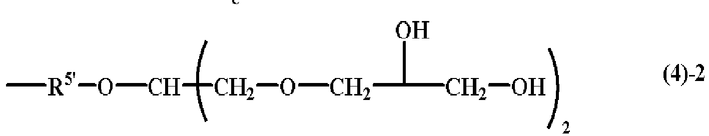 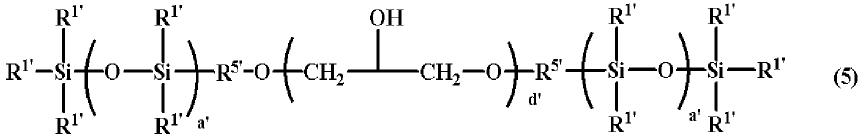 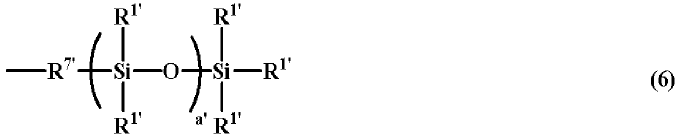 wherein each R¹' is identical to or different from one another, and independently represents a hydrogen atom, a phenyl group, a linear or branched alkyl group having 1 to 50 carbon atoms, or a silicone chain shown by a general formula (6), and optionally contains an ether group; R²' represents a group having a polyglycerin group structure shown by a general formula (4)-1 or (4)-2; each R³' is identical to or different from the other, and independently represents the R¹' group or the R²' group; each R⁴' is identical to or different from the other, and independently represents the R¹' group, the R²' group, or an oxygen atom, provided that when R⁴' represents an oxygen atom, the two R⁴' moieties bond to each other and optionally constitute an ether group to form a ring together with silicon atoms; each a' is identical to or different from one another and represents 0 to 100, b' represents 0 to 100, and a'+b' is 0 to 200, provided that when b' is 0, at least one R³' is the R²' group; R⁵' represents an alkylene group having 2 to 10 carbon atoms or an aralkylene group having 7 to 10 carbon atoms; R⁶' represents an alkylene group having 2 to 6 carbon atoms; R⁷' represents an alkylene group having 2 to 6 carbon atoms or an ether group; c' represents 0 to 20; and d' represents 1 to 20.

Incorporating such a silicone compound having a polyglycerin structure enables the bio-electrode composition to form a living body contact layer that can exhibit more excellent moisture-holding property and consequently exhibit more excellent sensitivity to ions released from skin.

The inventive bio-electrode composition may further comprise a resin component (D), which is one or more selected from the group consisting of a silicone-based resin, an acrylic-based resin, and a urethane-based resin.

The resin component (D) to be incorporated into the bio-electrode composition can be selected in accordance with properties to be imparted to the living body contact layer.

The resin component (D) can comprise any of:
a silicone resin having an SiO₂ unit and an RₓSiO_{(4-x)/2} unit, wherein R represents a substituted or unsubstituted monovalent hydrocarbon group having 1 to 10 carbon atoms, and "x" represents a number in a range of 2.5 to 3.5;
diorganosiloxane having an alkenyl group; and organohydrogenpolysiloxane having an SiH group.

Such resin components (D) are compatible with the polymer compound (A), thereby making it possible to prevent elution of the salt and to provide the bio-electrode composition with higher adhesion.

The inventive bio-electrode composition may further comprise an organic solvent (E).

In this case, the bio-electrode composition containing the organic solvent (E) can exhibit high coating properties.

The inventive bio-electrode composition may further comprise a component (F), which is a carbon powder, a silver powder, a silicon powder, and/or a lithium titanate powder.

A carbon powder and a silver powder function as electric conductivity improvers, and can impart more excellent electric conductivity to the living body contact layer formed from the bio-electrode composition. A silicon powder and a lithium titanate powder can further enhance the ion reception sensitivity of the living body contact layer formed from the bio-electrode composition.

The carbon powder is preferably one or both of carbon black and carbon nanotube.

Incorporating such a carbon powder(s) can provide higher electric conductivity.

The present invention also provides a bio-electrode comprising an electro-conductive base material and a living body contact layer formed on the electro-conductive base material, wherein
the living body contact layer comprises a cured product of the inventive bio-electrode composition.

Since the inventive bio-electrode has the living body contact layer containing the cured material of the above-described bio-electrode composition, the inventive bio-electrode is excellent in electric conductivity and biocompatibility, light-weight, manufacturable at low cost, capable of preventing significant reduction in the electric conductivity even when wetted with water or dried, transparent and excellent also in aesthetic feature, capable of quick signal collection after attached to skin, and does not leave residue on the skin.

The electro-conductive base material may comprise one or more selected from the group consisting of gold, silver, silver chloride, platinum, aluminum, magnesium, tin, tungsten, iron, copper, nickel, stainless steel, chromium, titanium, and carbon.

The inventive bio-electrode can use various electro-conductive base materials as mentioned above.

Preferably, the living body contact layer is humidified.

The bio-electrode including such a living body contact layer is capable of more quick signal collection after attachment to skin.

Further, the present invention provides a method for manufacturing a bio-electrode having an electro-conductive base material and a living body contact layer formed on the electro-conductive base material, comprising:
applying the inventive bio-electrode composition onto the electro-conductive base material; and
curing the bio-electrode composition to form the living body contact layer.

Such a manufacturing method makes it possible to manufacture easily at low cost a bio-electrode which is excellent in electric conductivity and biocompatibility, light-weight, capable of preventing significant reduction in the electric conductivity even when wetted with water or dried, transparent and also excellent in aesthetic feature, and capable of quick signal collection after attached to skin.

The electro-conductive base material to be used may comprise one or more selected from the group consisting of gold, silver, silver chloride, platinum, aluminum, magnesium, tin, tungsten, iron, copper, nickel, stainless steel, chromium, titanium, and carbon.

The inventive method for manufacturing a bio-electrode can employ various electro-conductive base materials as mentioned above.

Preferably, in this method, after the living body contact layer is formed, the living body contact layer is immersed in water, or the living body contact layer is humidified.

These allow more effective utilization of the moisture-holding effect of the living body contact layer.

### ADVANTAGEOUS EFFECTS OF INVENTION

As described above, the inventive bio-electrode composition makes it possible to form a living body contact layer for a bio-electrode which is excellent in electric conductivity and biocompatibility, light-weight, manufacturable at low cost, and free from significant reduction in the electric conductivity even when the bio-electrode is wetted with water or dried, and which is transparent, excellent also in aesthetic feature, capable of quick signal collection after attachment to skin, and does not leave residue on the skin.

### BRIEF DESCRIPTION OF DRAWINGS

FIG. 1 is a schematic sectional view showing an example of the inventive bio-electrode;
FIG. 2 is a schematic sectional view showing an example of the inventive bio-electrode worn on a living body;
FIG. 3 is a schematic view of printed bio-electrodes prepared in Examples of the present invention;
FIG. 4 is a schematic view of one of the bio-electrodes prepared in Examples of the present invention, the bio-electrode being cut out and provided with an adhesive layer thereon;
FIG. 5 is a view showing locations where electrodes and earth are attached on a human body in measuring biological signals in Examples of the present invention; and
FIG. 6 shows one of electrocardiogram waveforms obtained using the bio-electrodes in Examples of the present invention.

### DESCRIPTION OF EMBODIMENTS

As noted above, it has been desired to develop: a bio-electrode composition that can form a living body contact layer for a bio-electrode which can quickly collect signals after attachment to skin, and which does not leave residue on the skin; a bio-electrode including a living body contact layer formed from the bio-electrode composition; and a method for manufacturing the bio-electrode.

The surface of skin releases ions of sodium, potassium, and calcium in accordance with heartbeat. A bio-electrode has to convert the increase and decrease of ions released from skin to electric signals. Accordingly, the bio-electrode requires a material that is excellent in ionic conductivity to transmit the increase and decrease of ions.

In neutralized salts formed from highly acidic acids, the ions are strongly polarized to improve the ionic conductivity. This is why lithium salts of bis(trifluoromethanesulfonyl)imidic acid and tris(trifluoromethanesulfonyl)methide acid show high ionic conductivity as an electrolyte of a lithium ion battery. On the other hand, the higher acidity of the acid before the neutral salt formation results in a problem that the salt has stronger irritation to a body. That is, ionic conductivity and irritation to a body are in relation of trade-off. However, a salt applied to a bio-electrode has to achieve both high ionic conductivity and low irritation to a body.

Ion compound having higher molecular weight have such natures that the permeability and the stimulus to skin are decreased. Accordingly, the ion compound is preferably a polymer type with higher molecular weight. Thus, the present inventors have synthesized such a polymer by polymerizing an ion compound having a polymerizable double bond, that is, a polymer compound (A), and have found that adding this polymer enables formation of a bio-electrode sensitive to the increase and decrease of ions released from skin.

Patent Documents 7, 8, and 9 noted above disclose a copolymer of a strong acidic ionic repeating unit, a repeating unit having a silicone chain, and a hydrophilic repeating unit such as polyether. The ionic repeating unit and the hydrophilic repeating unit are units necessary to exhibit and enhance the ionic conductivity in accordance with the combination. However, these units alone make the hydrophilicity so high that when the resulting bio-electrode film comes into contact with water or sweat, the ion polymer is dissolved in water, and no biological signal is collected in some cases. Accordingly, the ion polymer needs to be insoluble in water. For this reason, the repeating unit having a silicone chain is also copolymerized.

When an ion polymer having all of an ionic repeating unit, a hydrophilic repeating unit, and a repeating unit with hydrophobic silicone is added to a silicone adhesive, ionic conductivity is exhibited, and biological signals can be obtained. The mechanism of ion conduction in the silicone adhesive, which is inherently an insulator, is conceivably attributable to the microphase separation structure of the ion polymer. Nafion, which is excellent in ionic conductivity, is described to exhibit high ionic conductivity by microphase separation of a hydrophilic sulfonate moiety and a hydrophobic fluoropolymer moiety thereof.

If an ionic polymer for bio-electrode can be formed to attain more prominent microphase separation, the ionic conductivity will be further improved, and it will be possible to form a dry electrode that can obtain biological signals in higher sensitivity.

Furthermore, the present inventors have found that when one or more kinds of filler particles selected from the group consisting of silica particles, alumina particles, titania particles, and zirconia particles are added to an ion polymer having an ionic repeating unit, a hydrophilic repeating unit, and a repeating unit with hydrophobic silicone, this enhances the effect of preventing the ion polymer from being dissolved in water. When the bio-electrode composition disclosed in Patent Documents 7, 8, and 9 is immersed in water, the ion polymer might be eluted (dissolved) into water. Nevertheless, the interaction among the silicon atoms is further enhanced by adding silica particles, and enables a bio-electrode composition whose components are not eluted into water.

In this respect, adding a hygroscopic material is effective to further improve the ionic conductivity. Patent Documents 7, 8, and 9 disclose the addition of a polyether-silicone compound. Further, examples of the hygroscopic material include silicone compounds having polyglycerin. Polyglycerin having not only a polyether group but also a hydroxy group is high in hygroscopicity, and high ionic conductivity can be obtained by combining an ion polymer with a compound having a silicone chain bonded to such polyglycerin. Adding polyglycerin silicone improves ionic conductivity, but lowered crosslinking density may cause residue to remain on skin after the bio-electrode film is peeled from the skin. In such cases, adding silica particles enhances the strength of the bio-electrode film, and can suppress the residue after peeling.

In sum, the inventive bio-electrode composition is capable of forming a living body contact layer for a bio-electrode which is excellent in electric conductivity and biocompatibility, light-weight, manufacturable at low cost, and free from significant reduction in the electric conductivity even when the bio-electrode is wetted with water or dried, and which enables quick signal collection after attachment to skin, does not leave residue on the skin, and is transparent and also excellent in aesthetic feature.

Specifically, the present invention is a bio-electrode composition comprising:
a polymer compound (A), which comprises a repeating unit-a having a structure selected from the group consisting of salts of ammonium, sodium, potassium, and silver formed with any of fluorosulfonic acid, fluorosulfonimide, and N-carbonyl-fluorosulfonamide; and
filler particles (B), which are one or more selected from the group consisting of silica particles, alumina particles, titania particles, and zirconia particles.

Hereinafter, the present invention will be described in detail, but the present invention is not limited thereto.

### <Bio-Electrode Composition>

The inventive bio-electrode composition contains: a polymer compound (polymer) (A) having an ionic repeating unit; and one or more kinds of filler particles (B) selected from the group consisting of silica particles, alumina particles, titania particles, and zirconia particles.

The inventive bio-electrode composition may further contain, for example, a compound (C) having a polyglycerin structure, a resin component (D), a metal powder, a carbon powder, a silicon powder, a lithium titanate powder, a tackifier, a crosslinking agent, a crosslinking catalyst, an ionic additive, and/or an organic solvent (E).

Hereinbelow, each component will be described in more details.

### [Polymer Compound (A)]

The polymer compound (A) blended in the inventive bio-electrode composition can also be called, for example, ionic material (conductive material). The polymer compound (A) is specifically a polymer compound (polymer) containing a repeating unit-a having a structure selected from the group consisting of salts of ammonium, sodium, potassium, and silver formed with any of fluorosulfonic acid, fluorosulfonimide, and N-carbonyl-fluorosulfonamide. Hence, the polymer compound (A) can be called salt, too.

The polymer compound (A) incorporated in the inventive bio-electrode composition allows electricity to pass therethrough due to the ion conductivity. In other words, the inventive bio-electrode composition is capable of exhibiting high ionic conductivity and thus excellent electric conductivity.

Additionally, the polymer compound (A) incorporated in the inventive bio-electrode composition can exhibit sufficiently low acidity to achieve low irritation to a body.

The repeating unit-a preferably has a structure shown by any of the following general formulae (1)-1 to (1)-4.

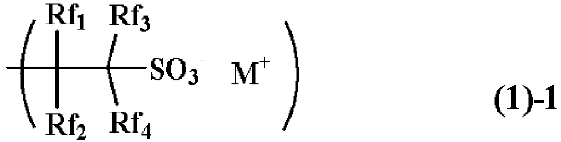

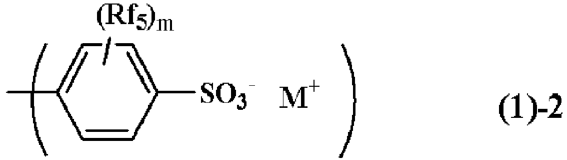

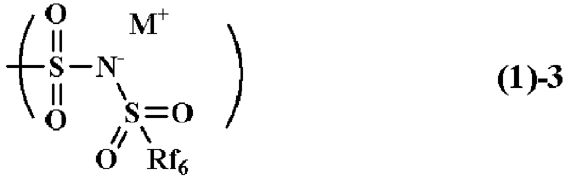

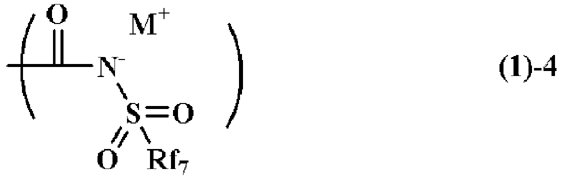

In the general formula (1)-1, Rf₁ and Rf₂ each represent a hydrogen atom, a fluorine atom, an oxygen atom, a methyl group, or a trifluoromethyl group. When Rf₁ and Rf₂ represent an oxygen atom, the single oxygen atom represented by Rf₁ and Rf₂ bonds to a single carbon atom to form a carbonyl group. Rf₃ and Rf₄ each represent a hydrogen atom, a fluorine atom, or a trifluoromethyl group. At least one of Rf₁ to Rf₄ is a fluorine atom or a trifluoromethyl group. In the general formulae (1)-2, (1)-3, and (1)-4, Rf₅, Rf₆, and Rf₇ each represent a fluorine atom, or a linear or branched alkyl group having 1 to 4 carbon atoms, and have at least one fluorine atom. In the general formulae (1)-1 to (1)-4, M⁺ represents an ion selected from the group consisting of an ammonium ion, a sodium ion, a potassium ion, and a silver ion. In the general formula (1)-2, "m" represents an integer of 1 to 4.

The repeating unit-a with such structures enables the bio-electrode composition to form a living body contact layer for a bio-electrode which is more excellent electric conductivity and biocompatibility.

The repeating unit-a further preferably includes at least one repeating unit selected from the group consisting of repeating units-al to -a7 shown in the following general formula (2).

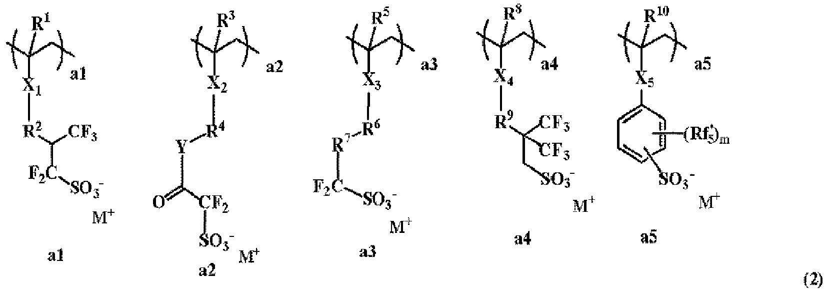

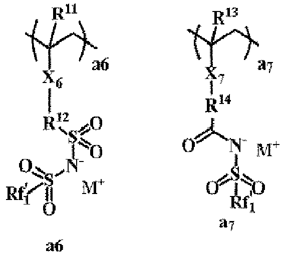

In the general formula (2), R¹, R³, R⁵, R⁸, R¹⁰, R¹¹, and R¹³ each independently represent a hydrogen atom or a methyl group. R², R⁴, R⁶, R⁹, R¹², and R¹⁴ each independently represent a single bond, or a linear, branched, or cyclic hydrocarbon group having 1 to 13 carbon atoms. This hydrocarbon group optionally has either or both of an ester group and an ether group. R⁷ represents a linear or branched alkylene group having 1 to 4 carbon atoms. One or two hydrogen atoms in R⁷ are optionally substituted with a fluorine atom. X₁, X₂, X₃, X₄, X₆, and X₇ each independently represent any of a single bond, a phenylene group, a naphthylene group, an ether group, an ester group, and an amide group. X₅ represents any of a single bond, an ether group, and an ester group. Y represents an oxygen atom or a -NR¹⁹- group. R¹⁹ represents a hydrogen atom, or a linear or branched alkyl group having 1 to 4 carbon atoms, and optionally forms a ring together with R⁴. Rf₁' and Rf₅' each represent a fluorine atom, a trifluoromethyl group, or a linear or branched alkyl group having 1 to 4 carbon atoms, and have at least one fluorine atom. "m" represents an integer of 1 to 4. a1, a2, a3, a4, a5, a6, and a7 satisfy 0≤a1≤1.0, 0≤a2≤1.0, 0≤a3≤1.0, 0≤a4≤1.0, 0≤a5≤1.0, 0≤a6≤1.0, 0≤a7≤1.0, and 0<a1+a2+a3+a4+a5+a6+a7≤1.0. M⁺ represents an ion selected from the group consisting of an ammonium ion, a sodium ion, a potassium ion, and a silver ion.

The repeating unit-a with such structures enables the bio-electrode composition to form a living body contact layer for a bio-electrode which is further excellent in electric conductivity and biocompatibility.

Note that a1, a2, a3, a4, a5, a6 and a7 are symbols to identify the respective repeating units, and also represent proportions of the corresponding repeating units in the polymer compound (A). The general formula (2) specifically illustrates: the repeating units-al, -a2, -a3, -a4, and -a5 in this order from left to right at the top; and the repeating units-a6 and -a7 in this order from left to right at the bottom.

Among the repeating units-al to -a7 shown by the general formula (2), the repeating units-al to -a5 can be obtained from fluorosulfonic acid salt monomers specifically exemplified below.

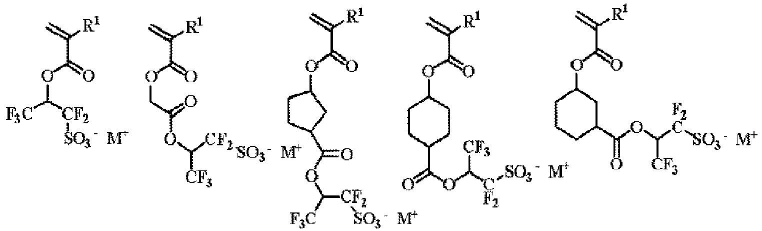

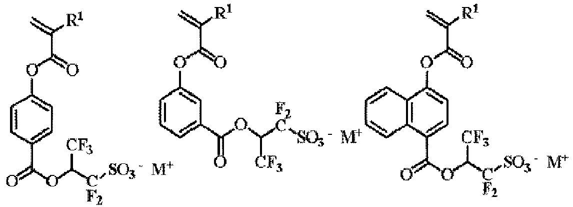

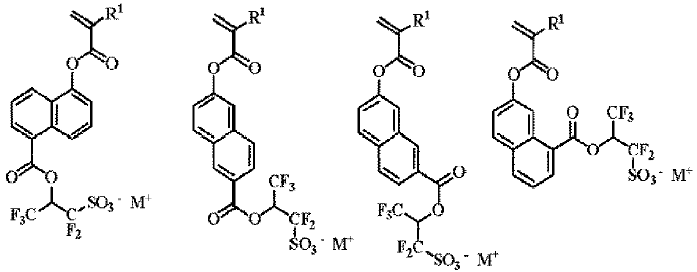

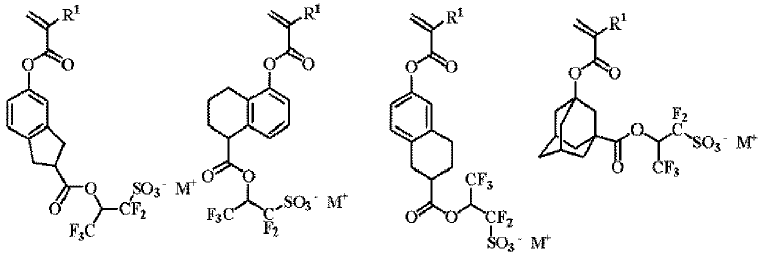

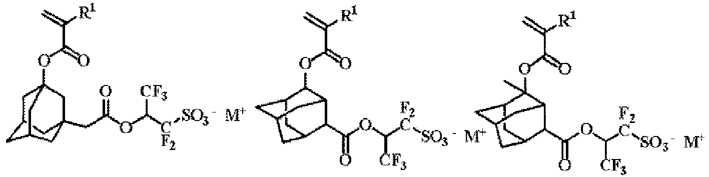

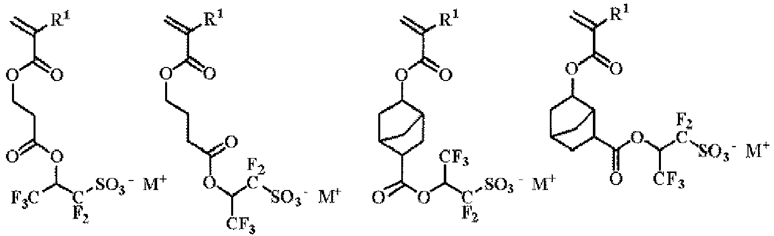

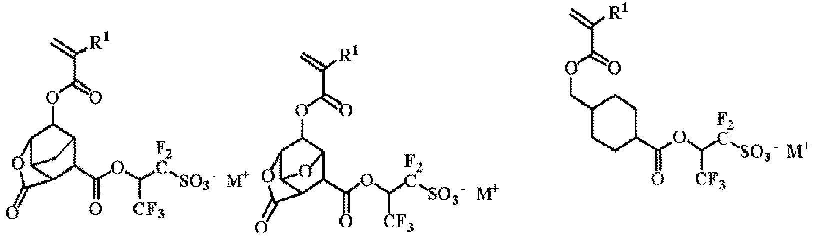

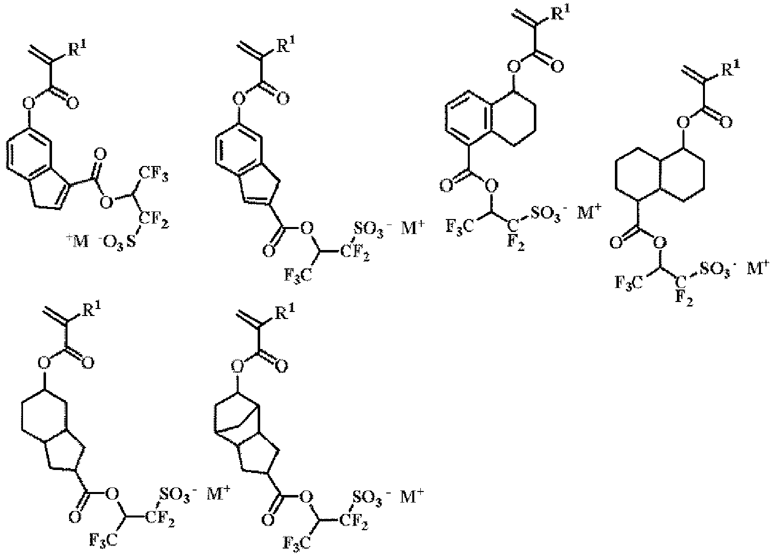

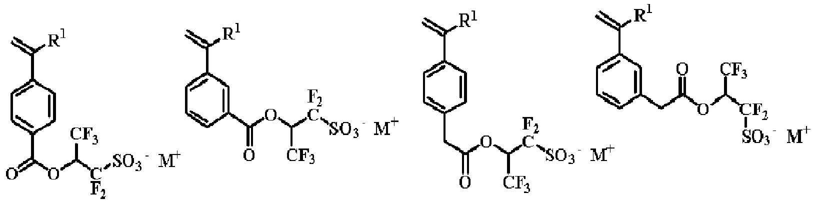

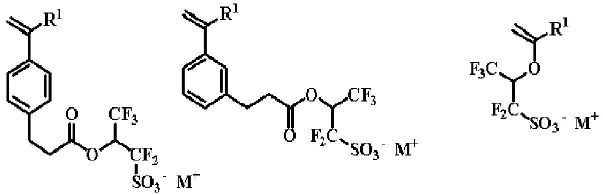

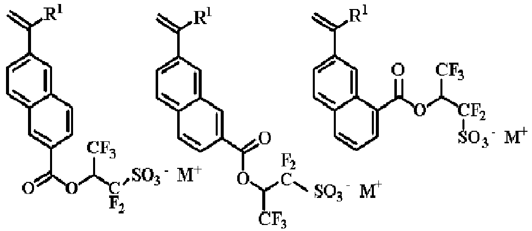

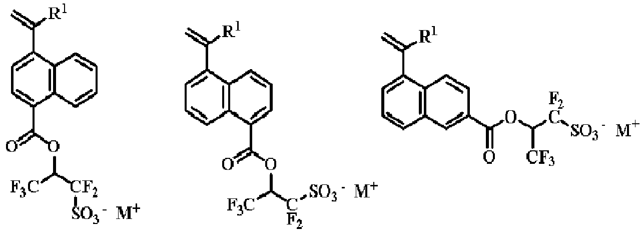

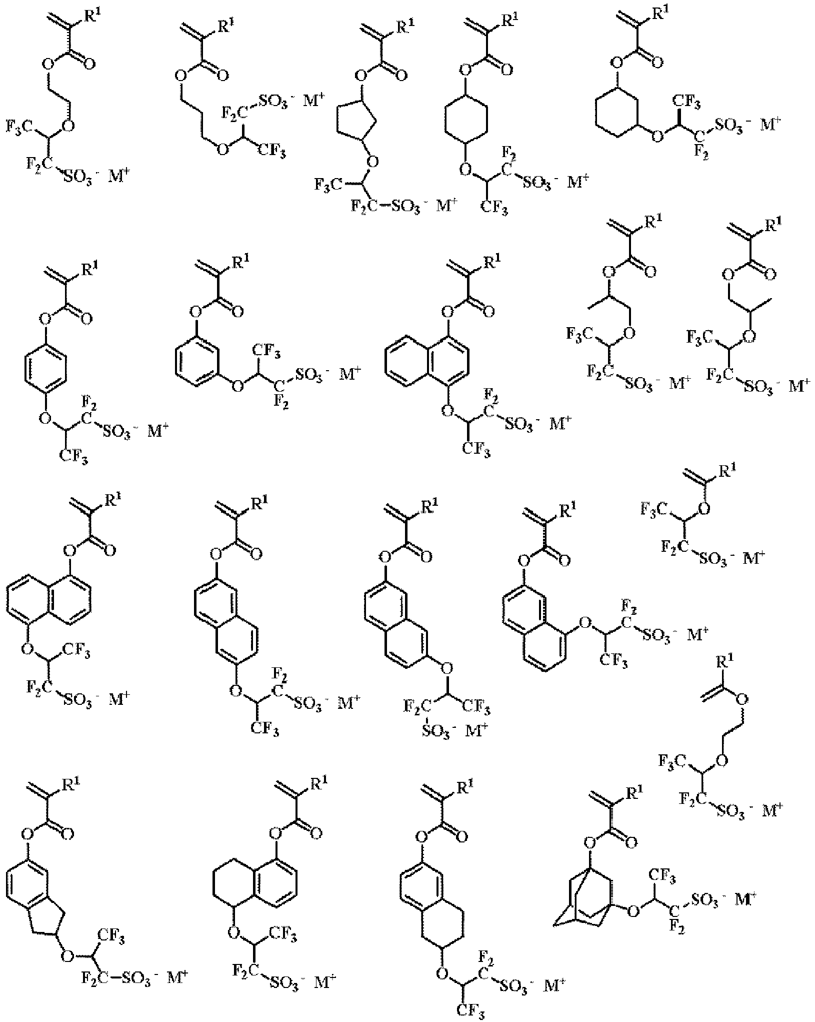

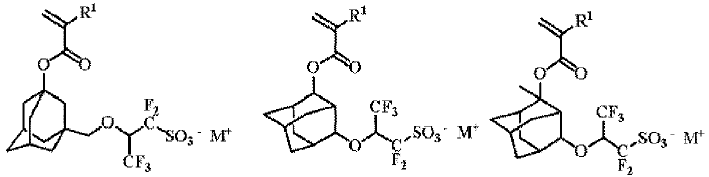

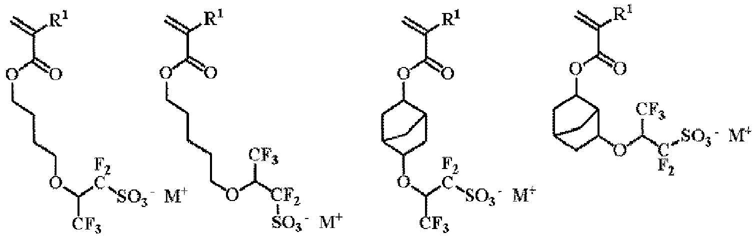

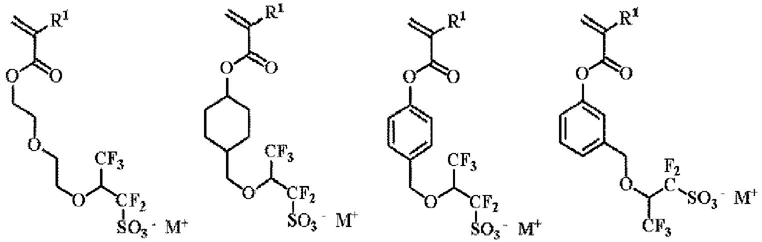

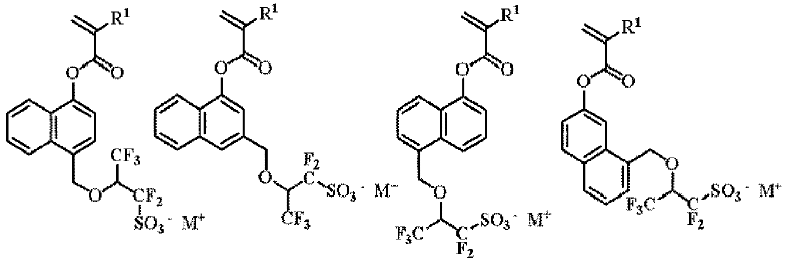

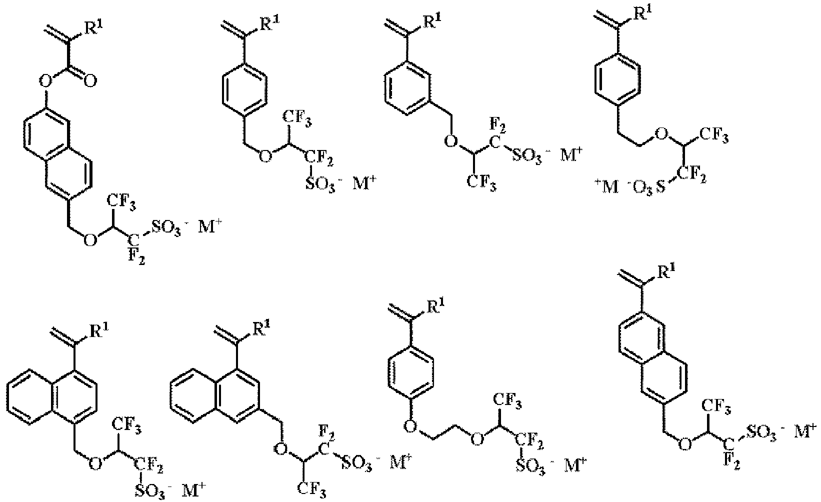

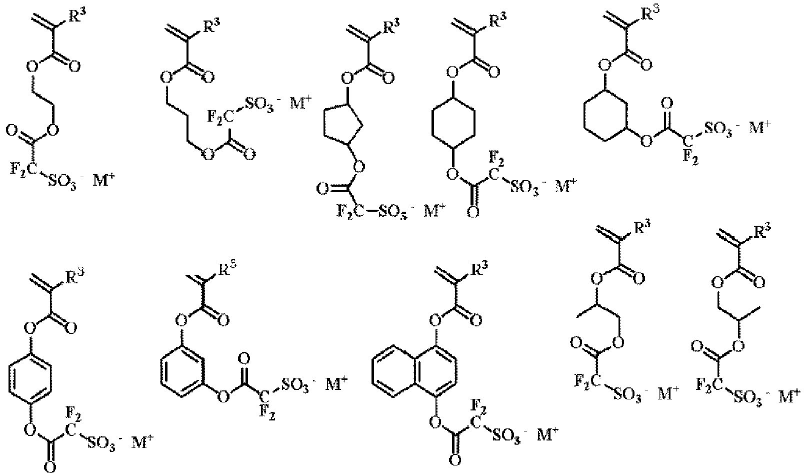

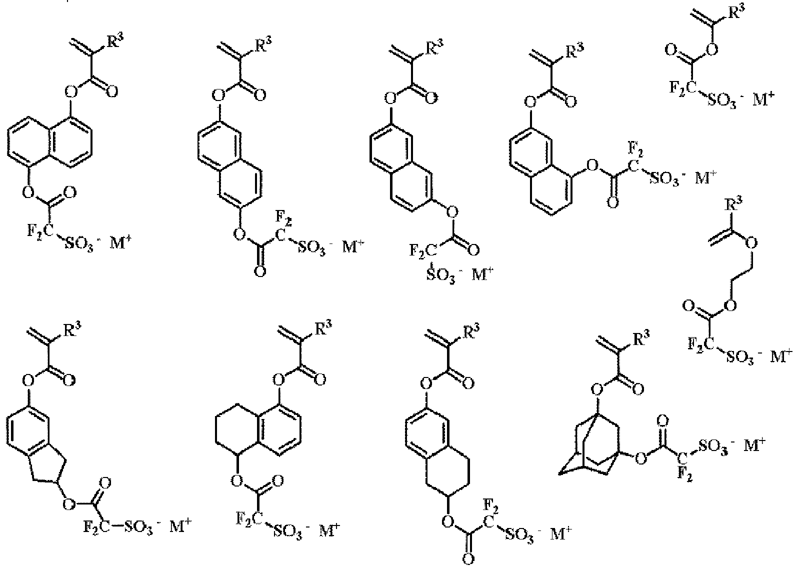

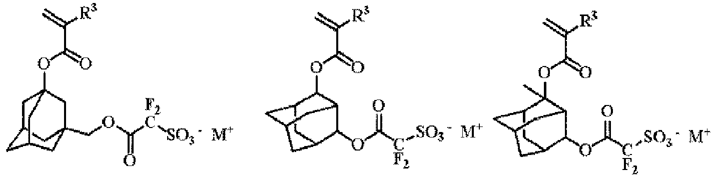

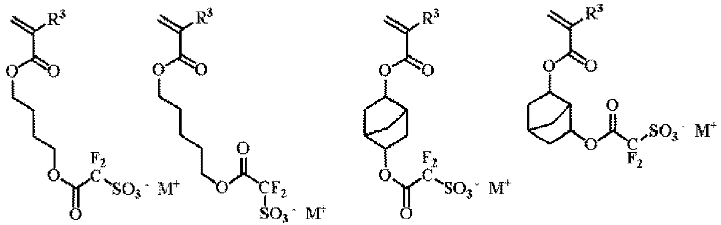

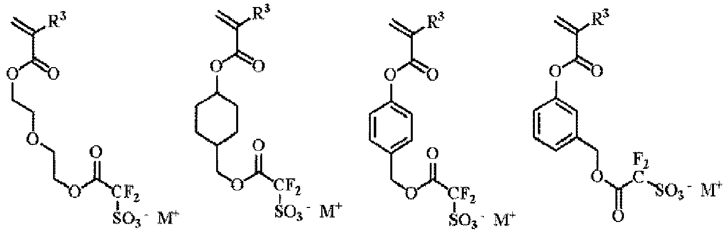

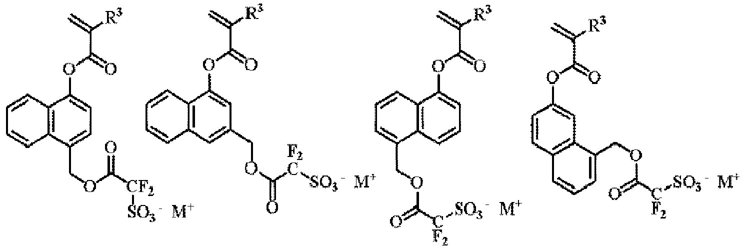

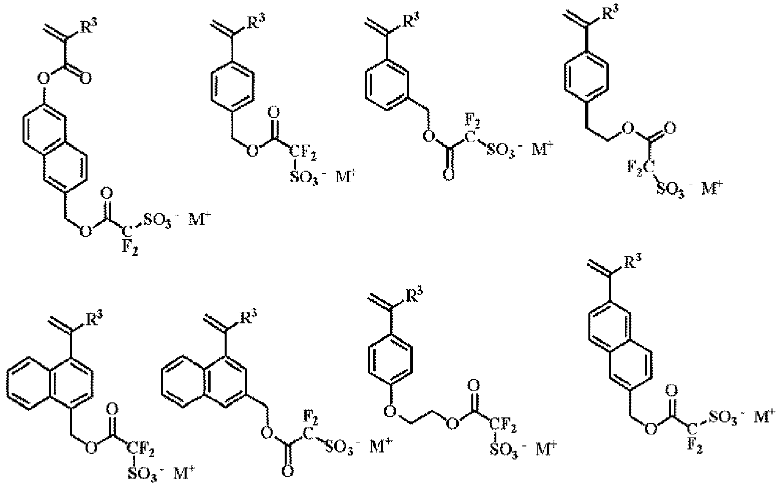

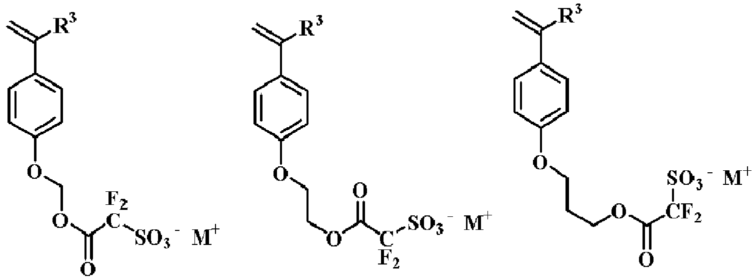

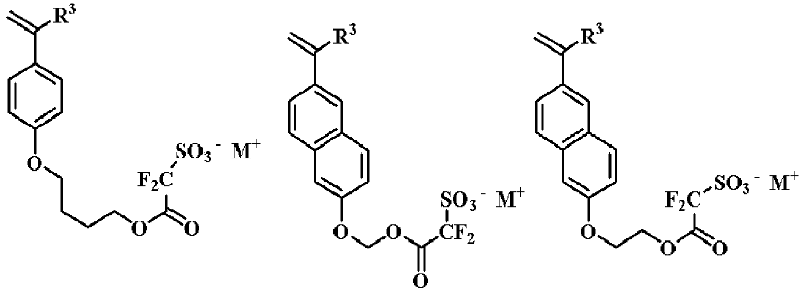

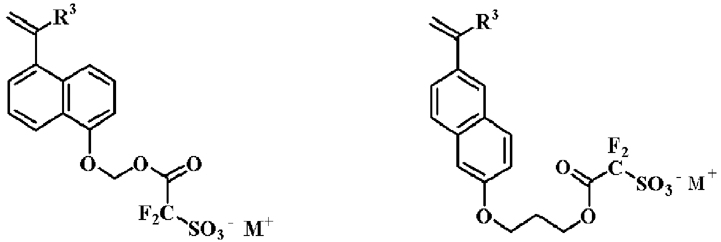

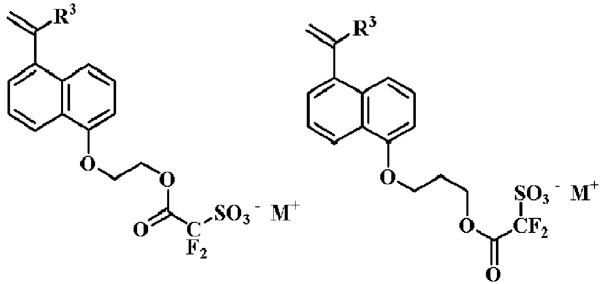

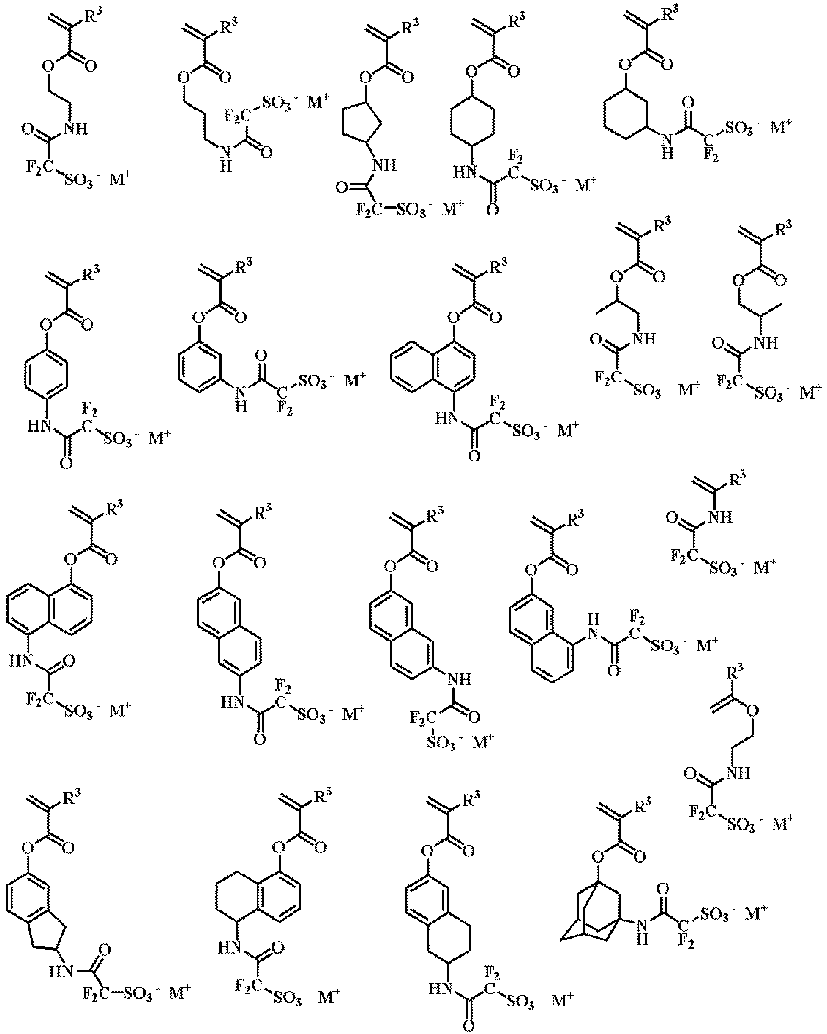

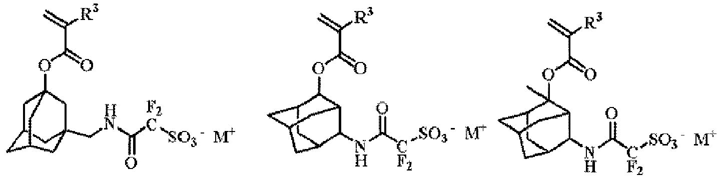

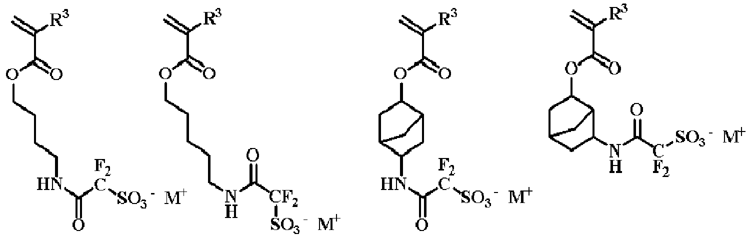

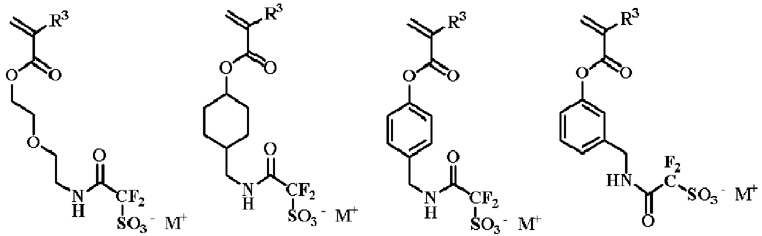

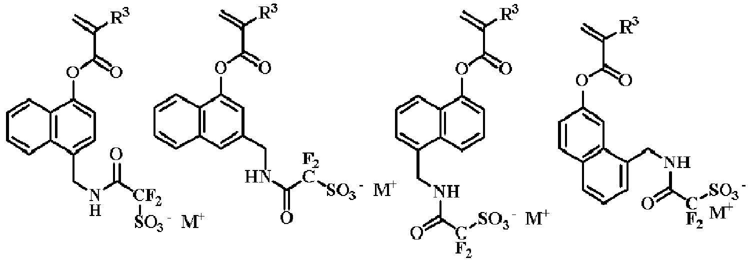

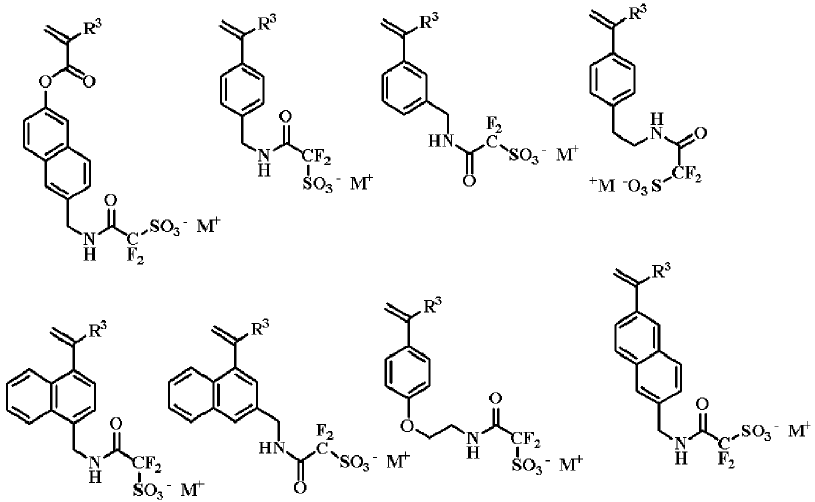

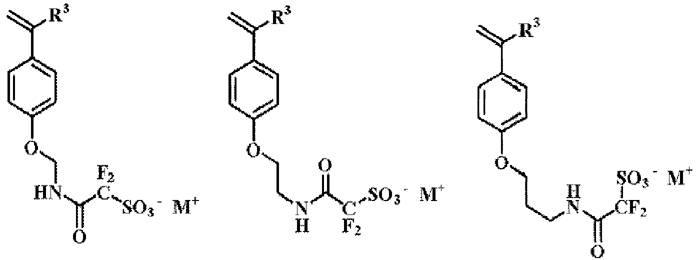

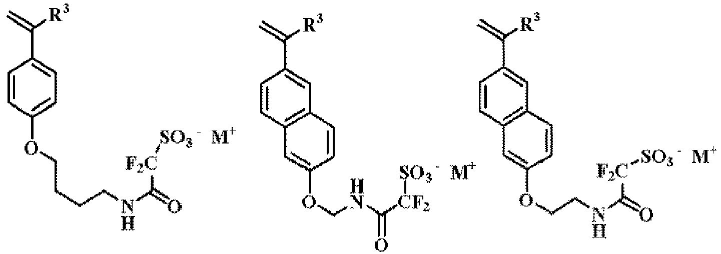

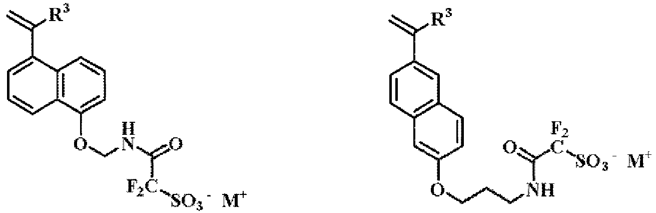

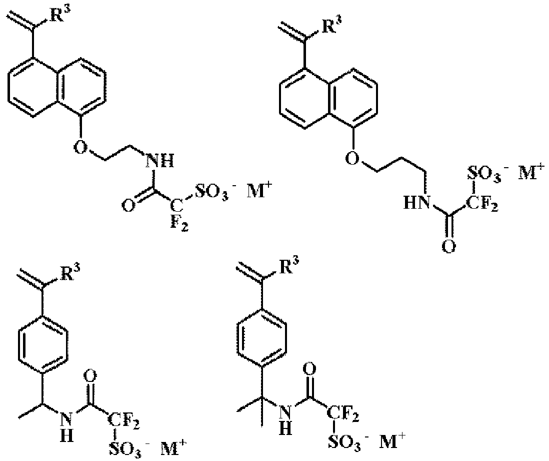

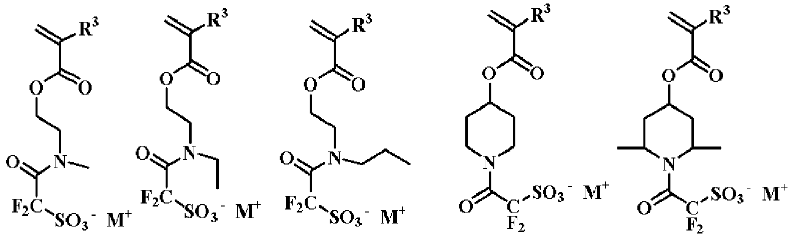

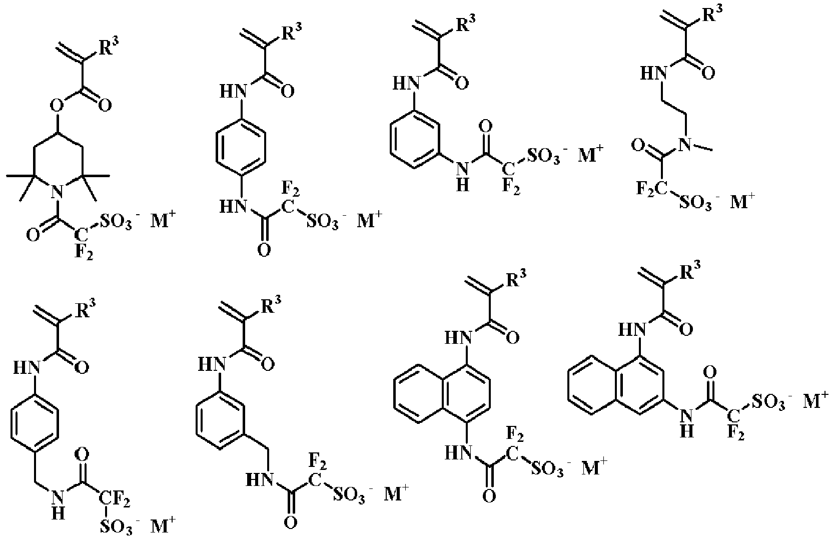

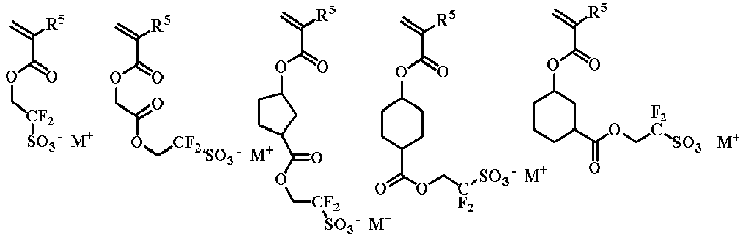

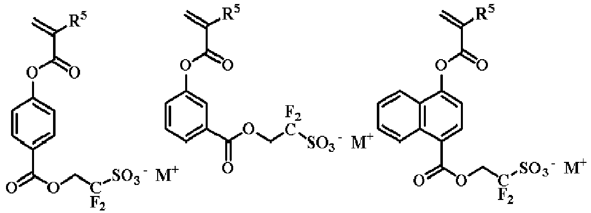

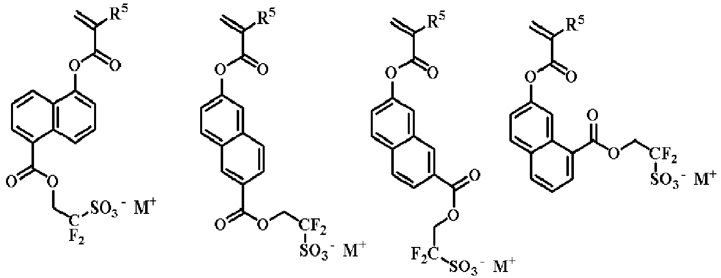

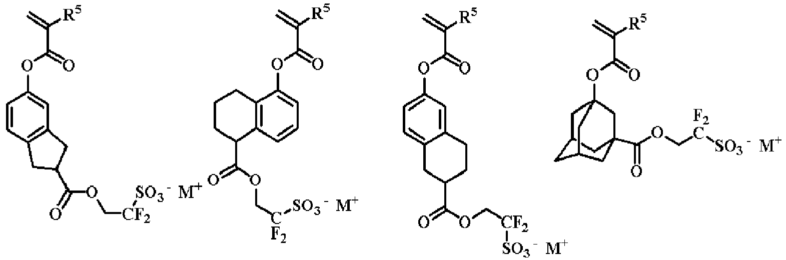

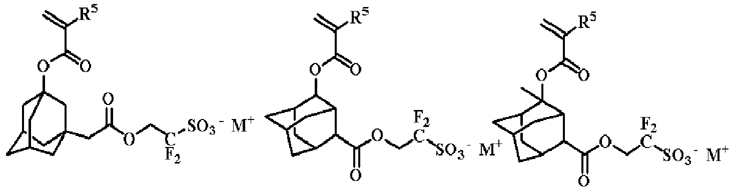

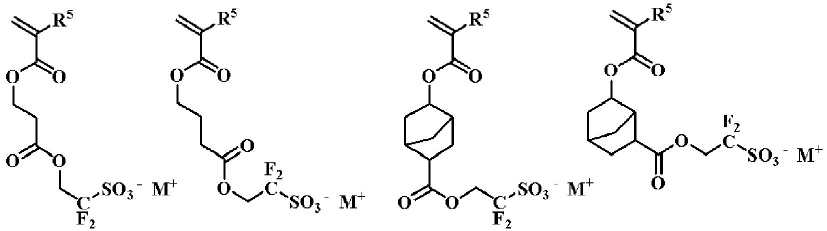

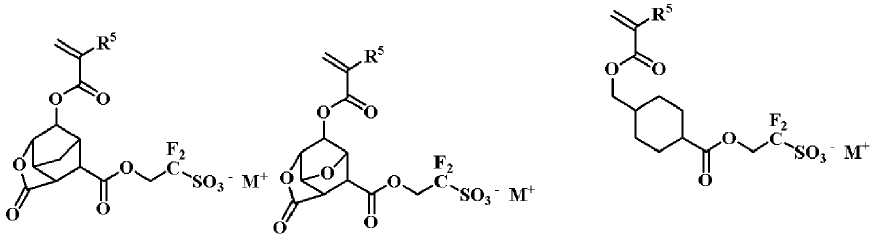

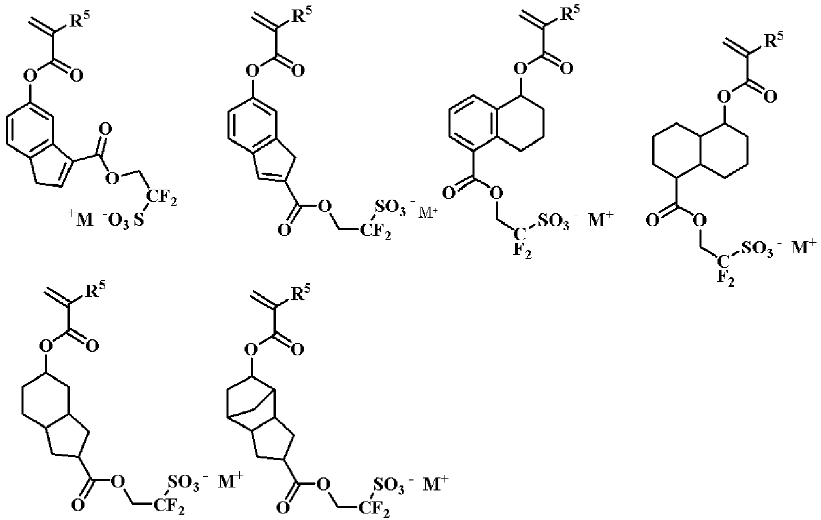

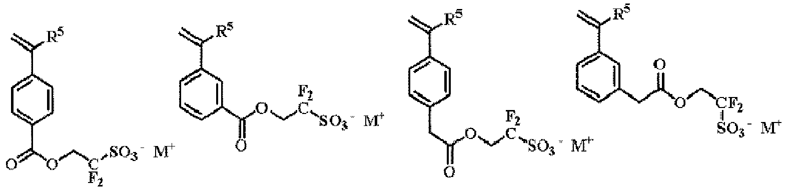

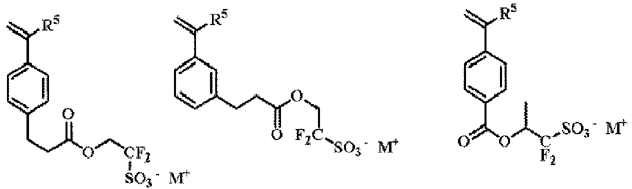

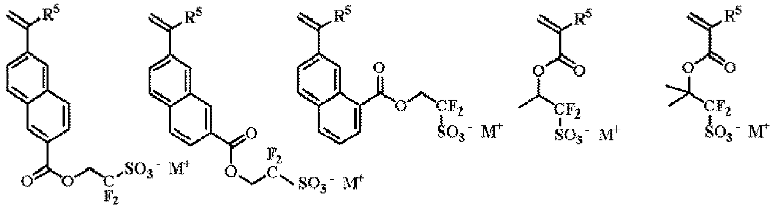

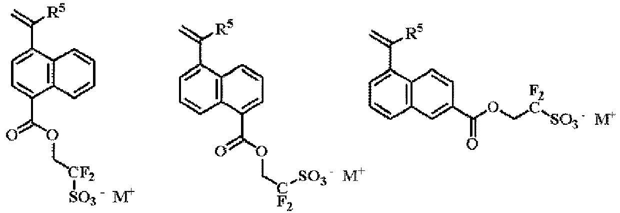

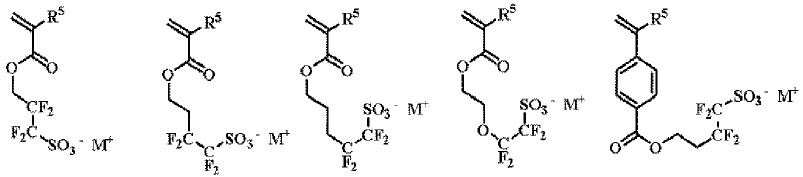

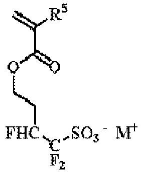

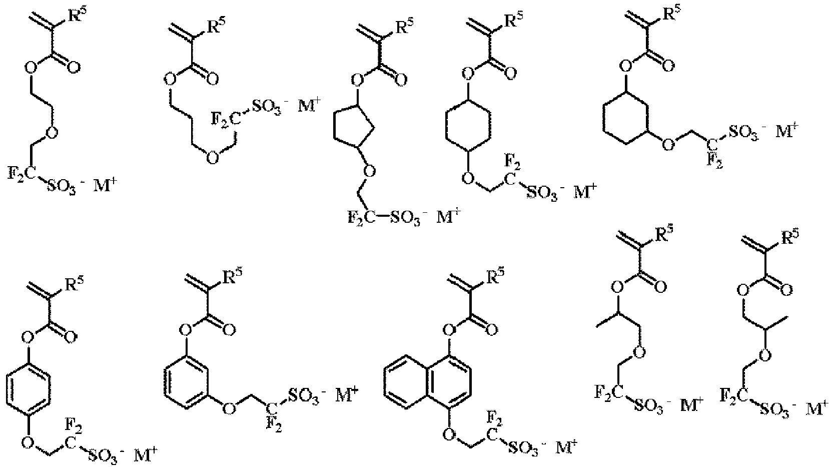

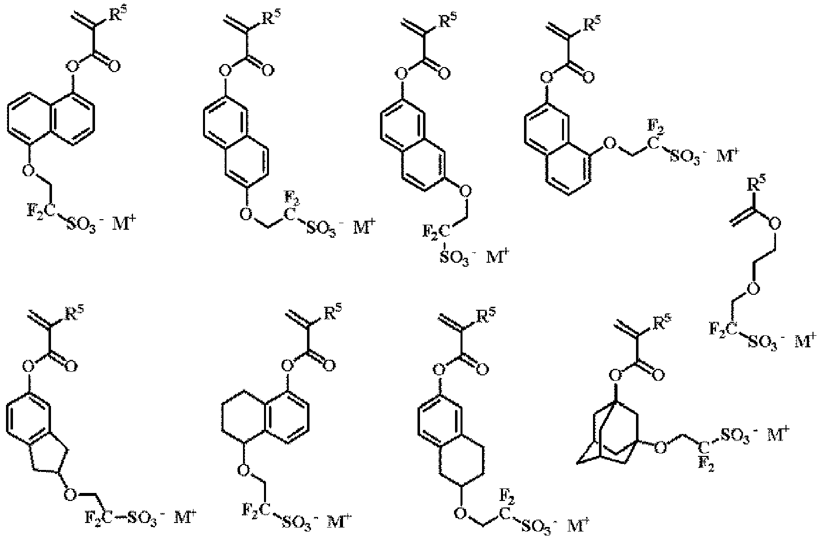

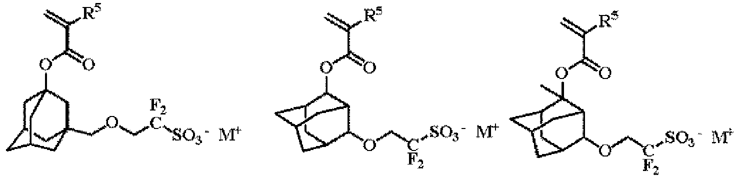

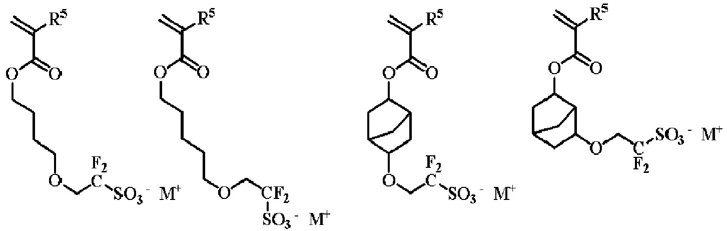

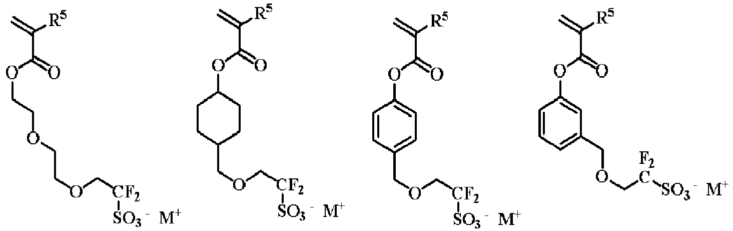

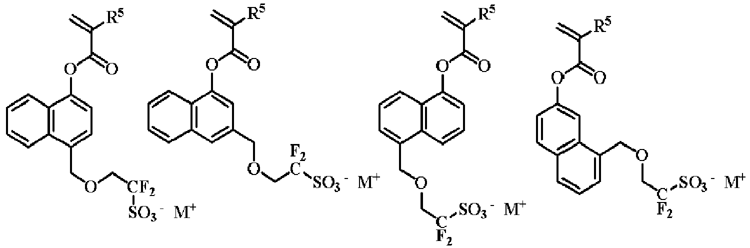

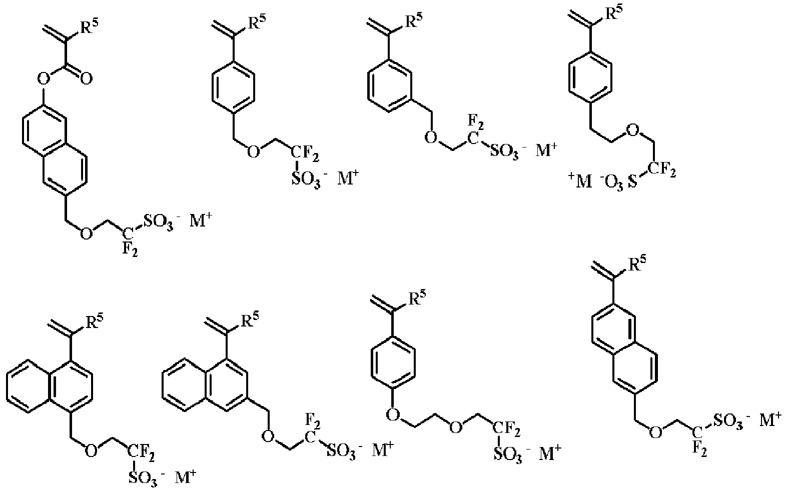

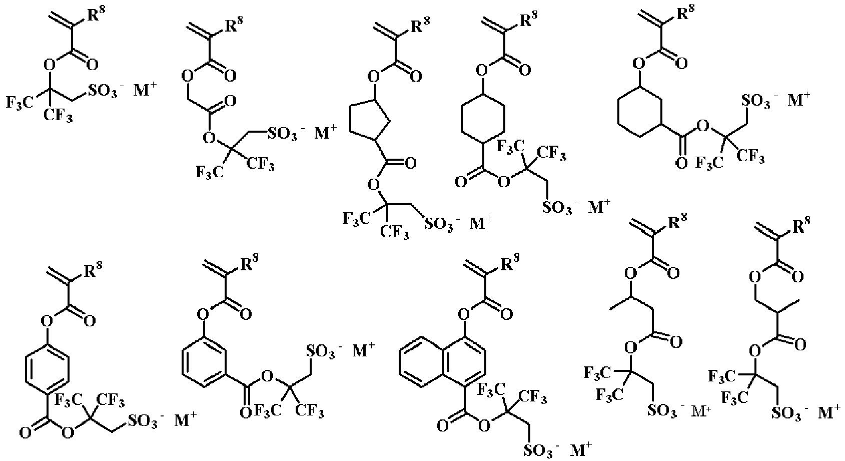

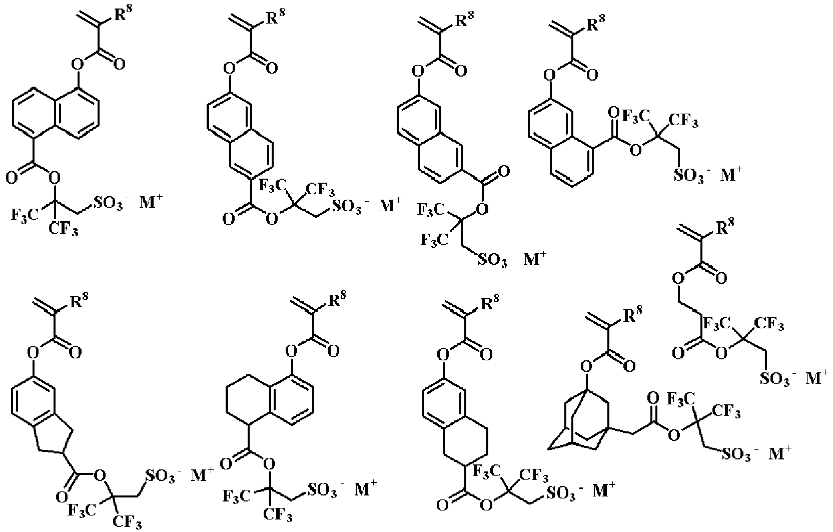

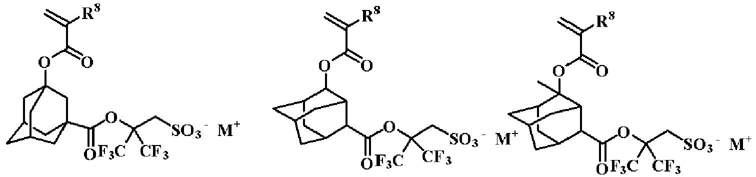

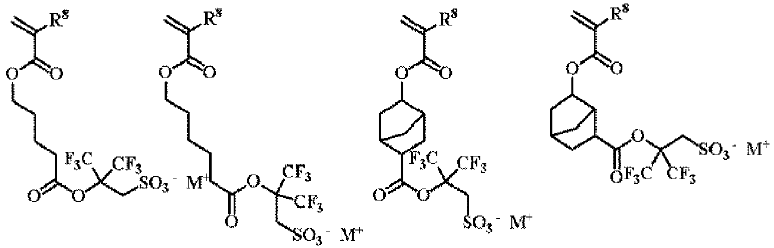

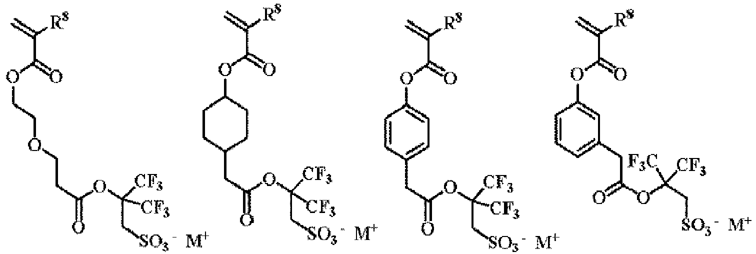

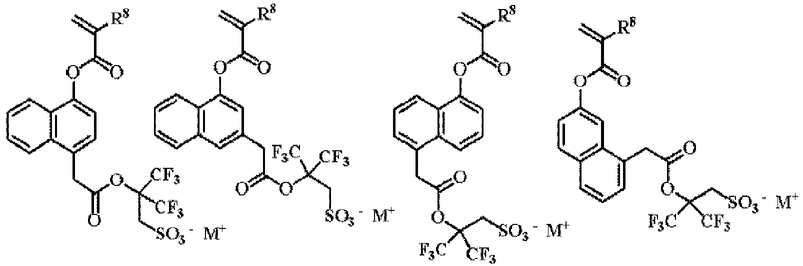

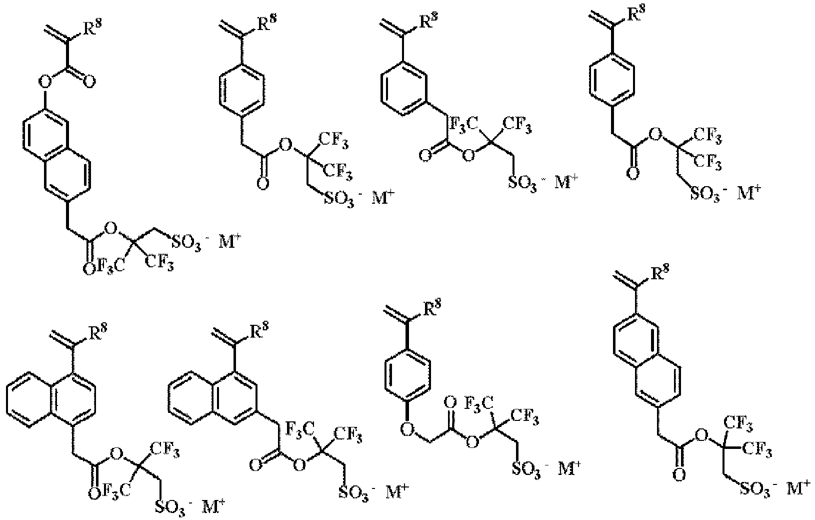

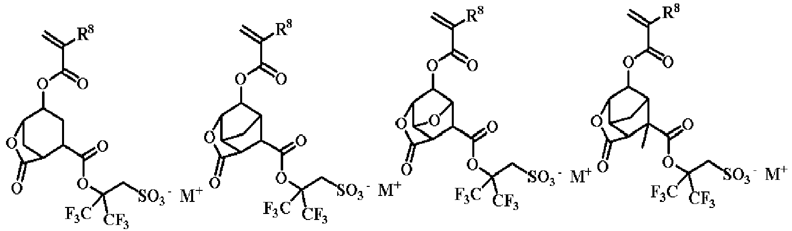

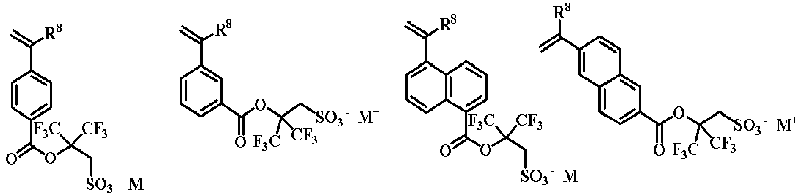

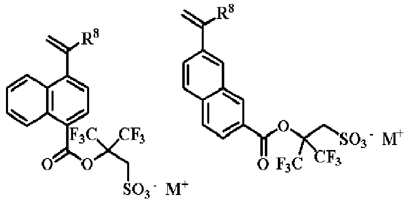

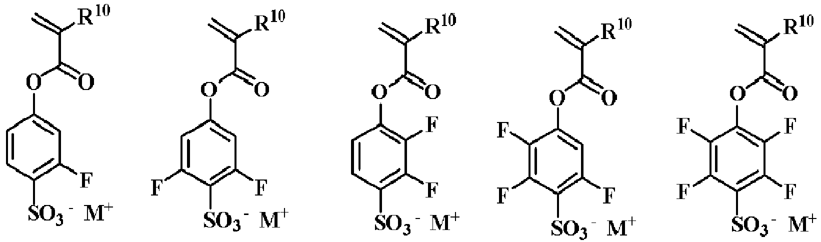

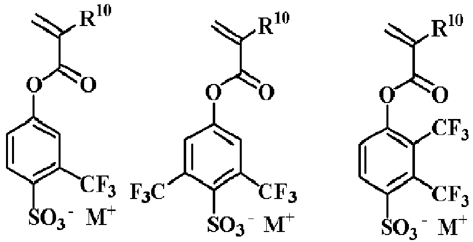

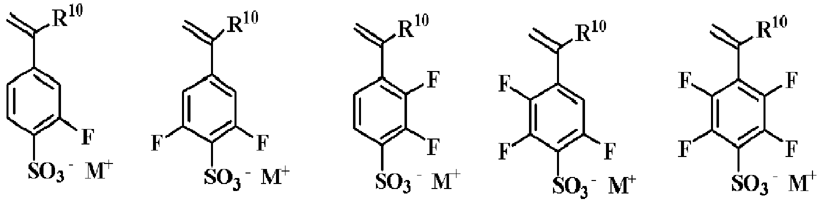

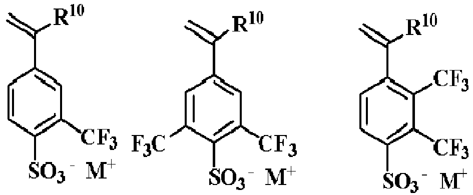

Specific examples of sulfonimide salt monomer to give the repeating unit-a6 in the above general formula can include the following.

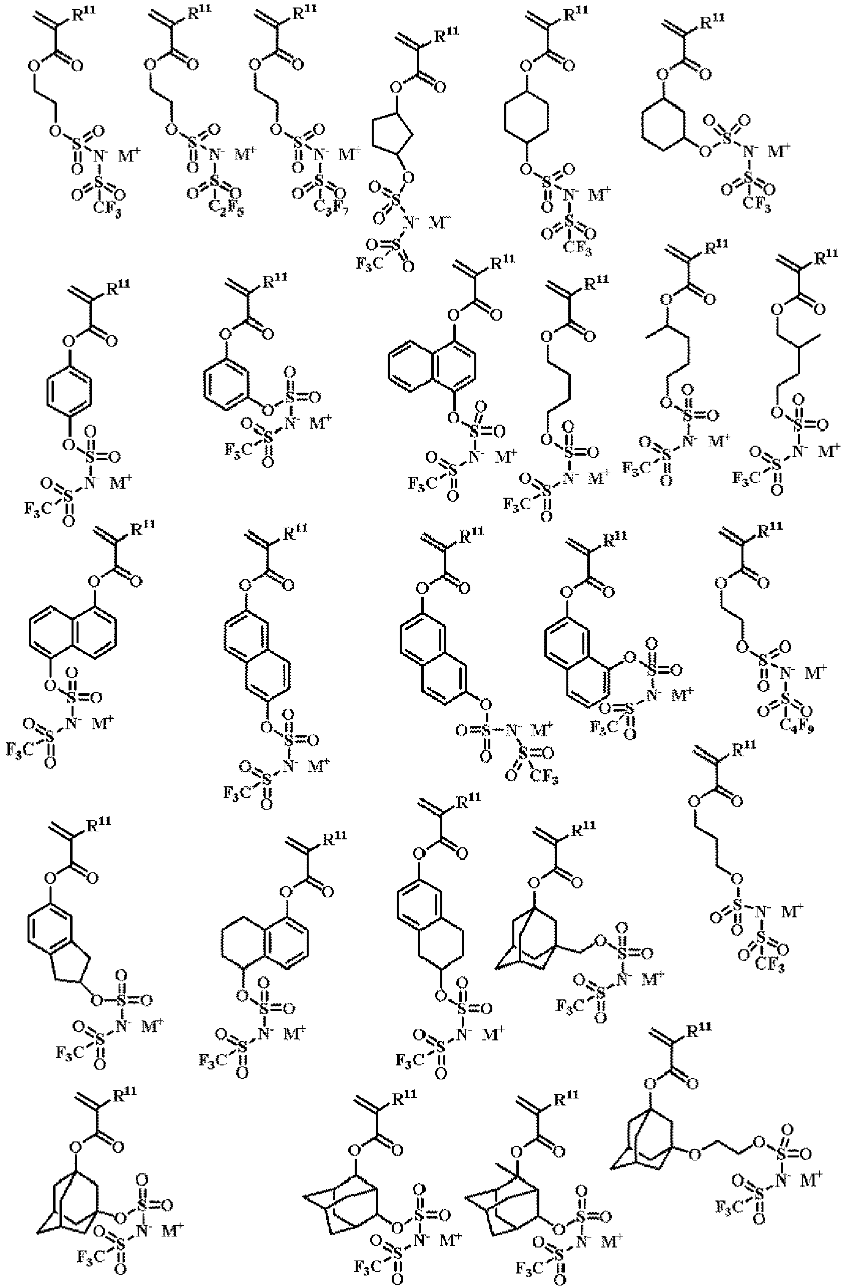

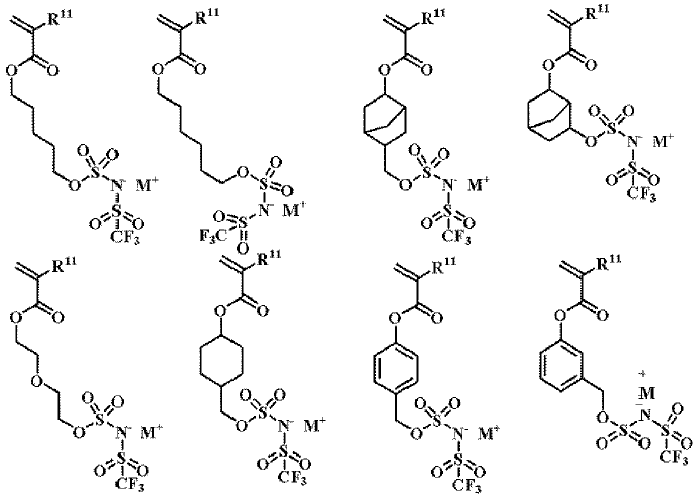

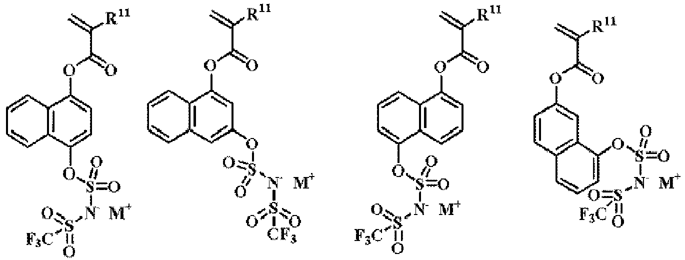

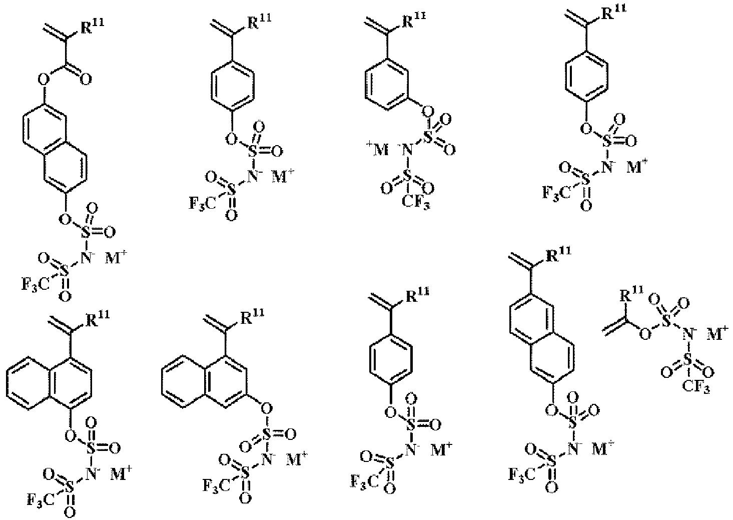

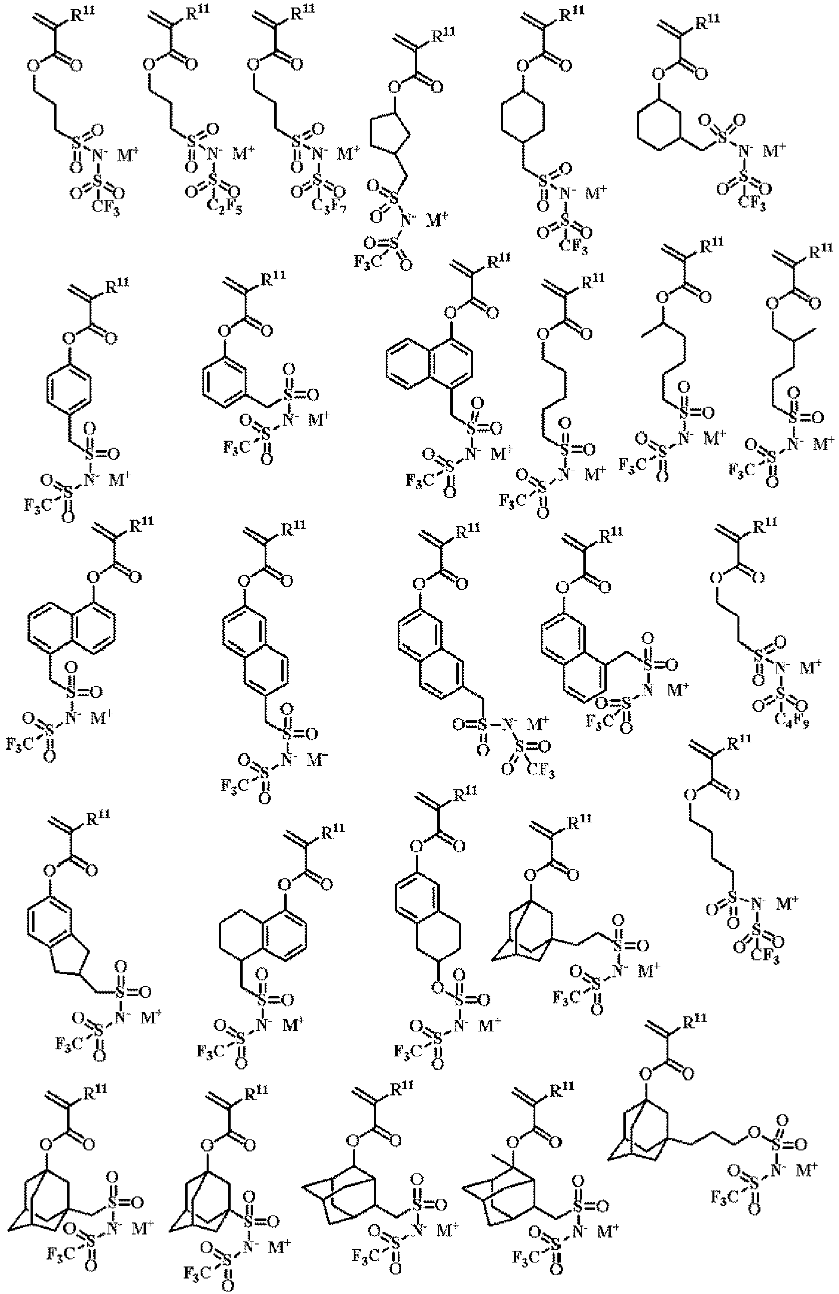

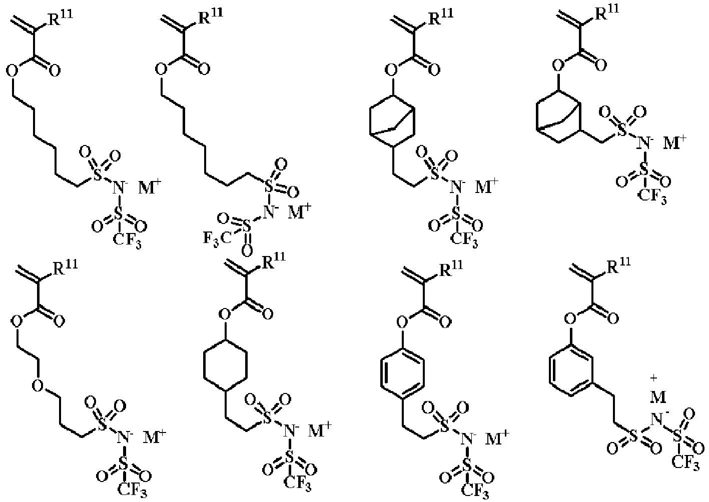

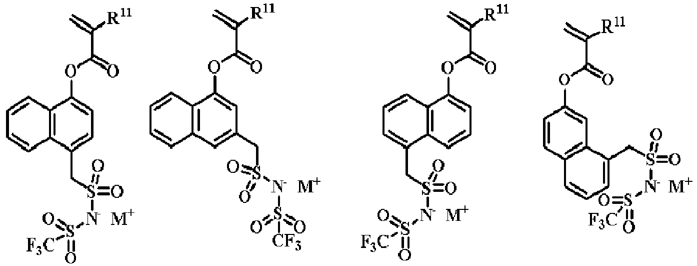

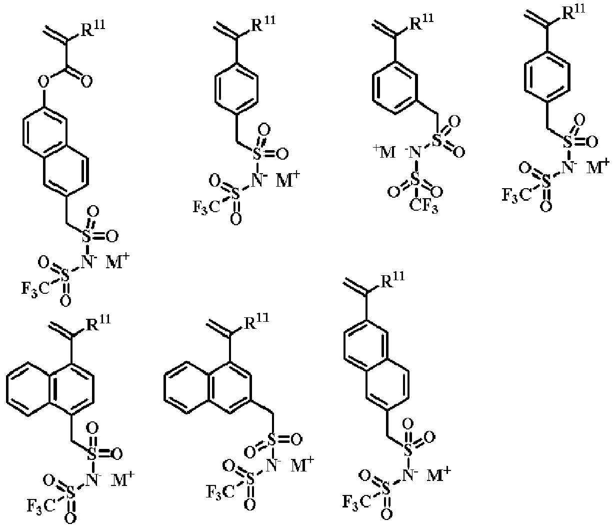

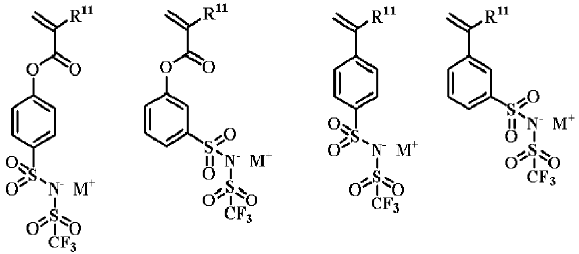

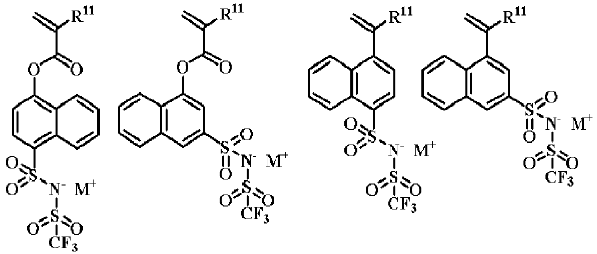

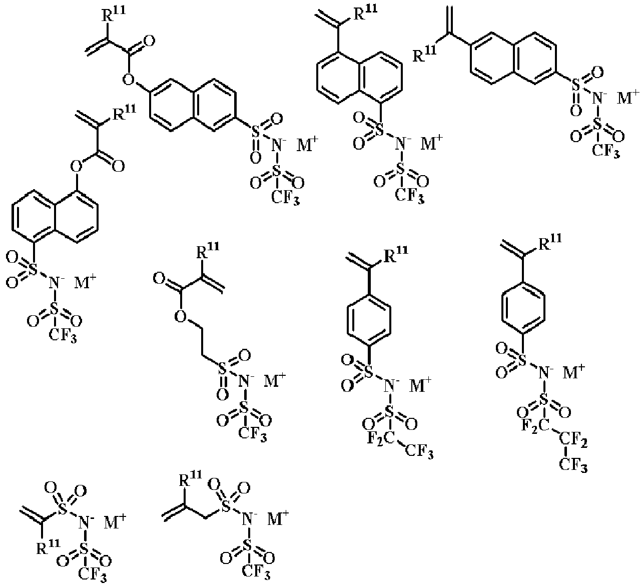

Specific examples of N-carbonyl-sulfonamide salt monomer to give the repeating unit-a7 in the above general formula can include the following.

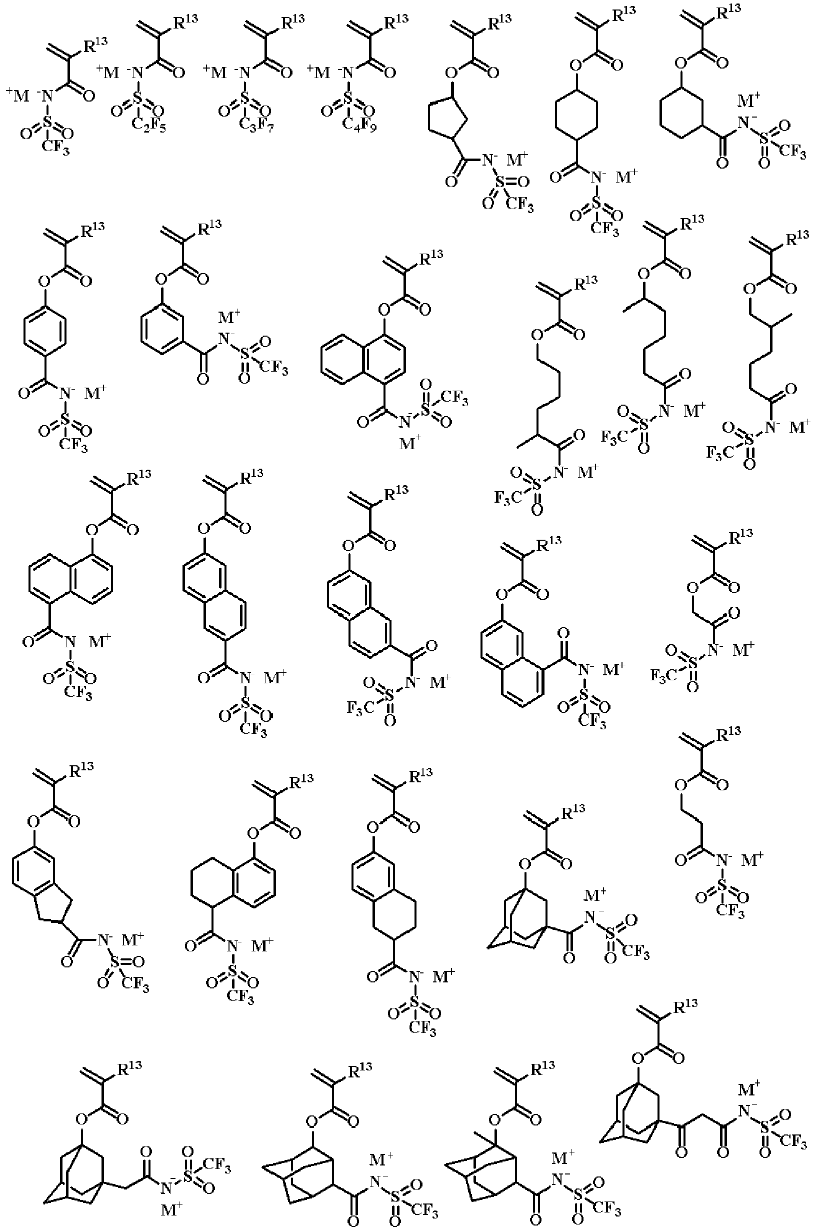

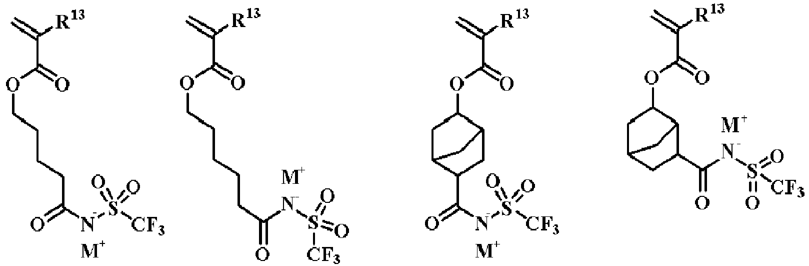

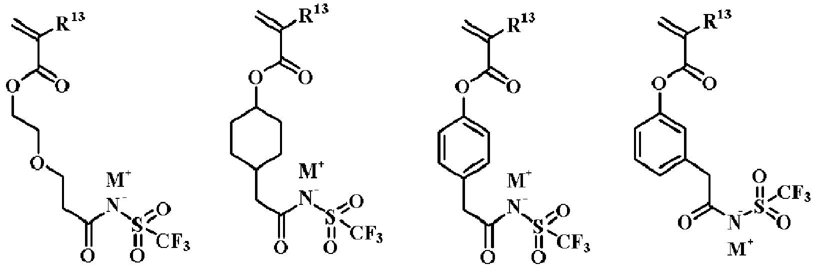

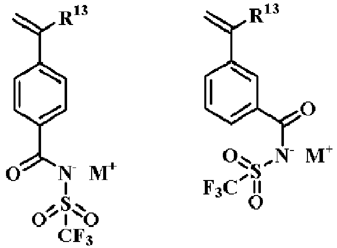

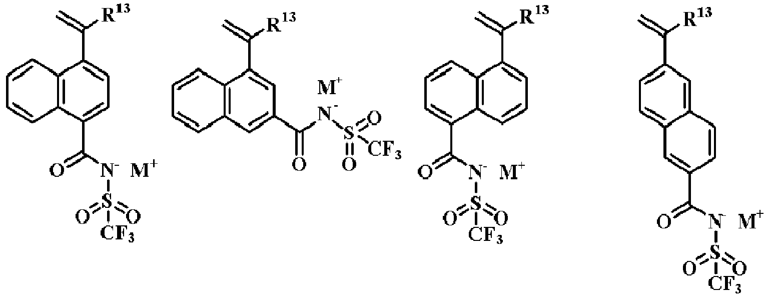

In the formulae, R¹, R³, R⁵, R⁸, R¹⁰, R¹¹, and R¹³ are as defined above.

The polymer compound (A) (component (A)) preferably contains an ammonium ion (ammonium cation) shown by the following general formula (3) as an ammonium ion for forming the ammonium salts, particularly as M⁺ in repeating units-a (e.g., the repeating units-al to -a7).

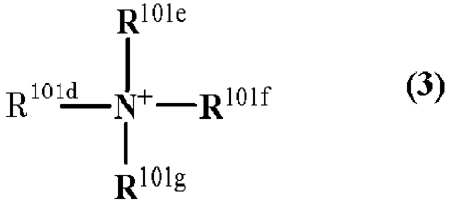

In the general formula (3), R^{101d}, R^{101e}, R^{101f}, and R^{101g} each represent a hydrogen atom, a linear, branched, or cyclic alkyl group having 1 to 14 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom. R^{101d} and R^{101e}, or R^{101d}, R^{101e} and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (3) within the ring.

Specific examples of the ammonium ion shown by the general formula (3) can include the following.

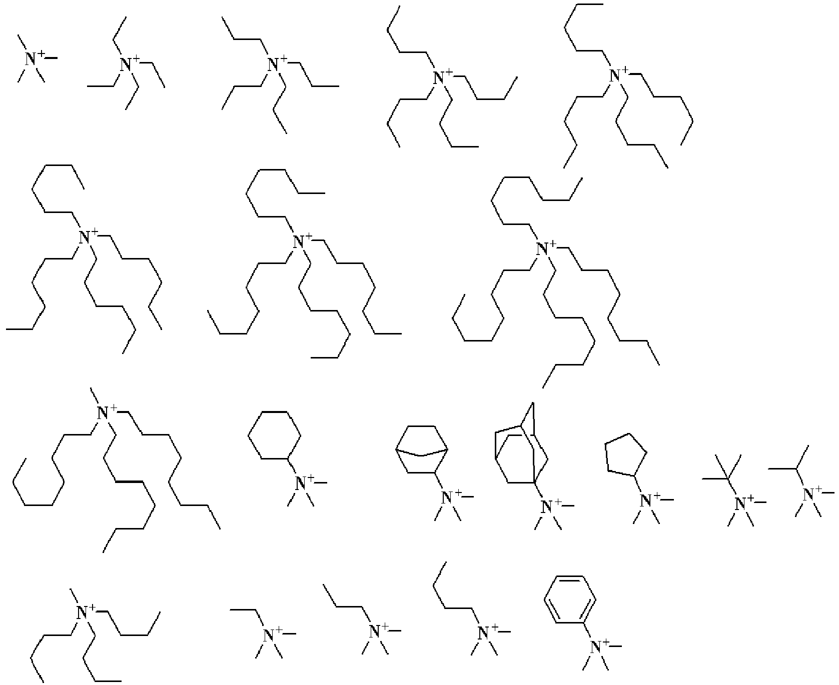

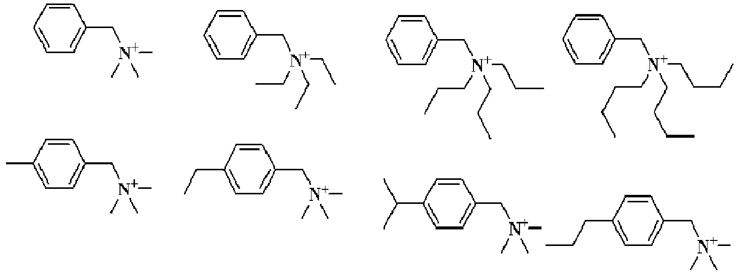

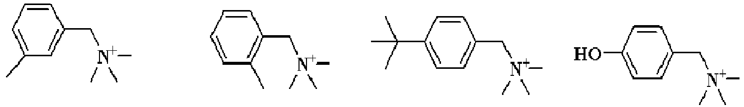

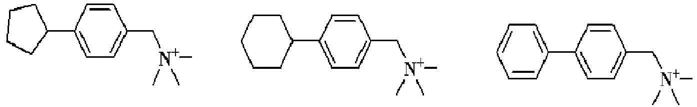

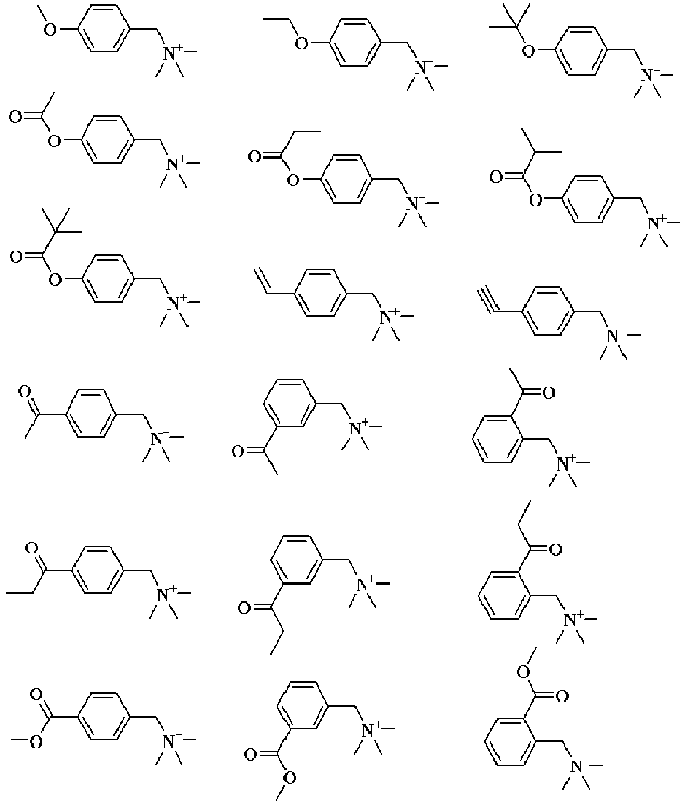

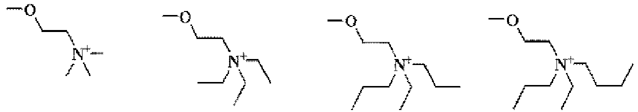

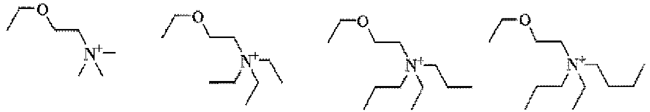

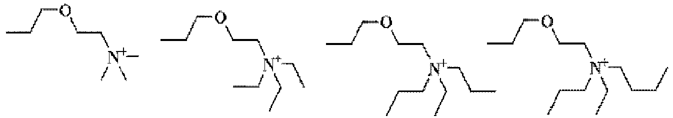

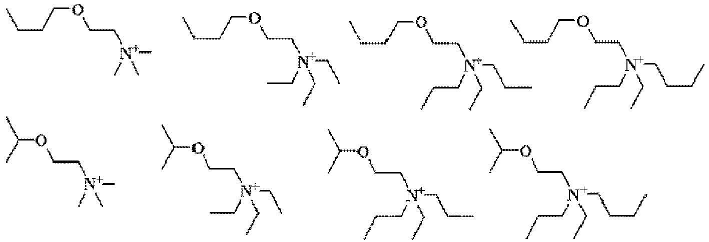

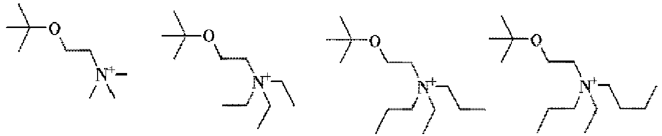

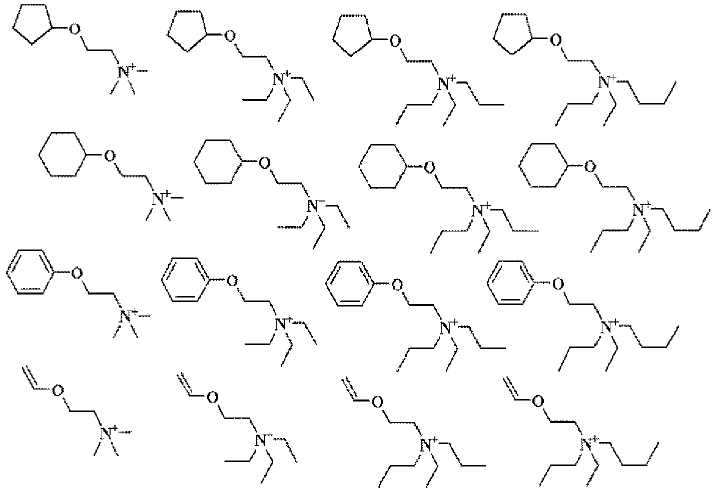

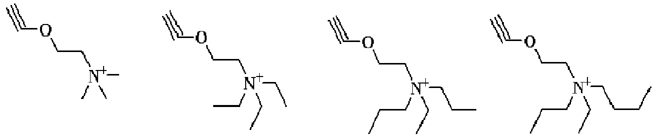

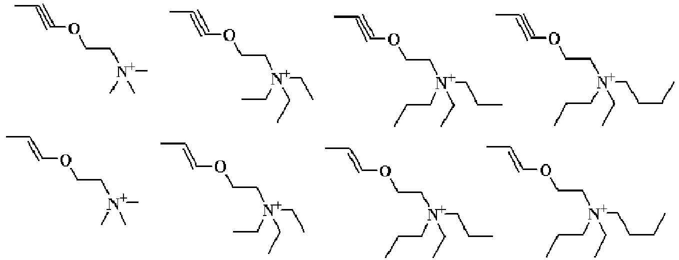

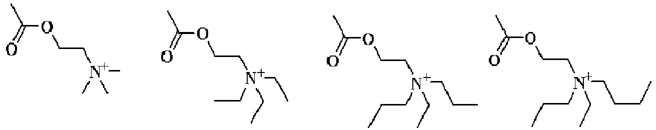

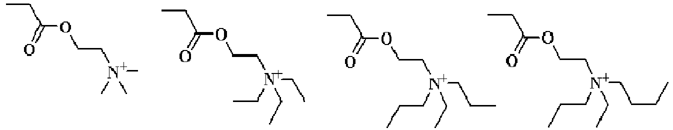

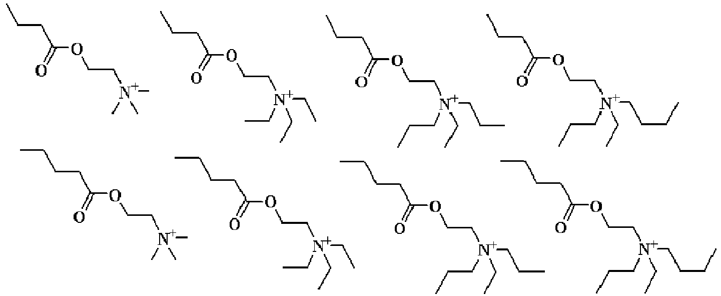

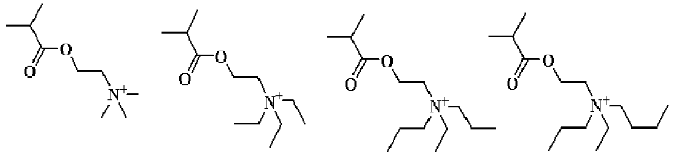

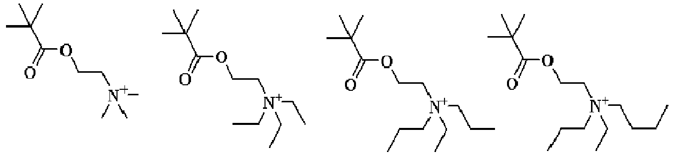

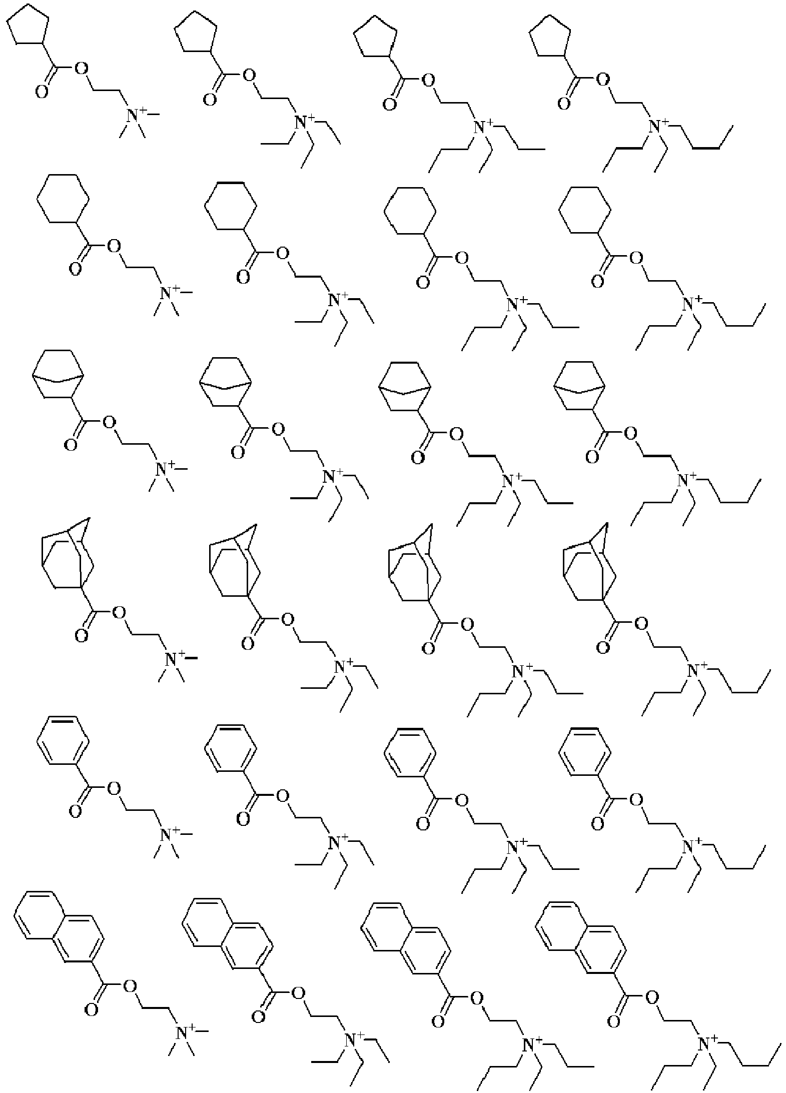

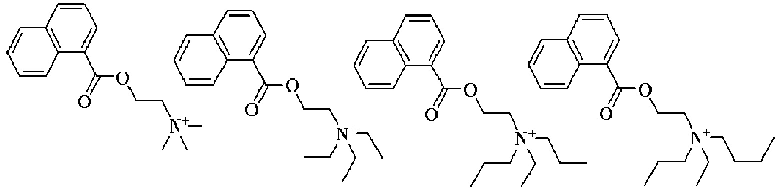

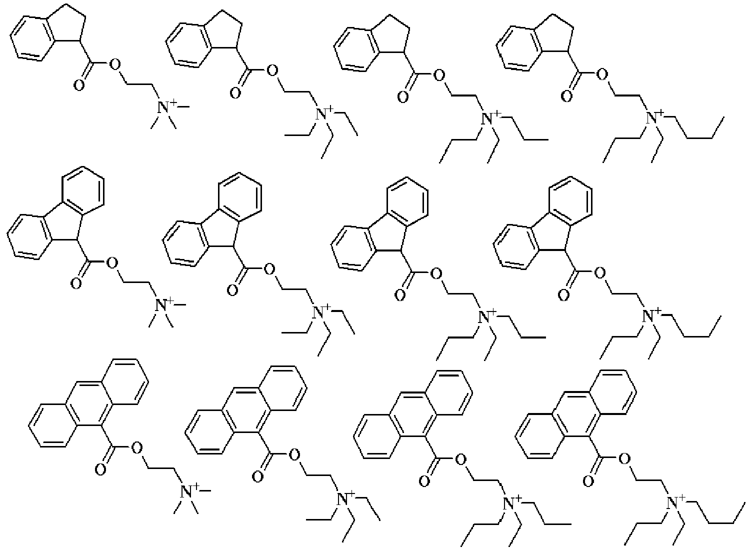

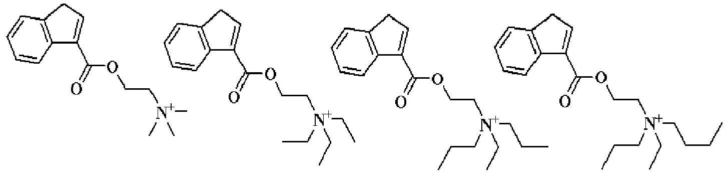

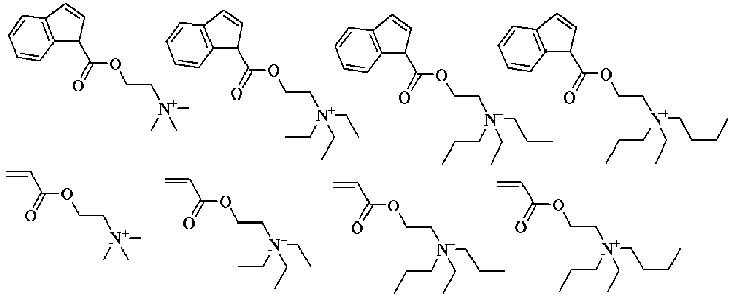

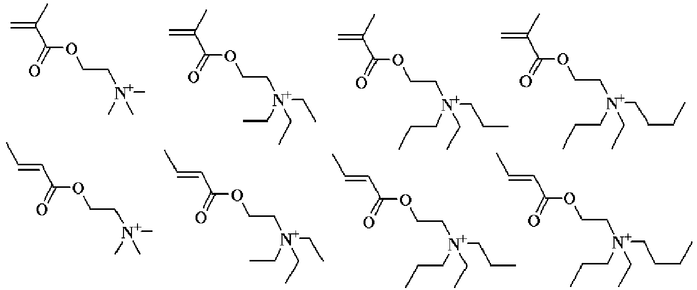

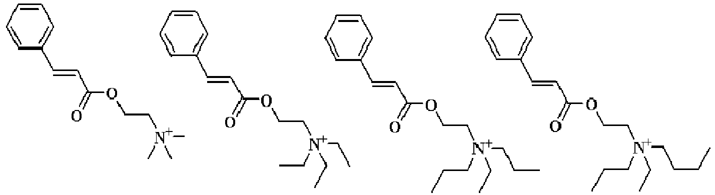

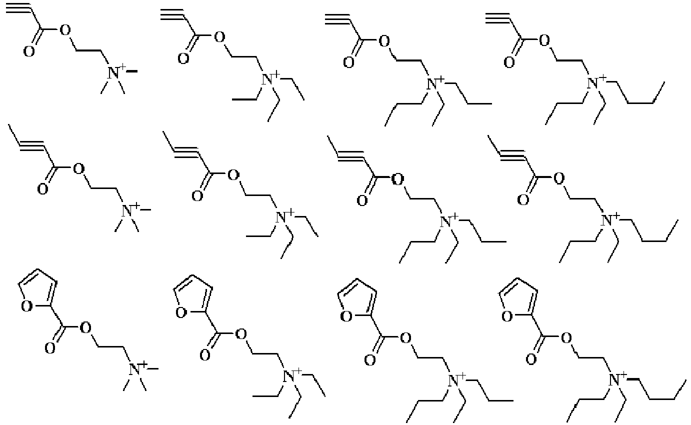

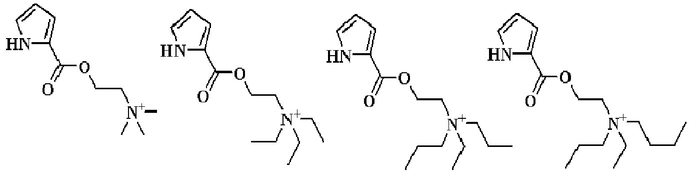

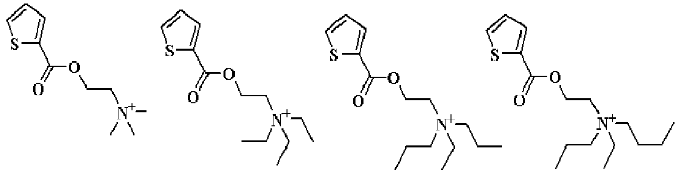

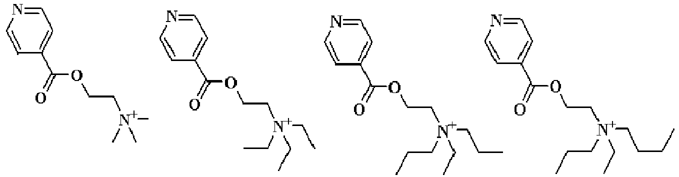

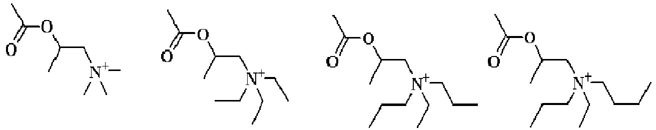

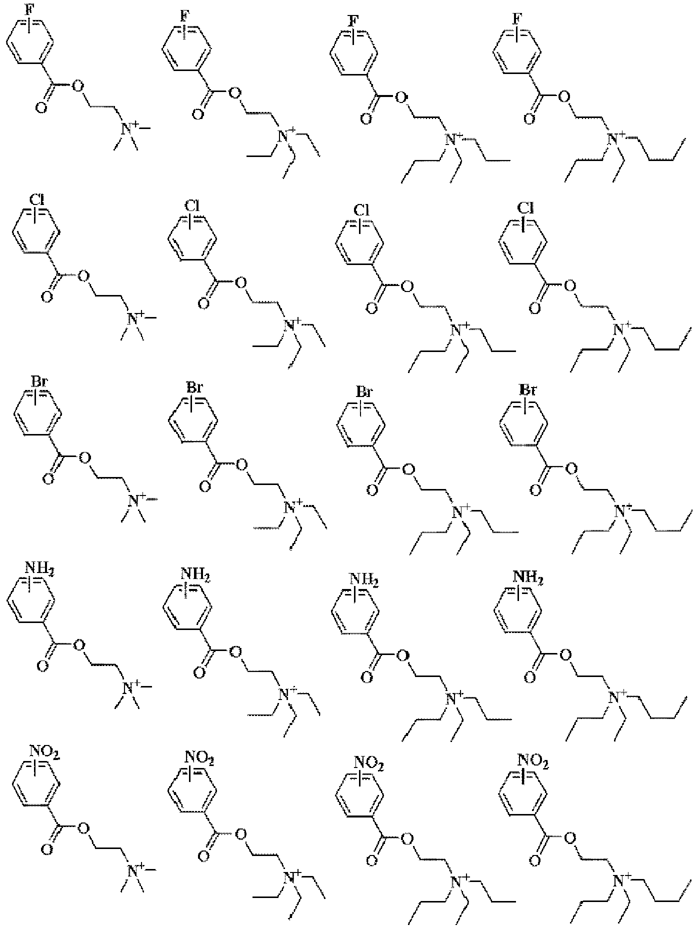

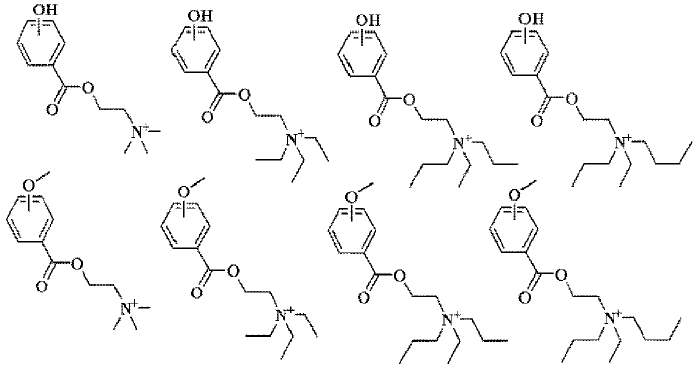

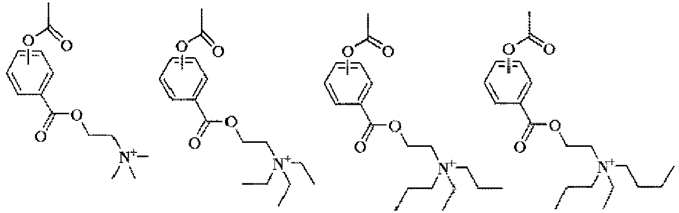

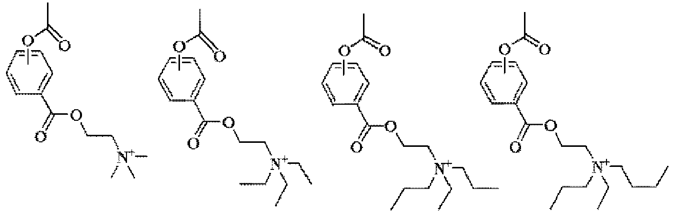

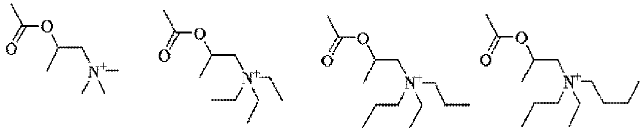

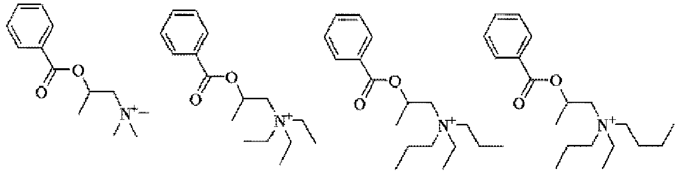

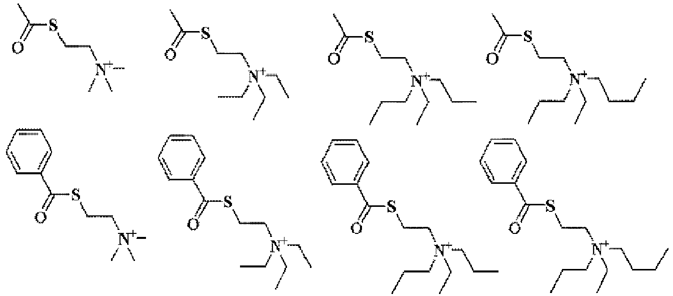

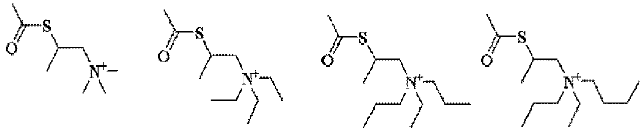

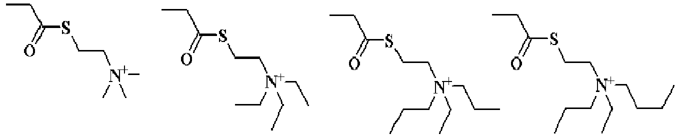

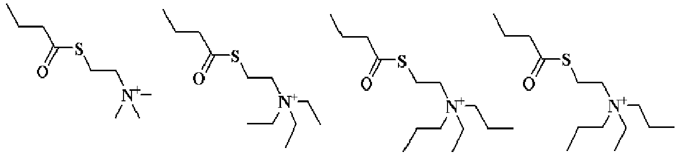

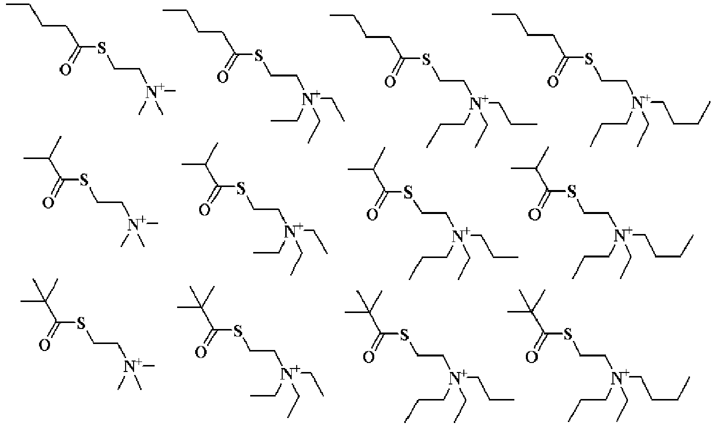

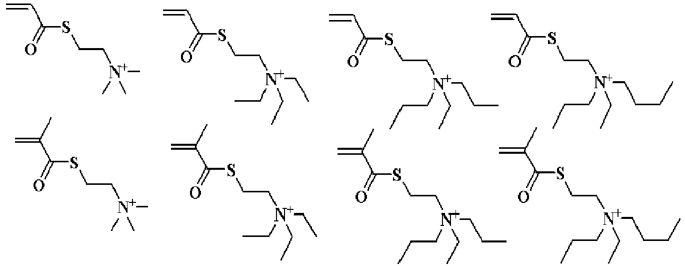

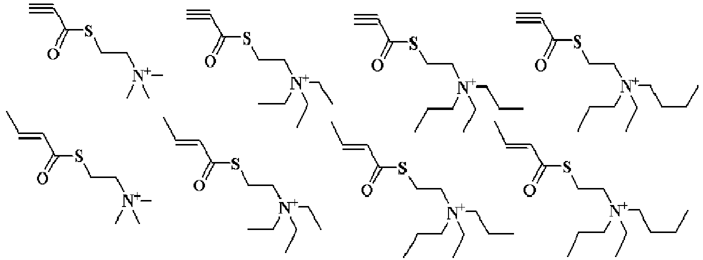

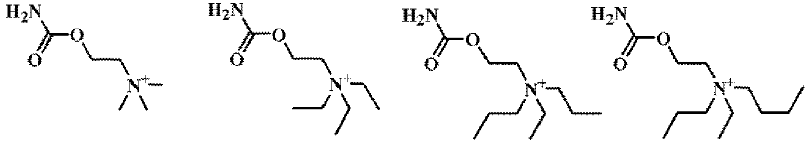

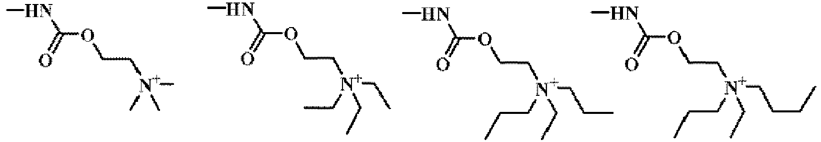

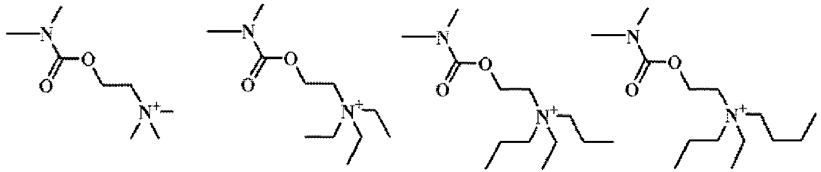

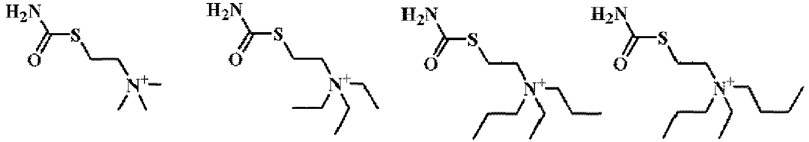

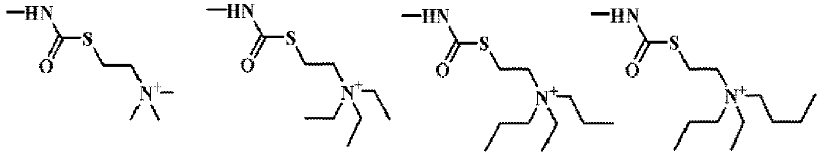

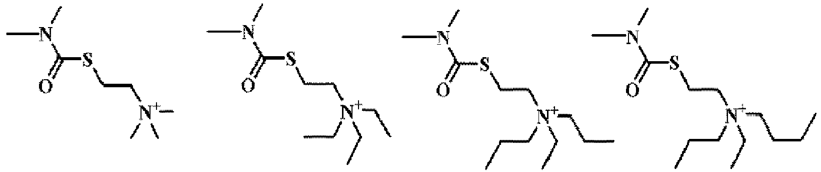

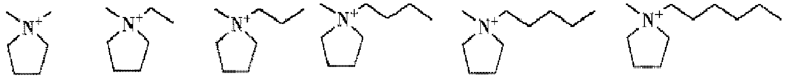

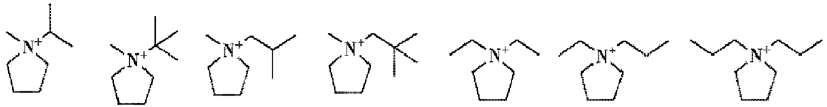

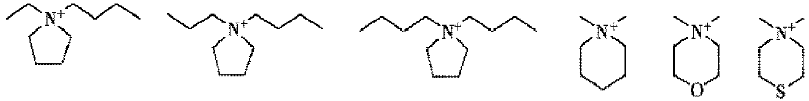

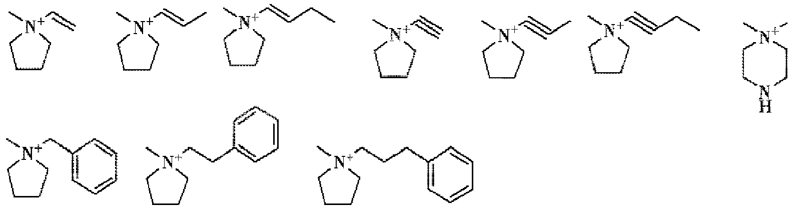

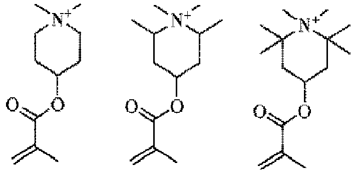

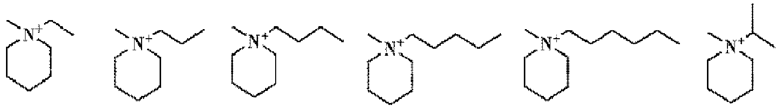

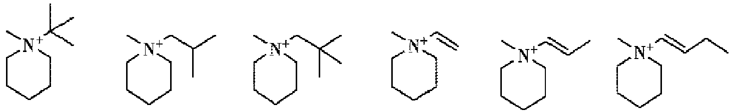

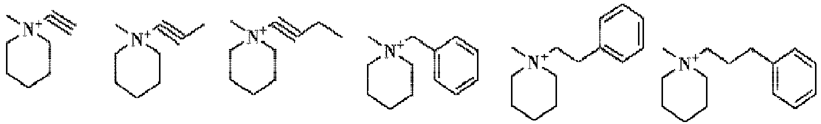

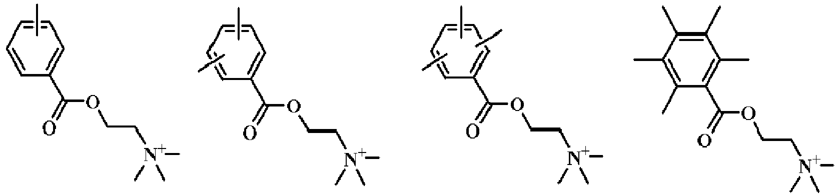

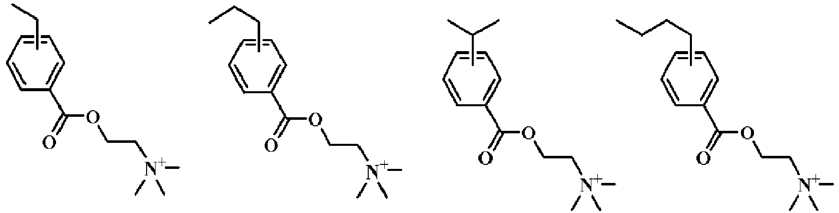

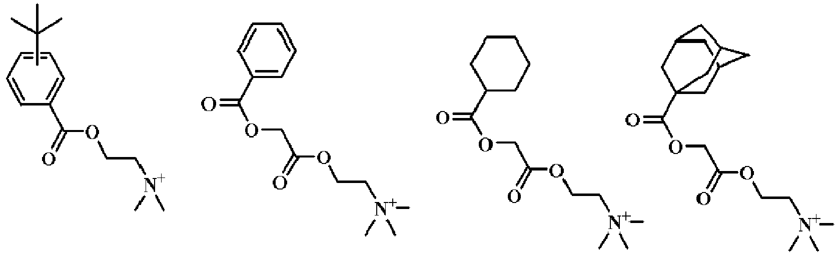

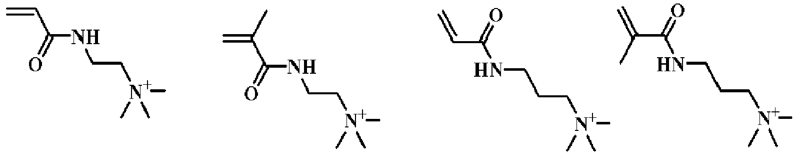

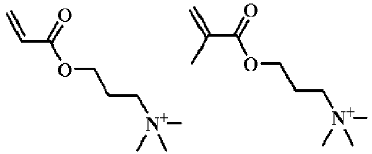

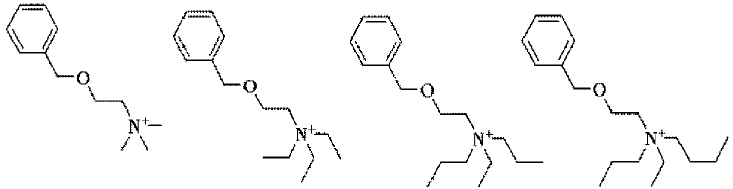

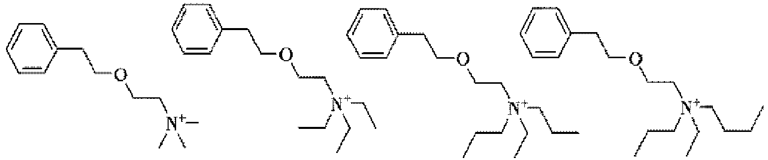

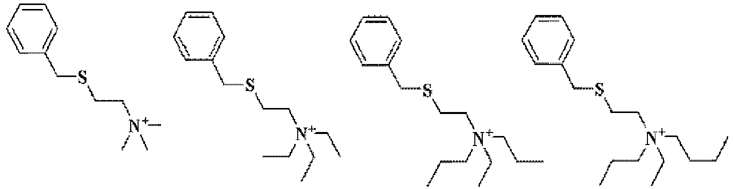

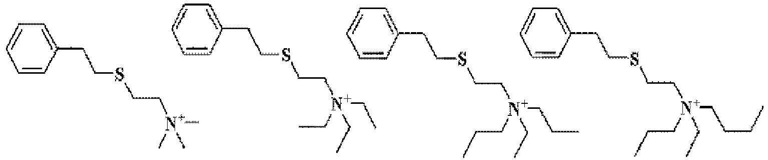

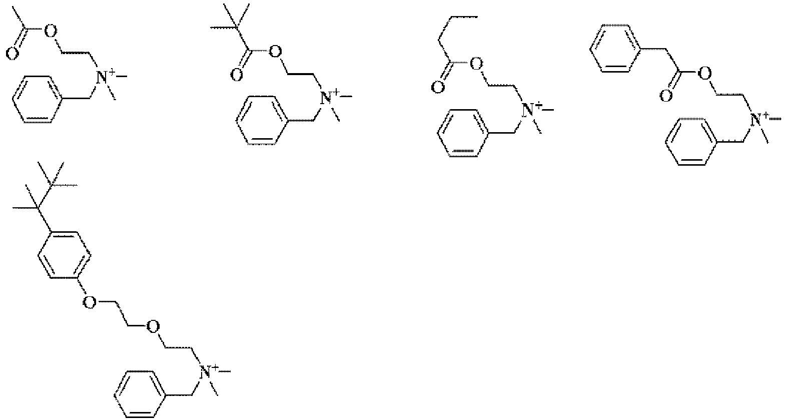

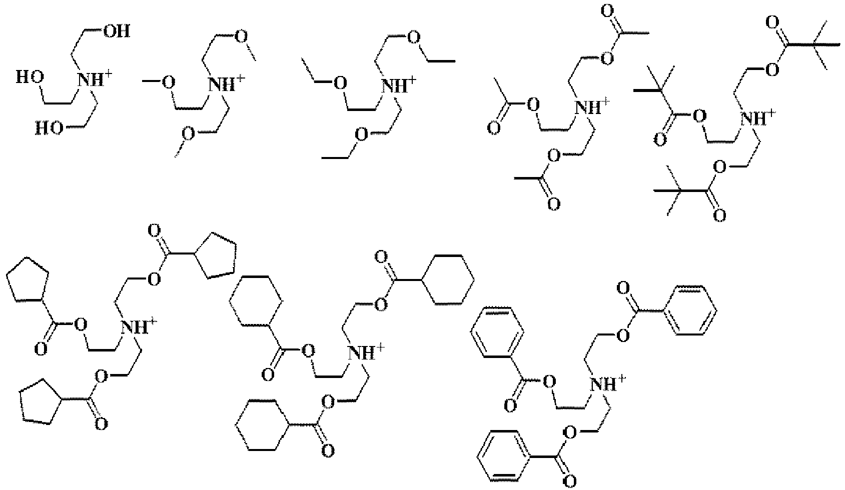

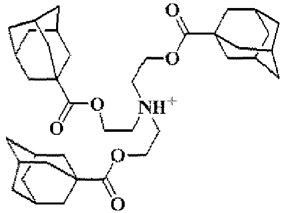

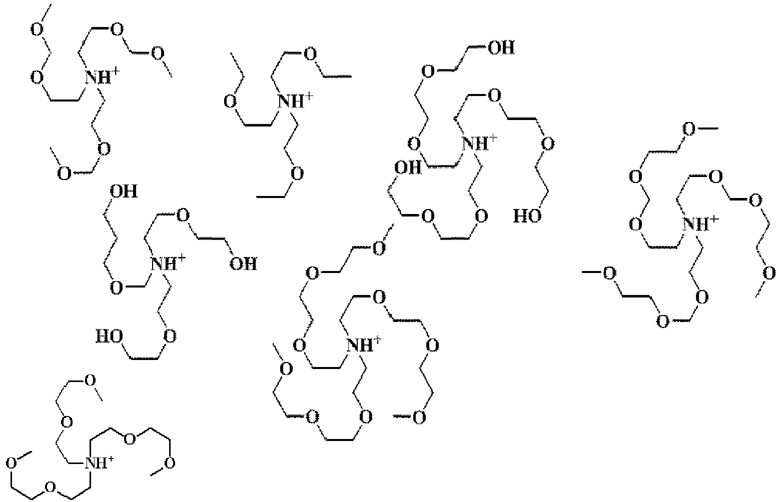

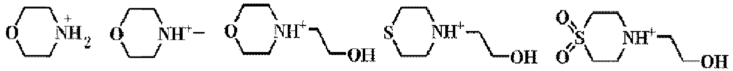

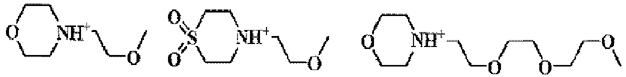

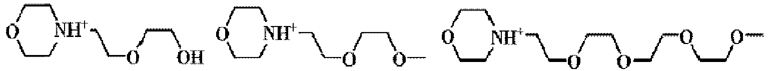

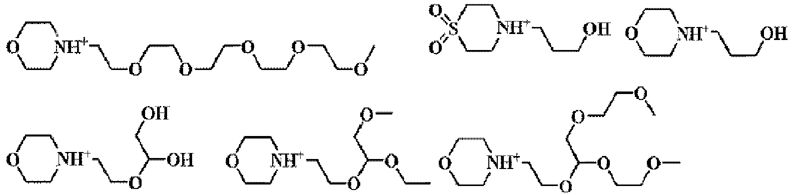

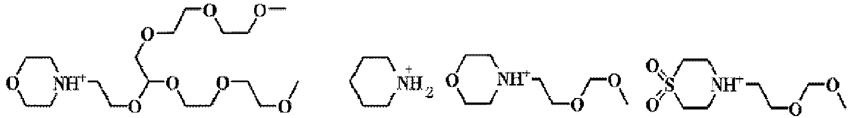

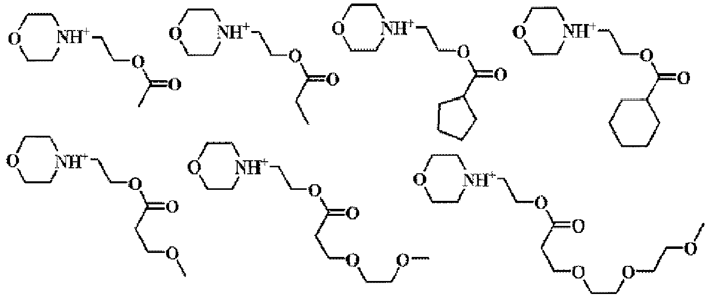

The ammonium ion shown by the general formula (3) is particularly preferably a tertiary or quaternary ammonium ion.

### (Repeating Unit-b)

In addition to the repeating units-al to -a7, a repeating unit-b having a glyme chain can also be copolymerized in the polymer compound (A) (component (A)) of the inventive bio-electrode composition in order to enhance the electric conductivity. Specific examples of a monomer to give the repeating unit-b having a glyme chain can include the following. The copolymerization with a repeating unit having a glyme chain facilitates the movement of ions released from skin in the living body contact layer (dry electrode film), and thus can increase the sensitivity of the bio-electrode (dry electrode).

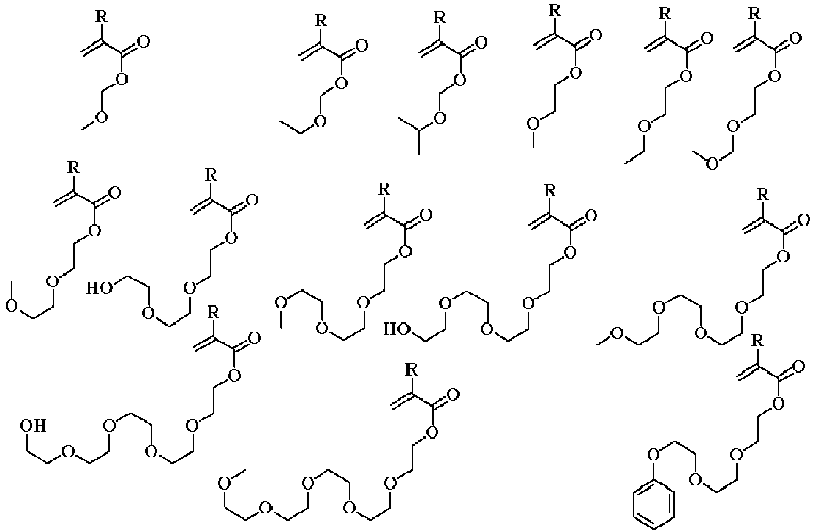

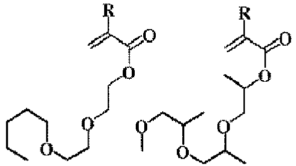

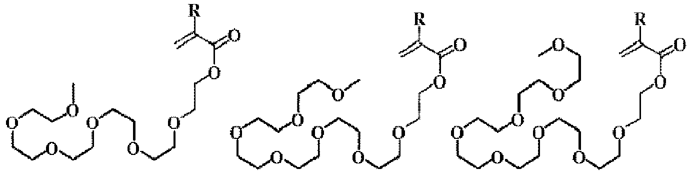

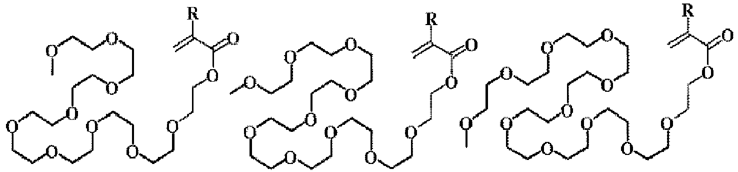

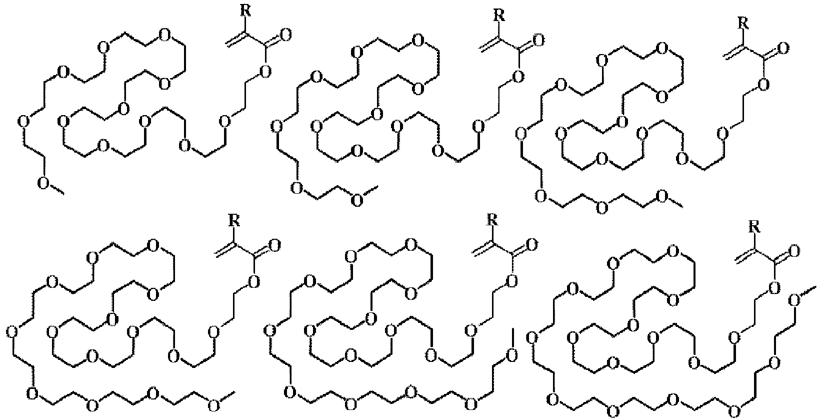

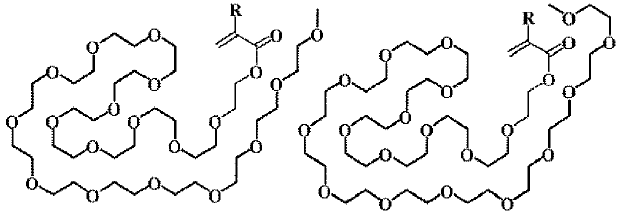

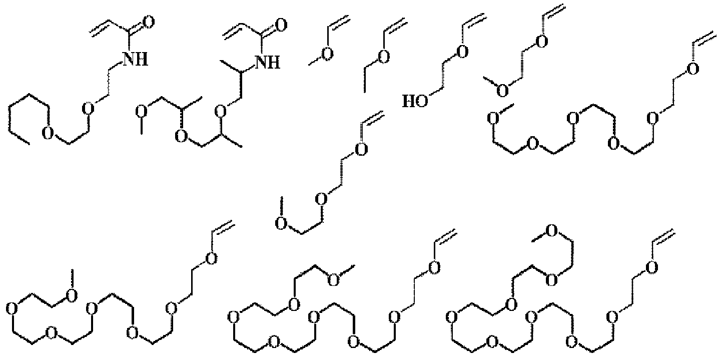

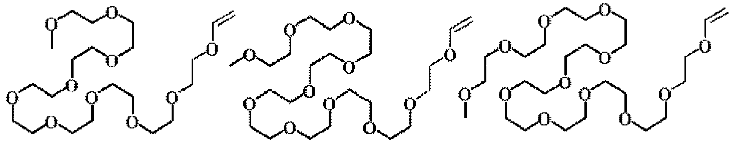

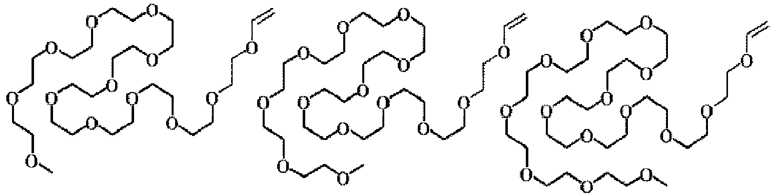

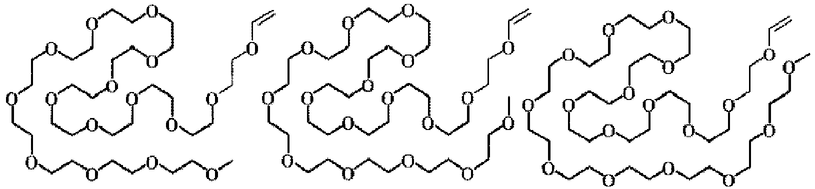

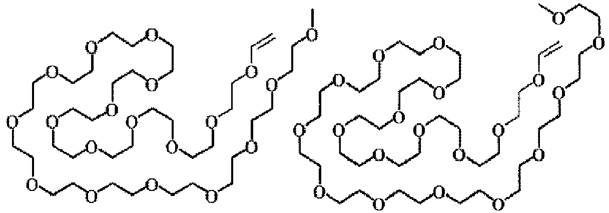

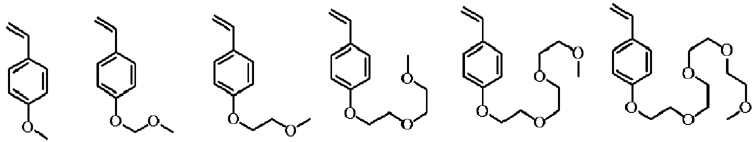

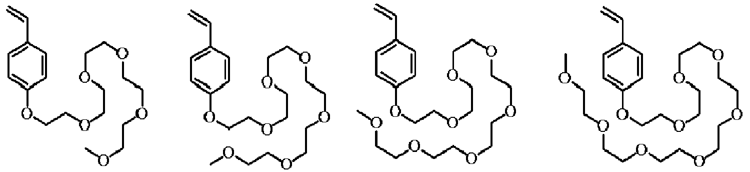

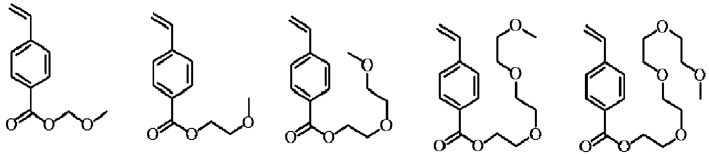

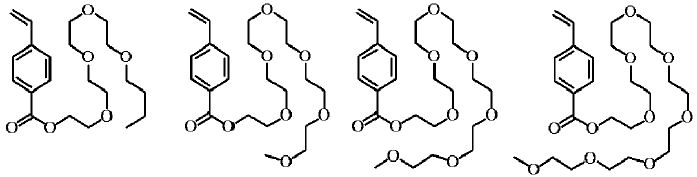

In the above formulae, R represents a hydrogen atom or a methyl group.

### (Repeating Unit-c)

Besides the repeating units-al to -a7 and the optional repeating unit-b, a hydrophilic repeating unit-c having a hydrophilic group, such as a hydroxy group, a carboxyl group, an ammonium salt, a betaine, an amide group, pyrrolidone, a lactone ring, a lactam ring, a sultone ring, a sodium salt of sulfonic acid, or a potassium salt of sulfonic acid, can also be copolymerized in the polymer compound (A) (component (A)) of the inventive bio-electrode composition in order to enhance the electric conductivity. Specific examples of a monomer to give the hydrophilic repeating unit-c can include the following. The copolymerization with repeating units containing such hydrophilic groups can increase the sensitivity of the bio-electrode (dry electrode) by increasing the sensitivity to ions released from skin.

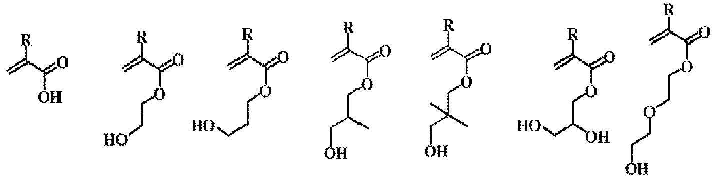

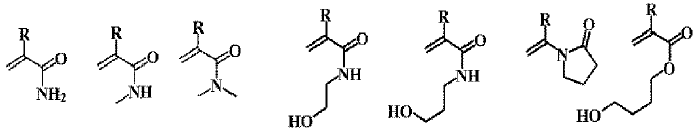

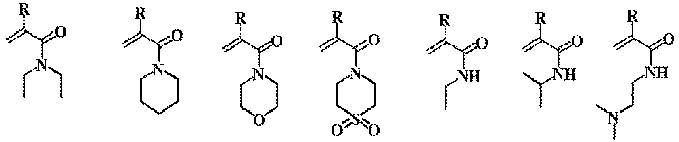

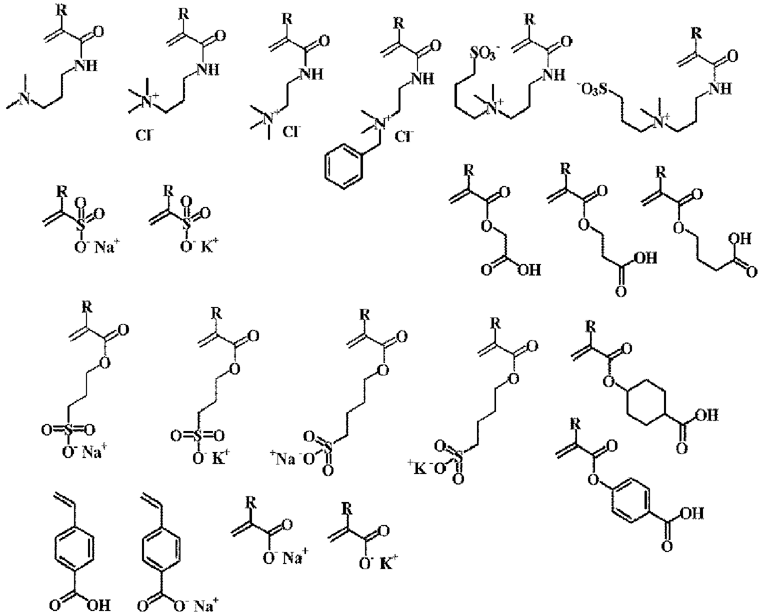

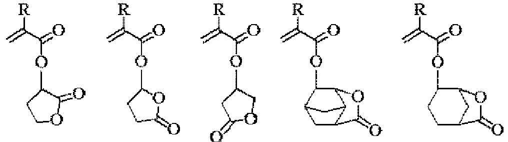

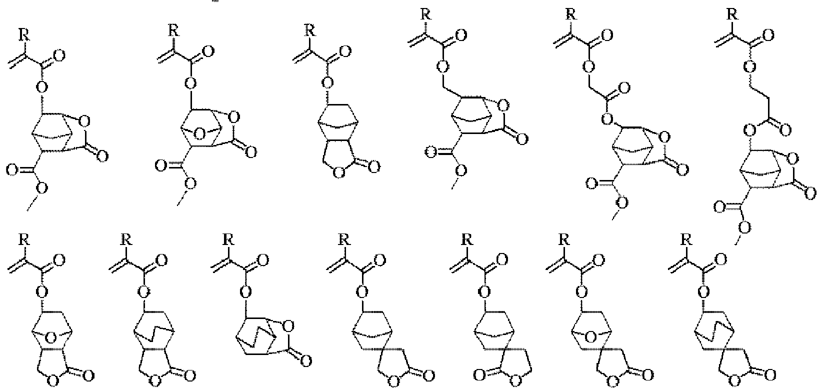

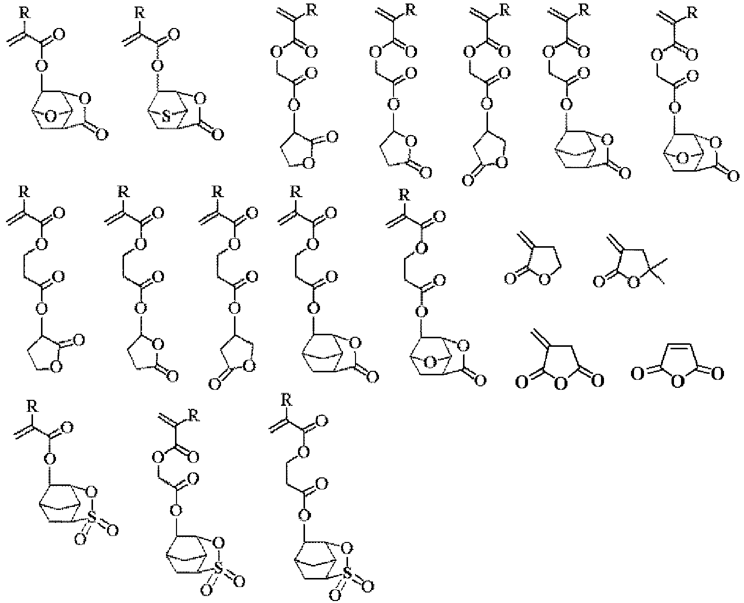

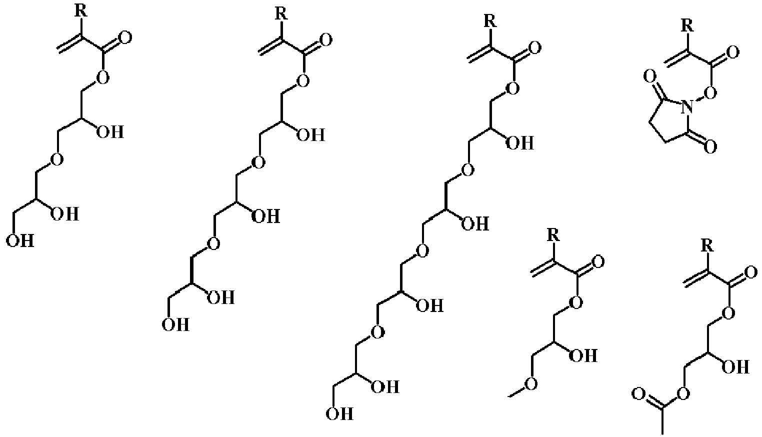

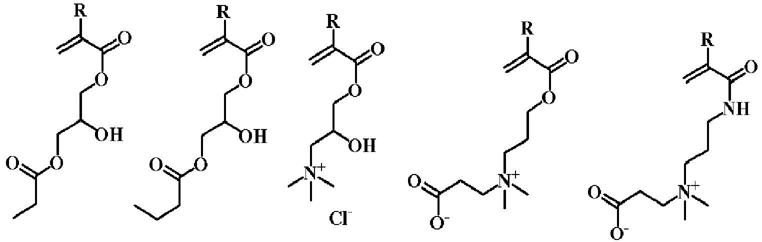

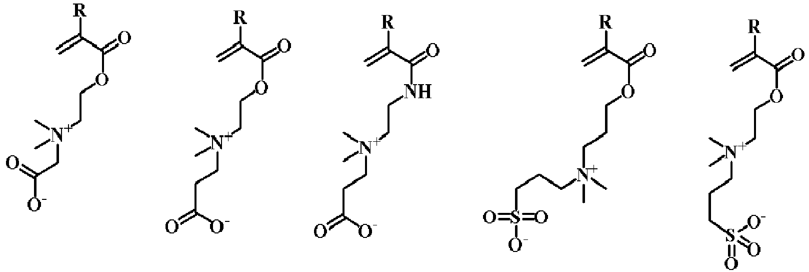

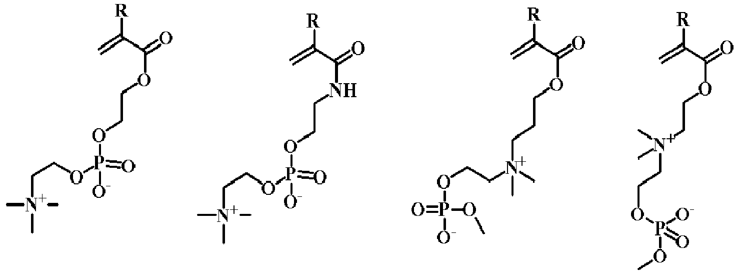

In the above formulae, R represents a hydrogen atom or a methyl group.

### (Repeating Unit-d)

The polymer compound (A) in the inventive bio-electrode composition can have a repeating unit-d to impart adhesion properties. Specific examples of a monomer to give the repeating unit-d can include the following.

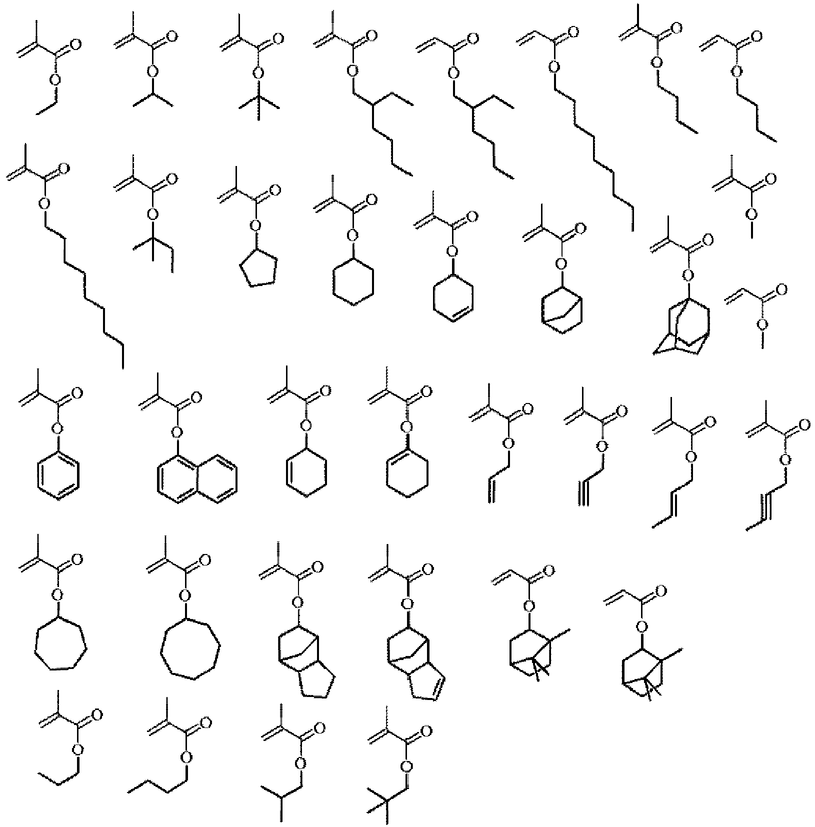

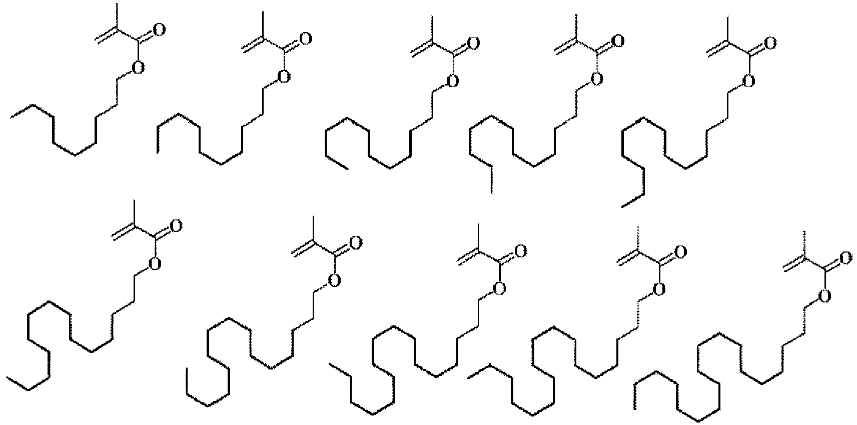

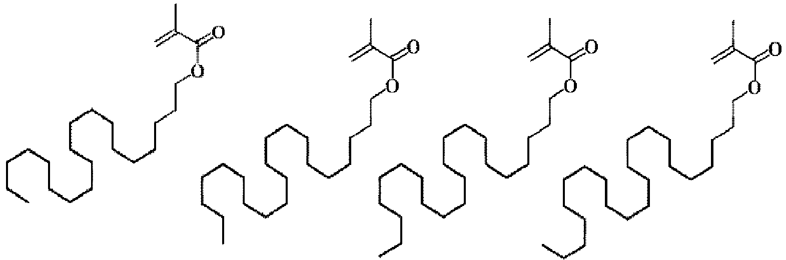

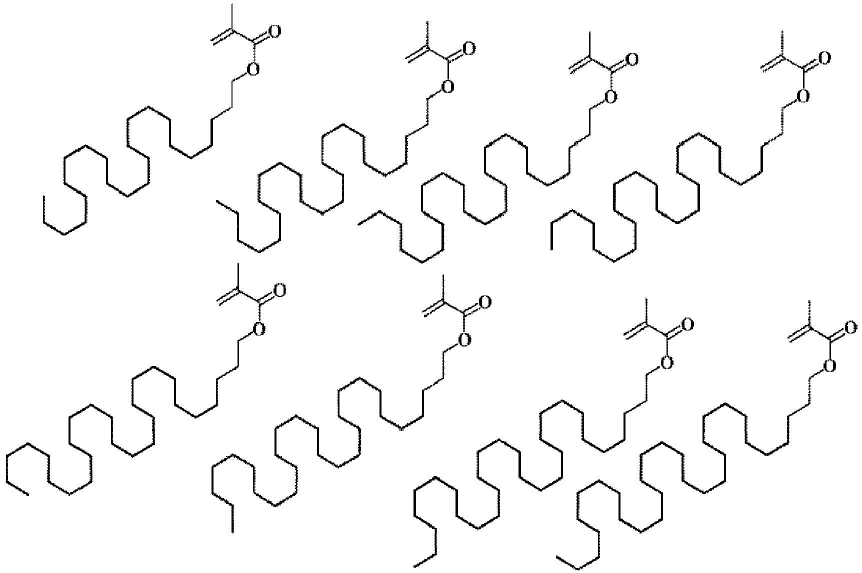

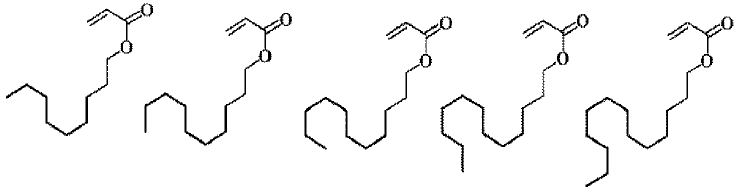

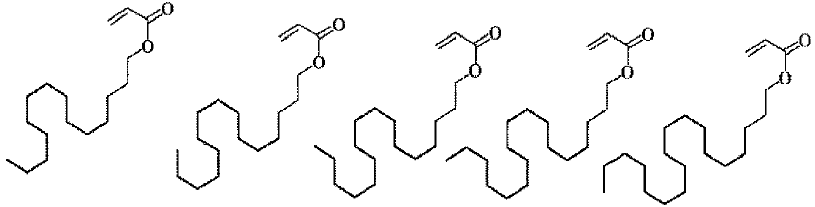

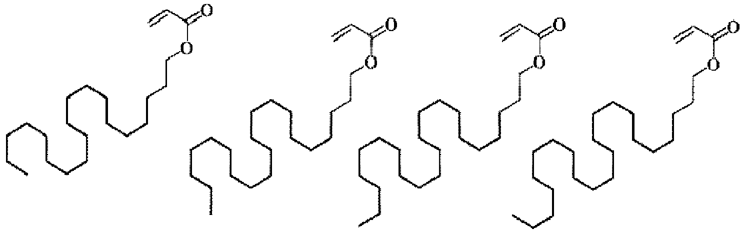

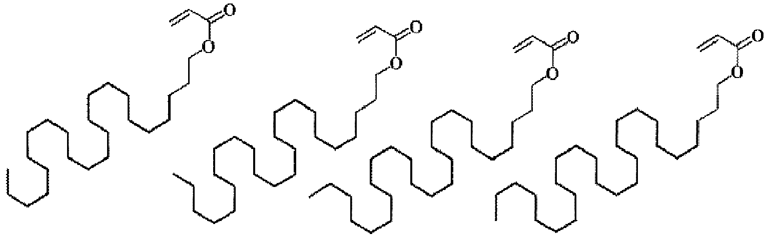

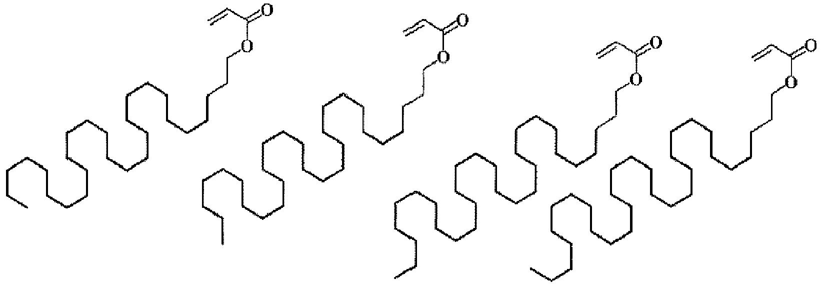

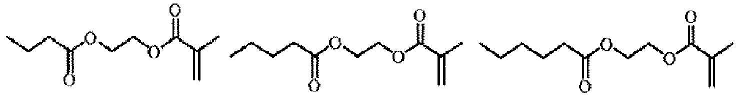

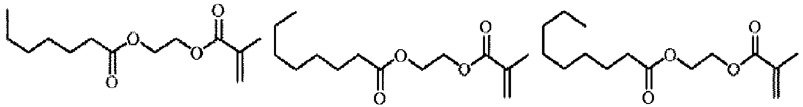

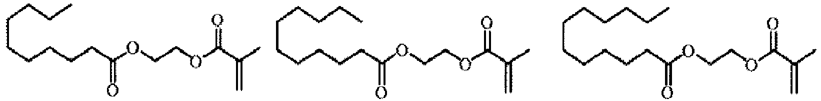

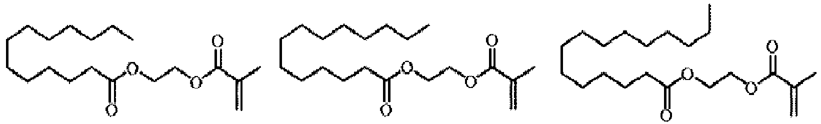

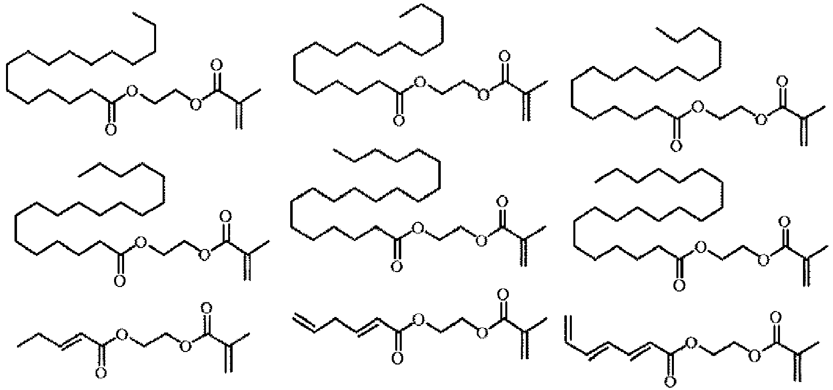

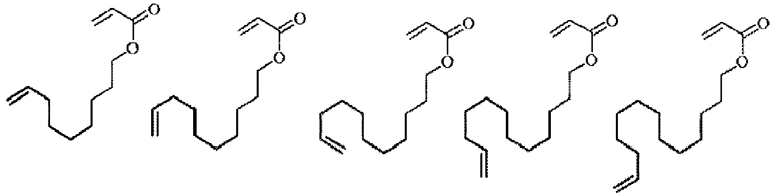

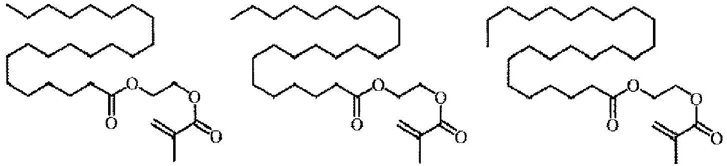

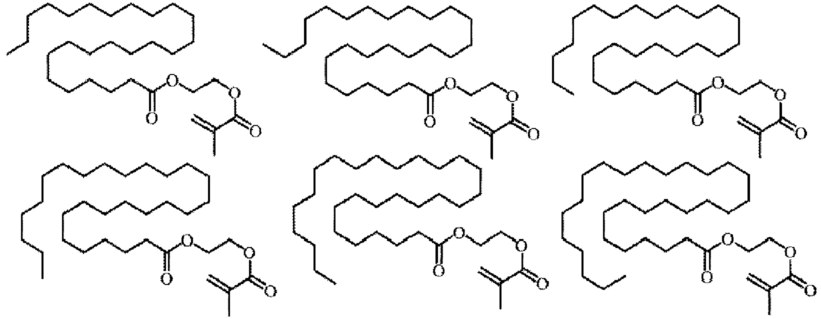

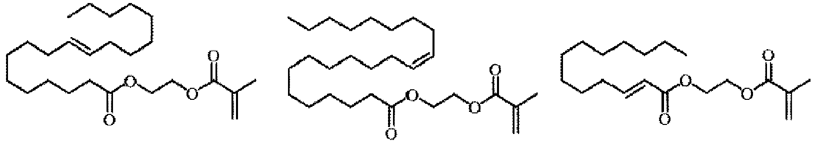

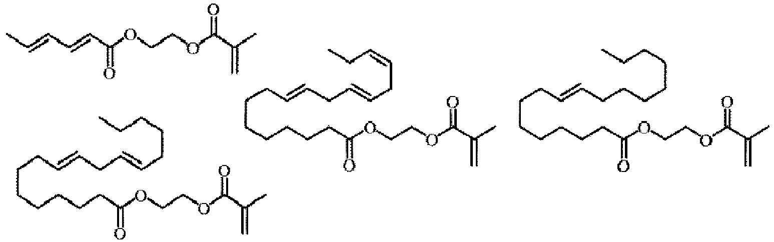

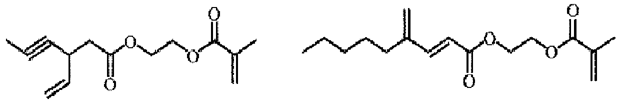

### (Repeating Unit-e)

Further, it is also possible to copolymerize a crosslinkable repeating unit-e. Examples of the crosslinkable repeating unit-e include repeating units having an oxirane ring or an oxetane ring.

Specific examples of monomers to give the repeating unit-e having an oxirane ring or an oxetane ring can include the following.

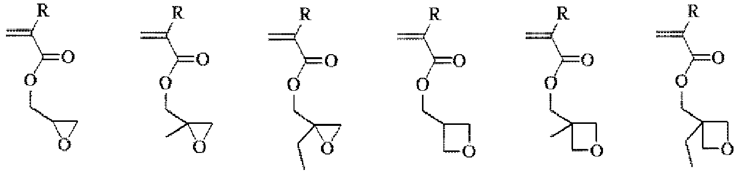

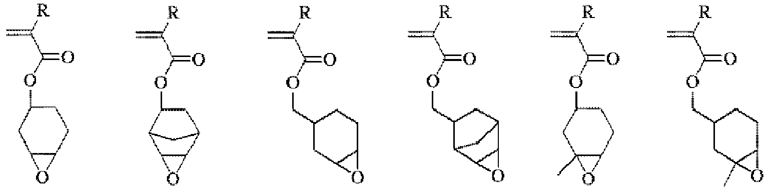

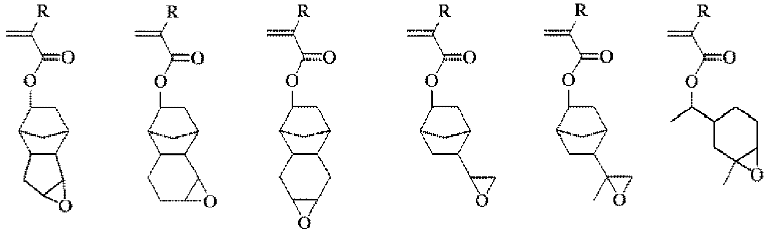

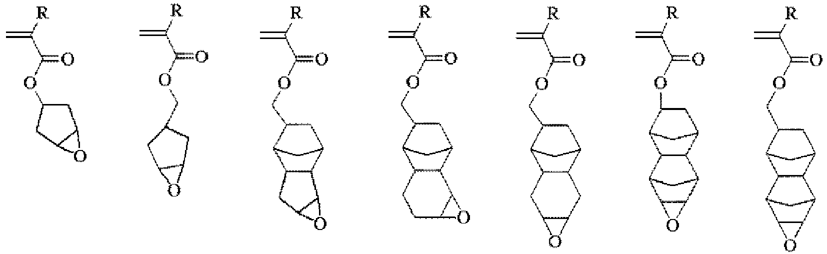

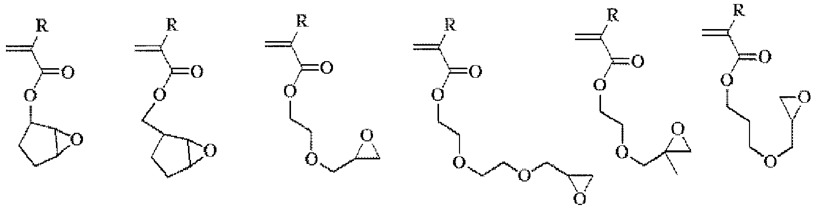

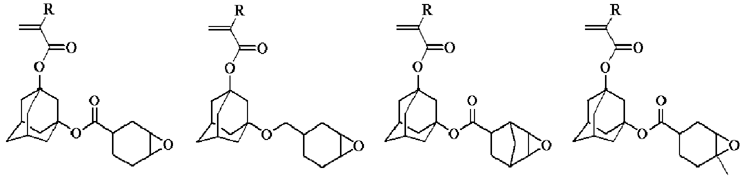

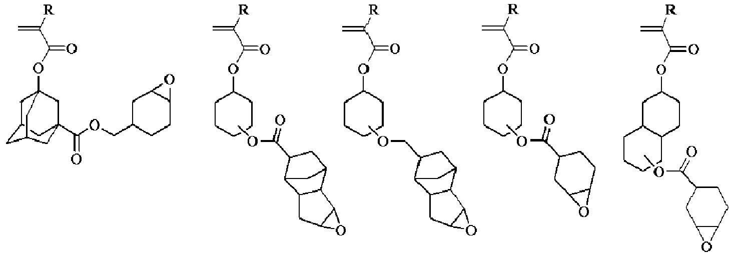

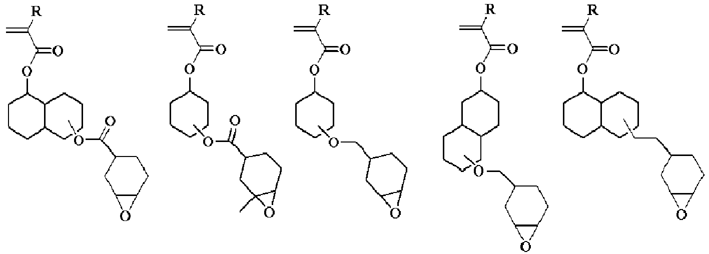

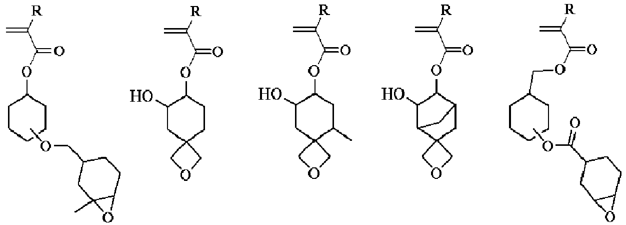

In these formulae, R represents a methyl group or a hydrogen atom.

### (Repeating Unit-f)

The polymer compound (A) (component (A)) of the inventive bio-electrode composition can have a repeating unit-f having silicon, besides the repeating units-al to -a7 and the optional repeating unit(s) selected from -b to -e. Specific examples of a monomer to give the repeating unit-f can include the following.

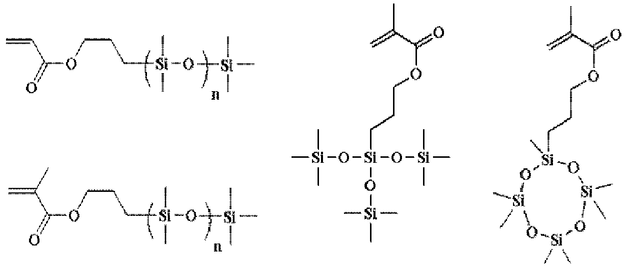

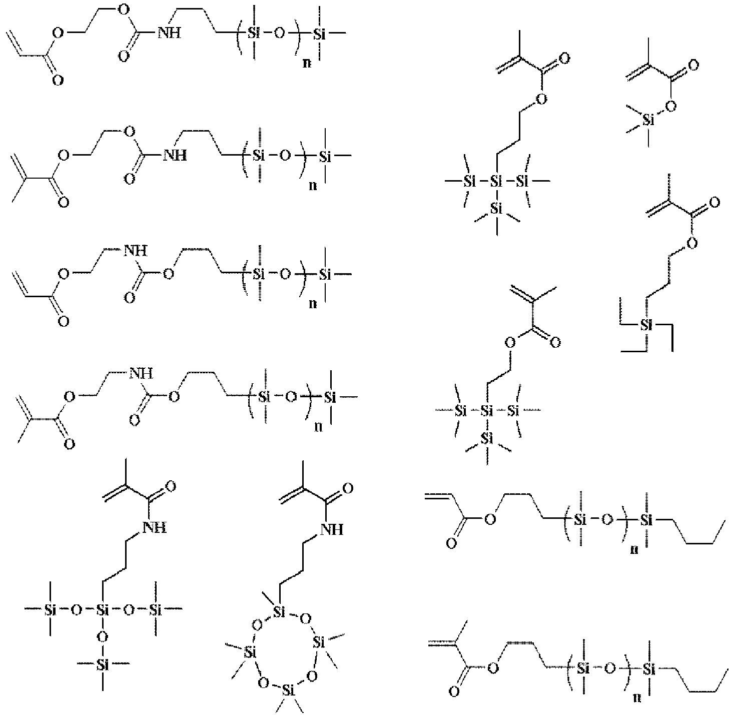

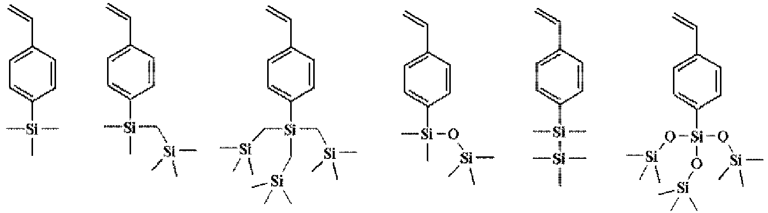

"n" represents an integer of 0 to 100.

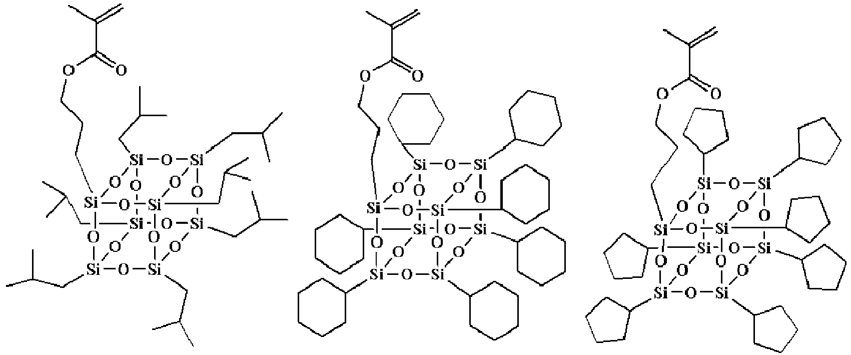

### (Repeating Unit-g)

The polymer compound (A) (component (A)) of the inventive bio-electrode composition can have a repeating unit-g having fluorine, besides the repeating units-al to -a7 and the optional repeating unit(s) selected from -b to -f.

Specific examples of a monomer to give the repeating unit-g having fluorine can include the following.

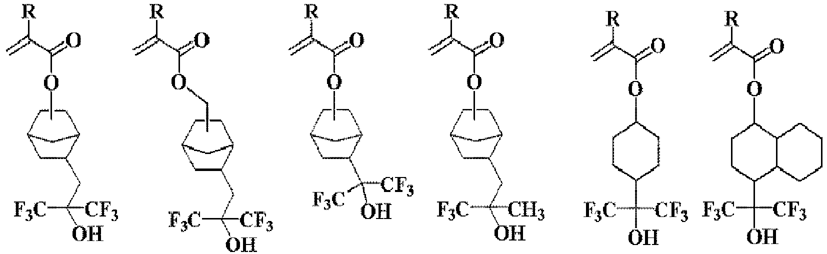

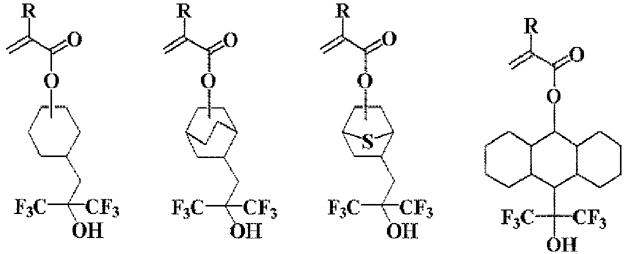

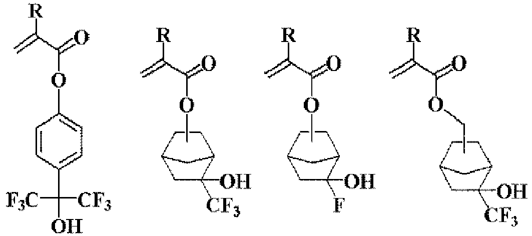

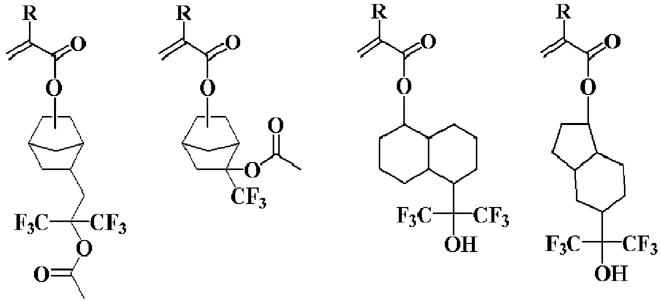

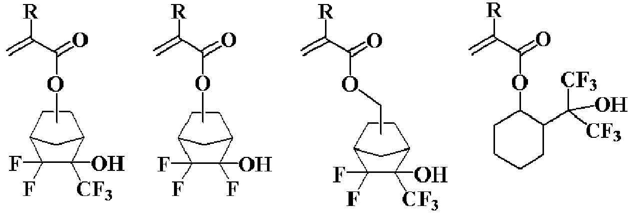

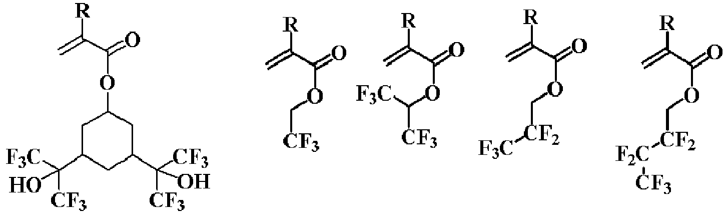

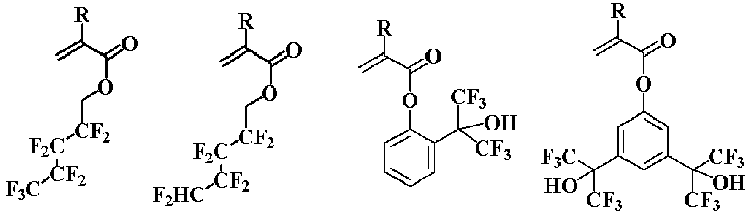

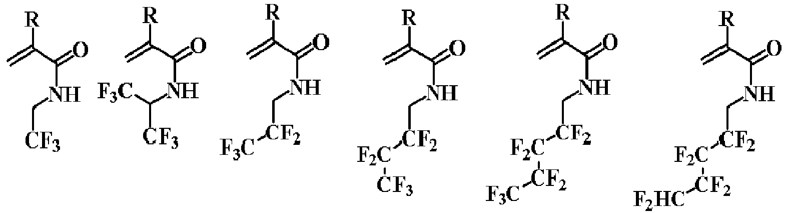

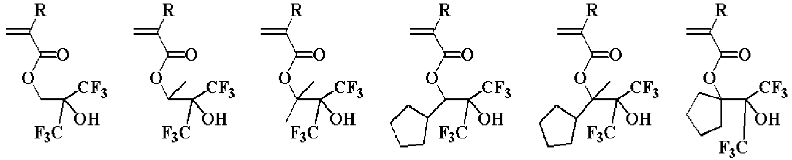

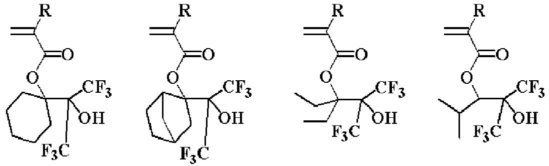

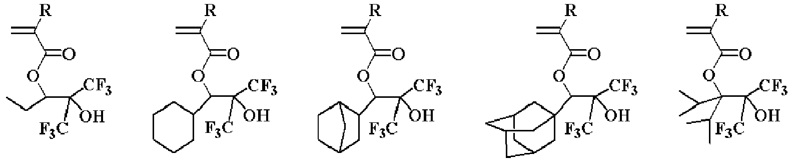

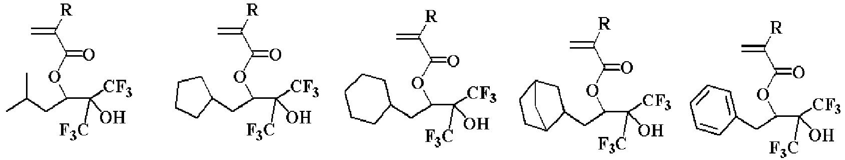

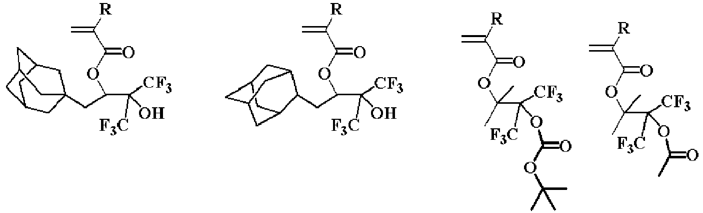

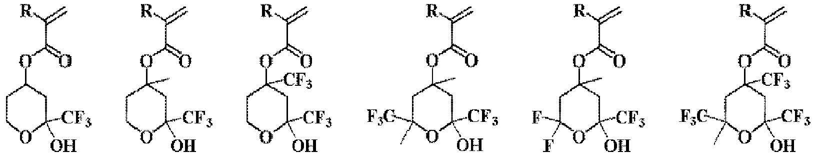

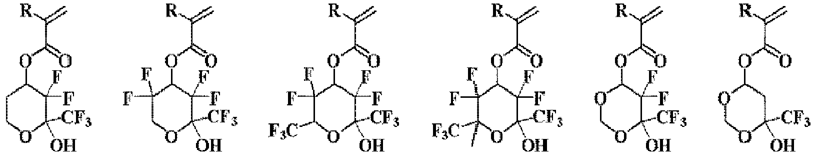

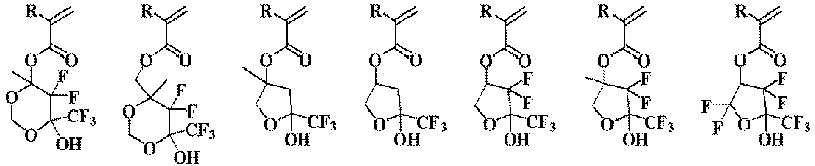

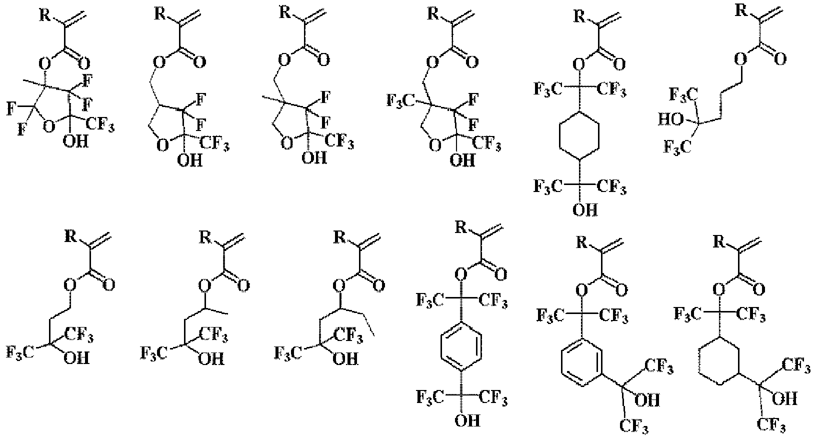

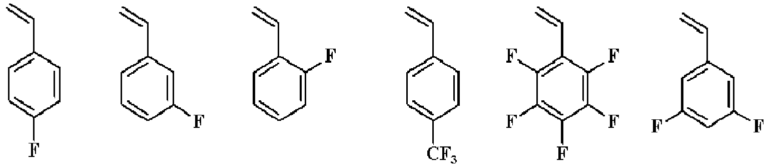

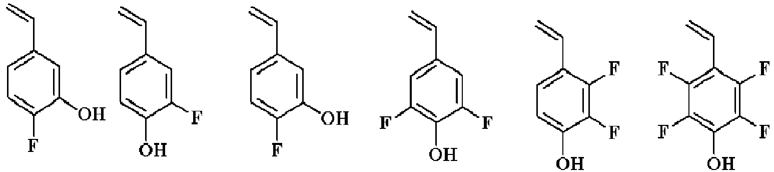

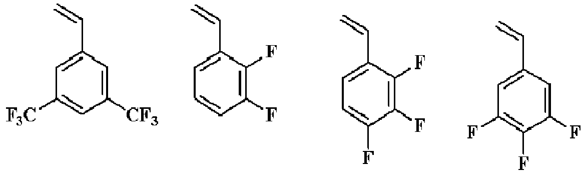

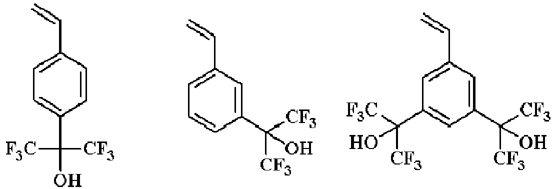

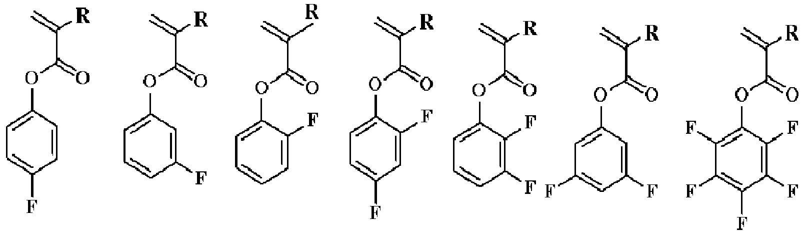

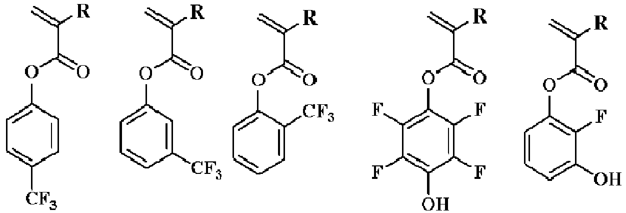

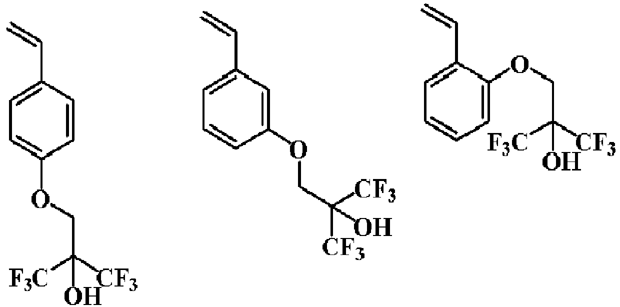

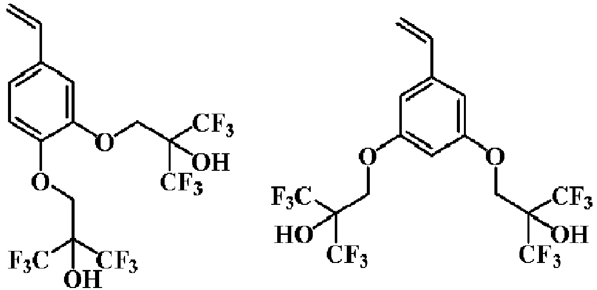

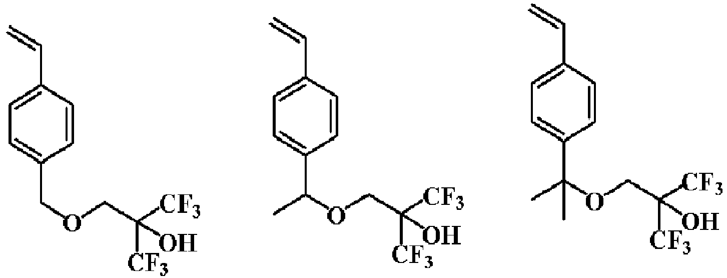

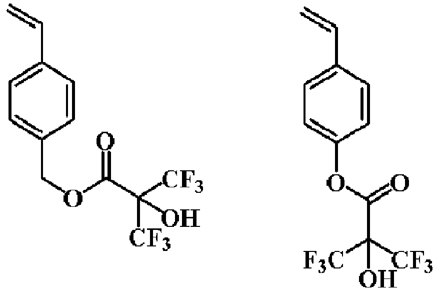

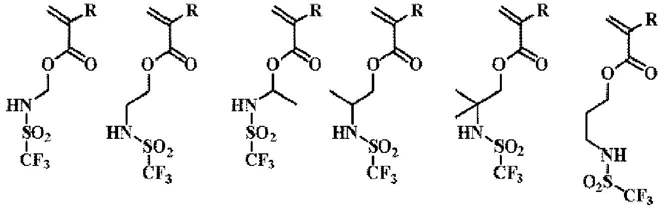

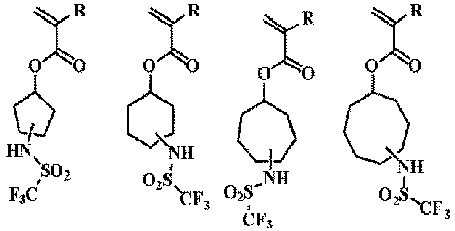

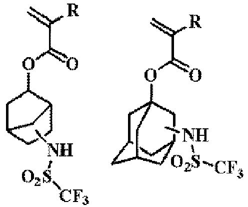

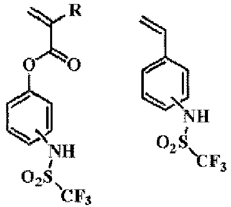

In these formulae, R represents a hydrogen atom or a methyl group.

As one method for synthesizing the polymer compound (A), which is the component (A), a copolymer compound can be obtained, for example, by a method in which desired monomer(s) among the monomers to give the repeating units-al to -a7, -b, -c, -d, -e, -f, and -g undergo heat polymerization in an organic solvent to which a radical polymerization initiator is added.

Examples of the organic solvent used in the polymerization include toluene, benzene, tetrahydrofuran, diethyl ether, dioxane, etc. Examples of the polymerization initiator include 2,2'-azobisisobutyronitrile (AIBN), 2,2'-azobis(2,4-dimethylvaleronitrile), dimethyl 2,2-azobis(2-methylpropionate), benzoyl peroxide, lauroyl peroxide, etc. The heating temperature is preferably 50 to 80°C, and the reaction time is preferably 2 to 100 hours, more preferably 5 to 20 hours.

Here, the ratios of the repeating units-al to -a7, -b, -c, -d, -e, -f, and -g in the polymer compound (A)(polymer (A)) can be 0≤a1≤1.0, 0≤a2≤1.0, 0≤a3≤1.0, 0≤a4≤1.0, 0≤a5≤1.0, 0≤a6≤1.0, 0≤a7≤1.0, 0<a1+a2+a3+a4+a5+a6+a7≤1.0, 0≤b<1.0, 0≤c<1.0, 0≤d<1.0, 0≤e<0.9, 0≤f<0.9, and 0≤g<0.9; preferably 0≤a1≤0.9, 0≤a2≤0.9, 0≤a3≤0.9, 0≤a4≤0.9, 0≤a5≤0.9, 0≤a6≤0.9, 0≤a7≤0.9, 0.01≤a1+a2+a3+a4+a5+a6+a7≤0.9, 0.03≤b≤0.9, 0≤c≤0.8, 0≤d≤0.8, 0≤e<0.8, 0≤f<0.8, and 0≤g<0.8; more preferably 0≤a1≤0.8, 0≤a2≤0.8, 0≤a3≤0.8, 0≤a4≤0.8, 0≤a5≤0.8, 0≤a6≤0.8, 0≤a7≤0.8, 0.02≤a1+a2+a3+a4+a5+a6+a7≤0.8, 0.05≤b≤0.9, 0≤c≤0.7, 0≤d≤0.5, 0≤e<0.3, 0≤f<0.7, and 0≤g<0.7.

Incidentally, for example, a1+a2+a3+a4+a5+a6+a7+b+c+d+e+f+g=1 means that the total amount of the repeating units-al, -a2, -a3, -a4, -a5, -a6, -a7, -b, -c, -d, -e, -f, and -g is 100 mol% on the basis of the total amount of the whole repeating units in the polymer compound (A) containing the repeating units-al, -a2, -a3, -a4, -a5, -a6, -a7, -b, -c, -d, -e, -f, and -g; and a1+a2+a3+a4+a5+a6+a7+b+c+d+e+f+g<1 means that the total amount of the repeating units-al, -a2, -a3, -a4, -a5, -a6, -a7, -b, -c, -d, -e, -f, and -g is less than 100 mol% on the basis of the total amount of the whole repeating units, which indicates that the polymer compound (A) contains another repeating unit(s) besides the repeating units-al, -a2, -a3, -a4, -a5, - a6, -a7, -b, -c, -d, -e, -f, and -g.

Regarding the molecular weight of the polymer compound (A) (component (A)), the weight-average molecular weight is preferably 500 or more, more preferably 1,000 or more and 1,000,000 or less, further preferably 2,000 or more and 500,000 or less. Regarding the ionic monomer (residual monomer) that is not incorporated into the component (A) after the polymerization, if the amount is small, the residual monomer can be prevented from permeating to skin in a biocompatibility test and from causing allergy. Accordingly, it is preferable to decrease the amount of residual monomer(s). The amount of residual monomer(s) is preferably 10 parts by mass or less on the basis of 100 parts by mass of the whole component (A). Additionally, as the component (A), one kind of the polymer compound may be used singly, or there can be used a mixture of two or more kinds of the polymer compound which differ in molecular weight, dispersity, and constitutive polymerizable monomer.

The weight-average molecular weight of the polymer compound (A) can be determined by gel permeation chromatography (GPC) using tetrahydrofuran (THF) as a solvent.

### [Filler Particles (B)]

The inventive bio-electrode composition has a feature of containing filler particles (B), which are one or more selected from the group consisting of silica particles, alumina particles, titania particles, and zirconia particles. The filler particles (B) successfully enhance the ion reception sensitivity. The filler particles (B) is blended in an amount within preferably 1 to 50 parts by mass relative to 100 parts by mass of the resin in the polymer compound (A) and a resin component (D) to be described later. Moreover, one kind of the filler particles (B) may be used singly, or two or more kinds thereof may be used in mixture.

The inventive bio-electrode composition enables quick biological-signal acquisition without electroconductive particles because one or more kinds of the filler particles (B) selected from the group consisting of silica particles, alumina particles, titania particles, and zirconia particles enhance the ion reception sensitivity of the living body contact layer formed from the bio-electrode composition.

Moreover, one or more kinds of the filler particles (B) selected from the group consisting of silica particles, alumina particles, titania particles, and zirconia particles contained in the inventive bio-electrode composition interact with a water-soluble silicone-containing composition incorporated in the bio-electrode composition, making it possible to prevent the elution into water.

In addition, one or more kinds of the filler particles (B) selected from the group consisting of silica particles, alumina particles, titania particles, and zirconia particles contained in the inventive bio-electrode composition enables viscosity adjustment in the event of printing, and makes the appearance after the printing transparent, so that the aesthetic feature can be enhanced, too.

Further, among silica particles, alumina particles, titania particles, and zirconia particles which the inventive bio-electrode composition can incorporate, silica particles are particularly preferably used. Silica particles especially strongly interact with silicone-containing compound incorporated in the inventive bio-electrode composition, making it possible to enhance the effect of preventing components including the polymer compound (A) and a compound (C) having a polyglycerin structure, which is to be described later, from being eluted into water from the bio-electrode composition.

Untreated silica particles, alumina particles, titania particles, and zirconia particles may be used, or may be subjected to surface treatment with an organic compound before use as necessary. For example, the particles to be used may be surface-treated with: a hydrophilic group like alcohol or carboxylic acid; silane having a hydrophilic group such as a hydroxy group, a carboxyl group, an amino group, or a thiol group; polydimethylsiloxane or silane having an alkyl group; or a hydrophobic group like hexamethyldisilazane. Meanwhile, among silica particles, alumina particles, titania particles, and zirconia particles, silica particles are particularly preferably used from the viewpoints of biocompatibility and affinity to the bio-electrode composition.

The silica particles, alumina particles, titania particles, and zirconia particles have an average diameter of preferably 0.001 µm to 20 µm. To make the inventive bio-electrode composition transparent, the particle diameters are preferably 0.001 µm to 2 pm, further preferably 0.001 µm to 0.1 pm, and particularly preferably 0.001 µm to 0.01 µm.

Note that, in the present invention, particle diameters can be determined by a laser diffraction method.

Moreover, the shape of silica particles, alumina particles, titania particles, and zirconia particles is selected from spherical, hollow, porous, rod-like, platy, fibrous, and irregular powders. Preferably, spherical shape is selected.

Incorporating such silica particles, alumina particles, titania particles, and zirconia particles makes it possible to suppress the elution of the water-soluble polymer compound (A) having the repeating unit-a owing to the affinity to silicone, increases the ion reception sensitivity of the living body contact layer formed from the bio-electrode composition, and enables the resulting bio-electrode composition to form a living body contact layer for a bio-electrode which does not leave residue on skin, and which is transparent and excellent in aesthetic feature.

The silica to be used may be wet-type or dry-type fumed silica, fused silica, etc. It is possible to employ, for example, AEROSIL 200 (average particle diameter: 12 nm), AEROSIL 300 (average particle diameter: 7 nm), AEROSIL RX200, AEROSIL RX300, and AEROSIL RY300 manufactured by Nippon Aerosil Co., Ltd.; hydrophobically surface-treated silica, such as REOLOSIL HM-30S manufactured by Tokuyama Corporation; fumed silica manufactured by Sigma-Aldrich Co., LLC.; ADMAFINE manufactured by Admatechs Company Limited; etc.

### [Compound (C) having Polyglycerin Structure]

The inventive bio-electrode composition can further contain a compound (C) having a polyglycerin structure (component (C)), besides the polymer compound (A) and the filler particles (B). The component (C) is blended in an amount of preferably 0.01 to 100 parts by mass, more preferably 0.5 to 60 parts by mass, relative to 100 parts by mass of the component (A). Moreover, one kind of the component (C) may be used singly, or two or more kinds thereof may be used in mixture. The compound (C) having a polyglycerin structure is preferably a silicone compound having a polyglycerin structure.

The silicone compound having a polyglycerin structure is preferably shown by any of the following general formulae (4) and (5).

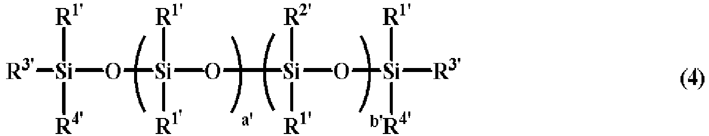

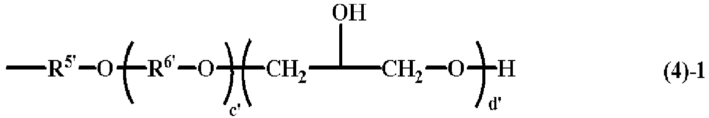

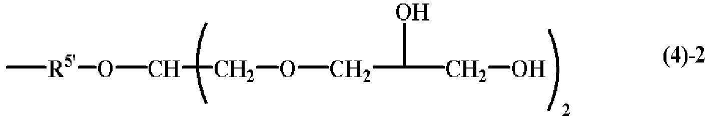

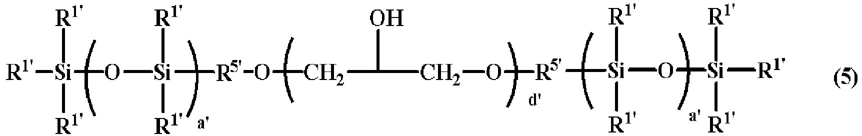

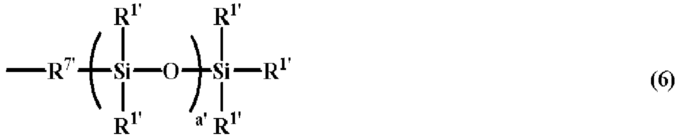

In the formulae, each R¹' is identical to or different from one another, and independently represents a hydrogen atom, a phenyl group, a linear or branched alkyl group having 1 to 50 carbon atoms, or a silicone chain shown by a general formula (6), and optionally contains an ether group. R²' represents a group having a polyglycerin group structure shown by a general formula (4)-1 or (4)-2. Each R³' is identical to or different from the other, and independently represents the R¹' group or the R²' group. Each R⁴' is identical to or different from the other, and independently represents the R¹' group, the R²' group, or an oxygen atom. When R⁴' represents an oxygen atom, the two R⁴' moieties bond to each other and optionally constitute an ether group to form a ring together with silicon atoms. Each a' is identical to or different from one another and represents 0 to 100, b' represents 0 to 100, and a'+b' is 0 to 200. Nevertheless, when b' is 0, at least one R³' is the R²' group. R⁵' represents an alkylene group having 2 to 10 carbon atoms or an aralkylene group having 7 to 10 carbon atoms. R⁶' represents an alkylene group having 2 to 6 carbon atoms. R⁷' represents an alkylene group having 2 to 6 carbon atoms or an ether group. c' represents 0 to 20. d' represents 1 to 20.

Examples of such a silicone compound having a polyglycerin structure can include the following.

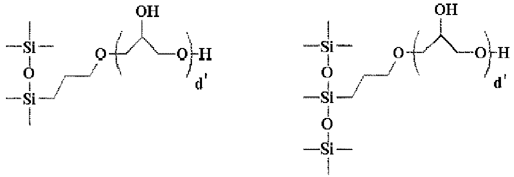

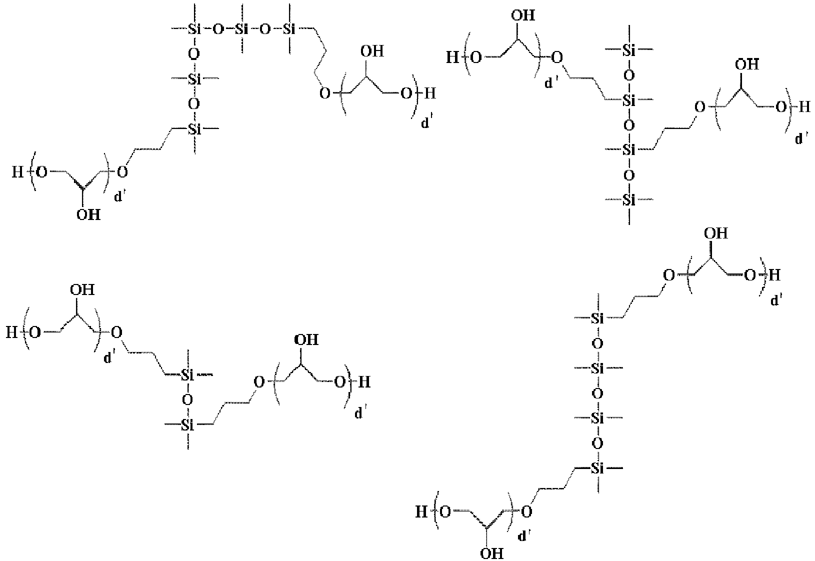

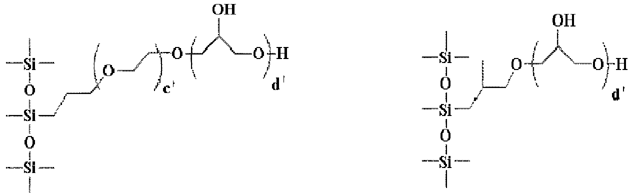

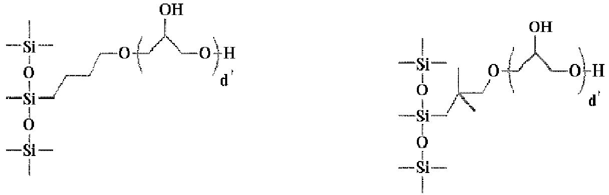

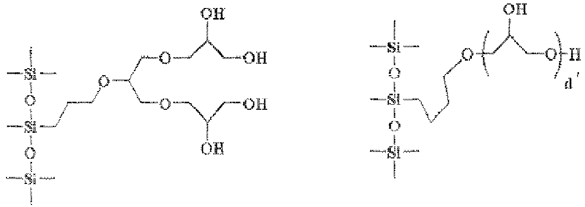

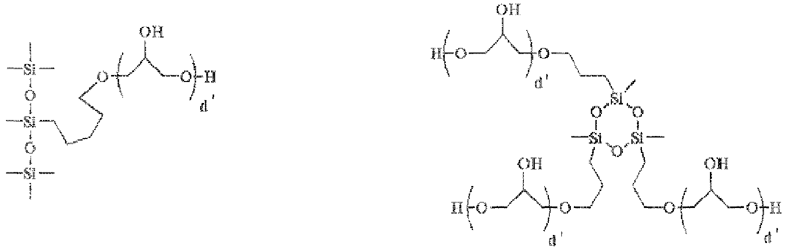

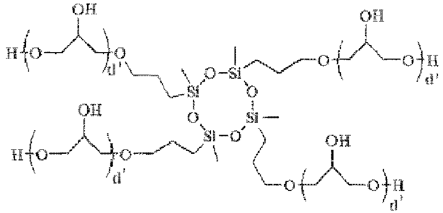

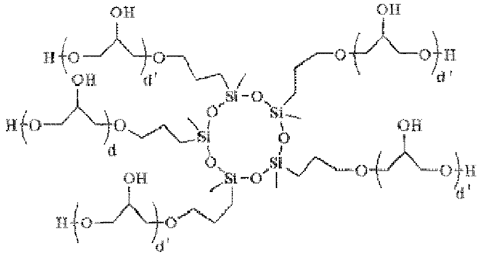

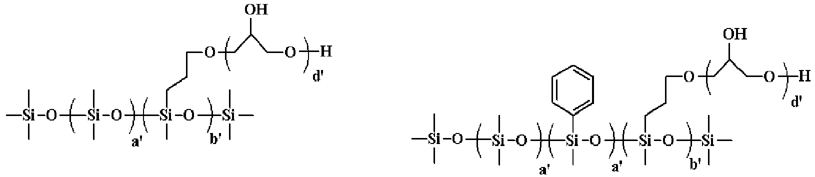

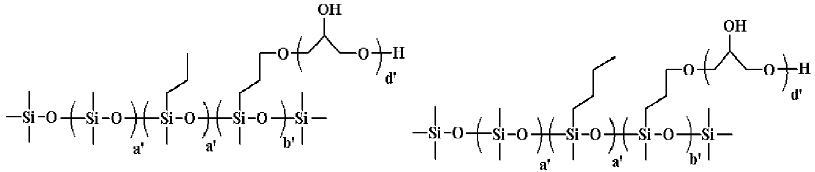

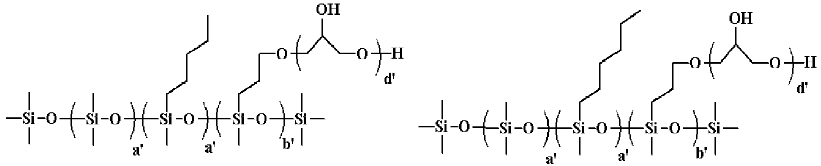

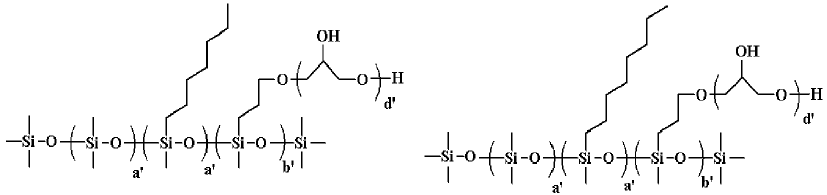

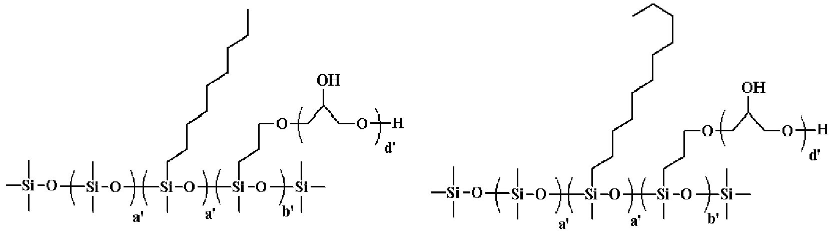

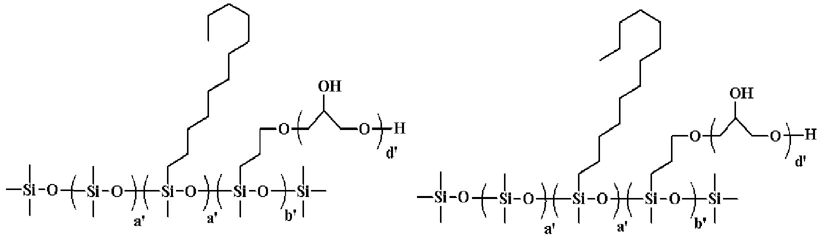

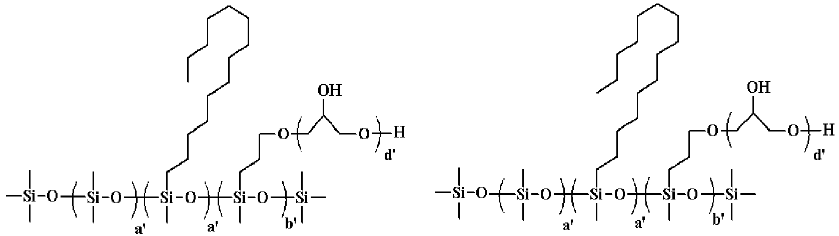

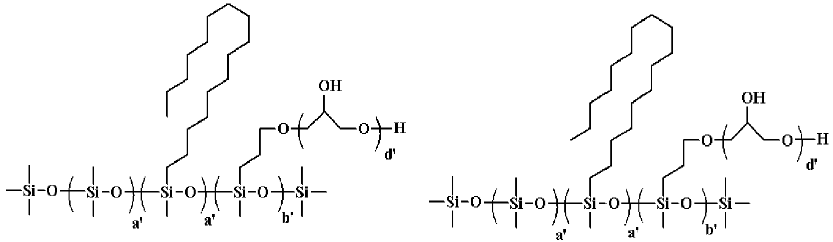

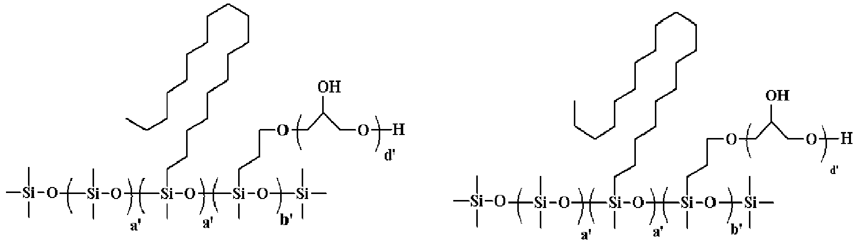

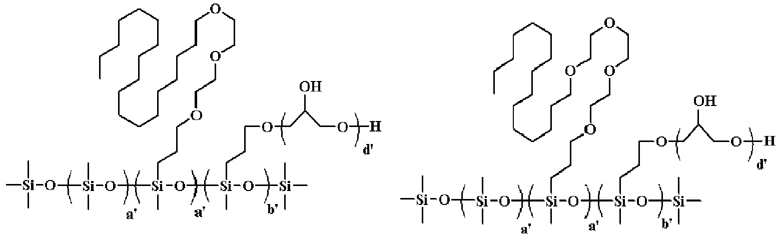

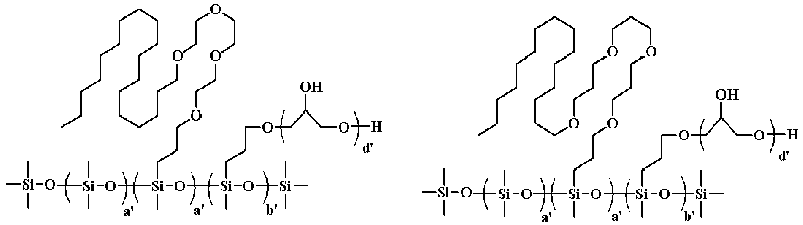

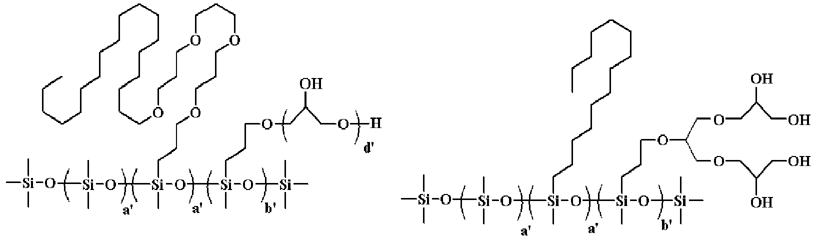

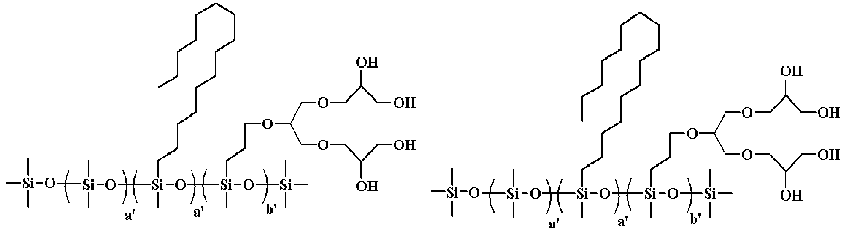

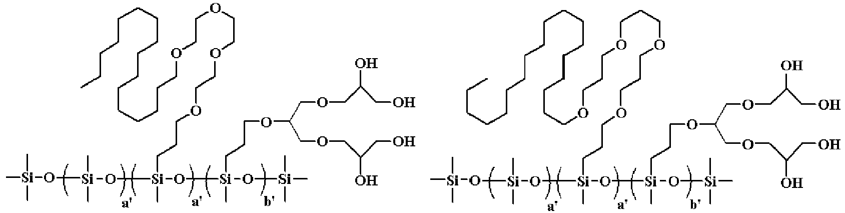

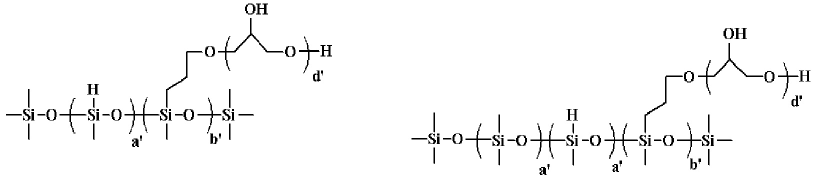

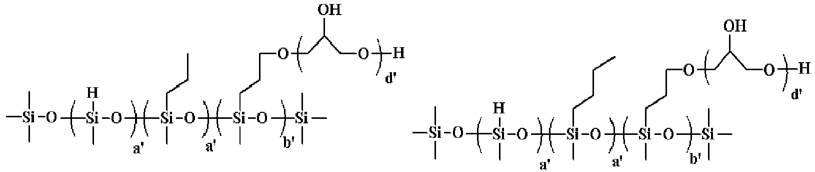

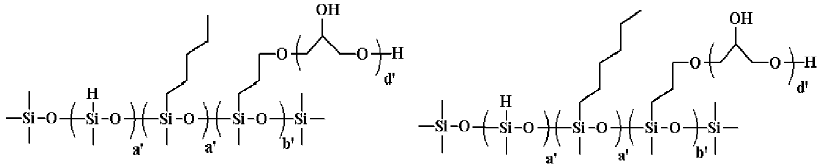

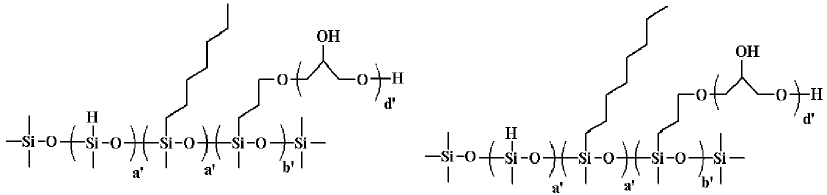

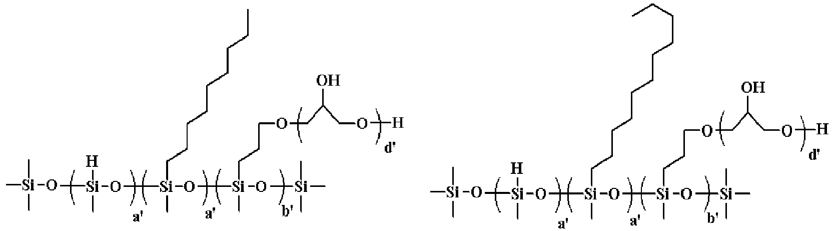

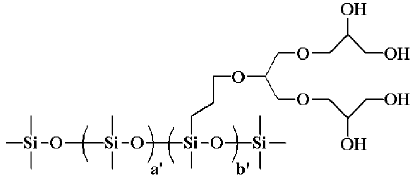

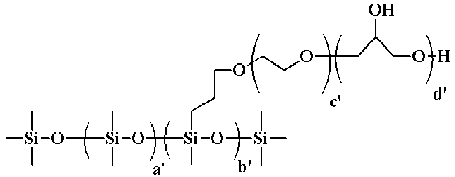

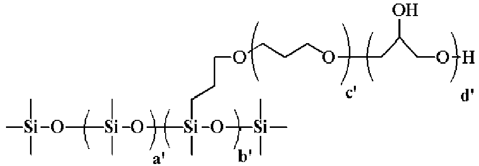

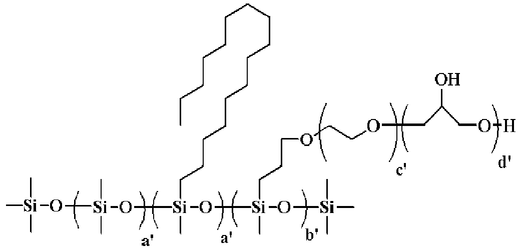

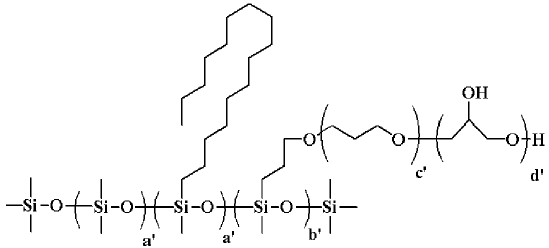

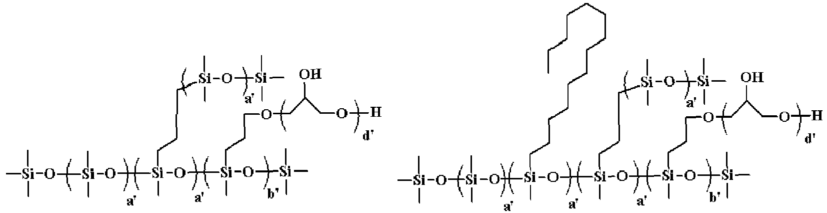

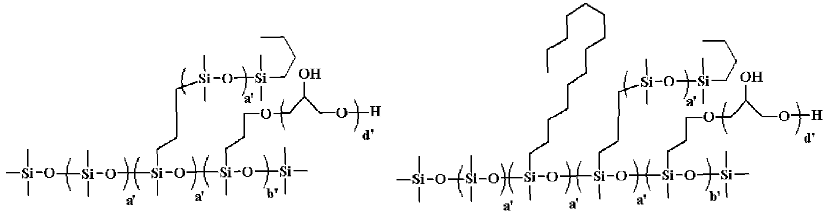

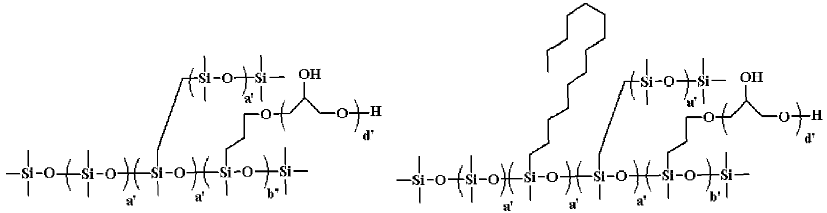

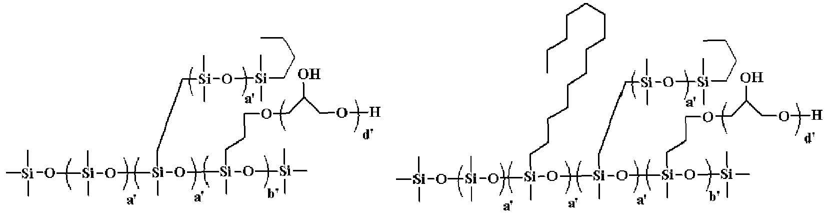

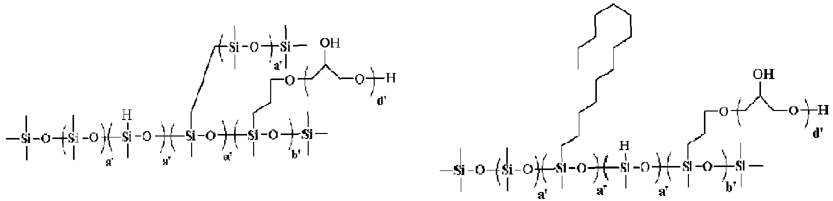

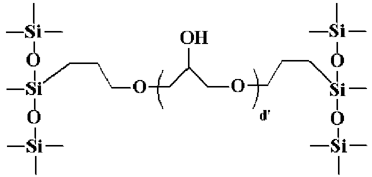

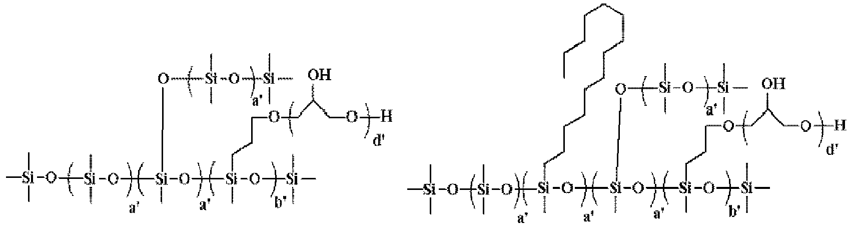

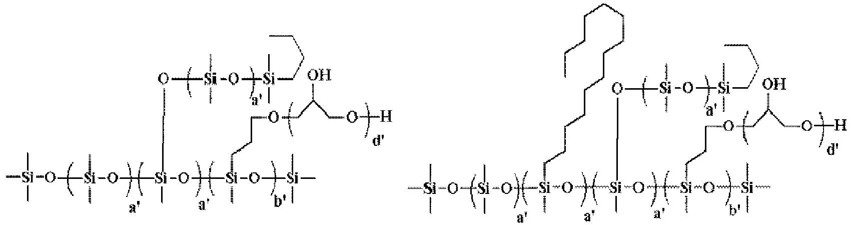

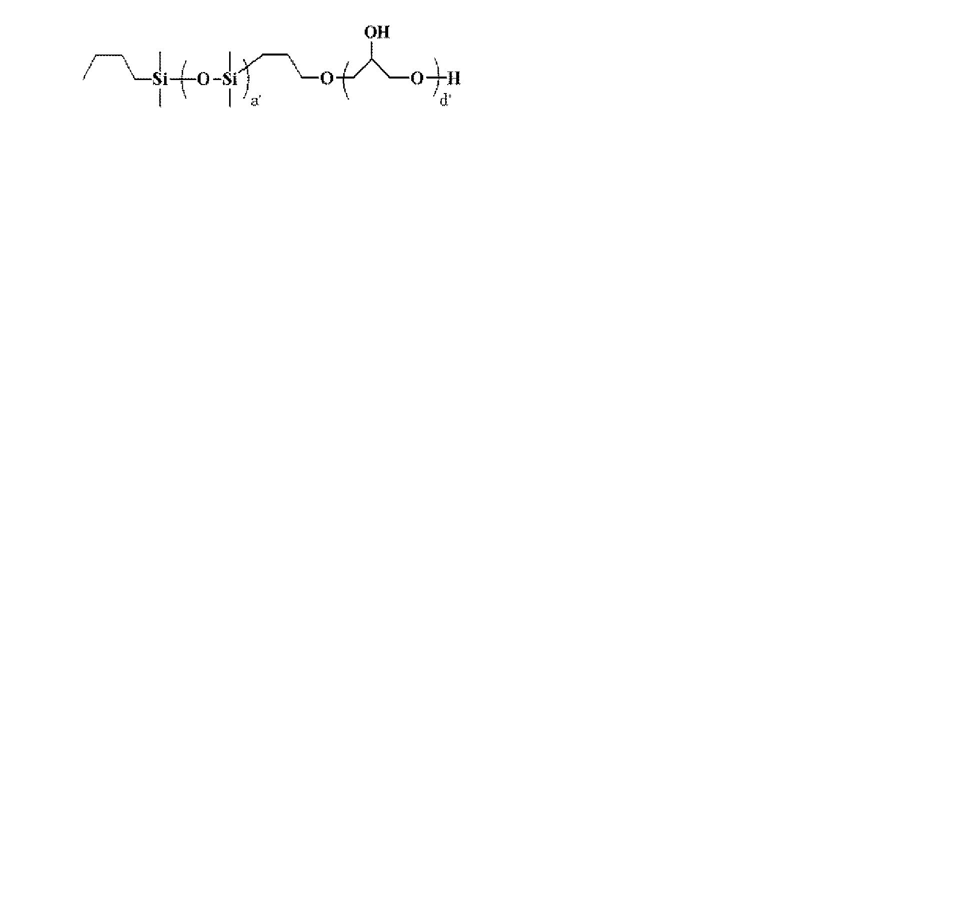

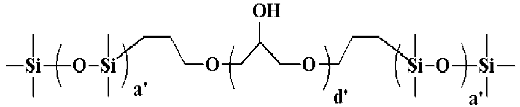

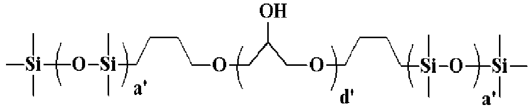

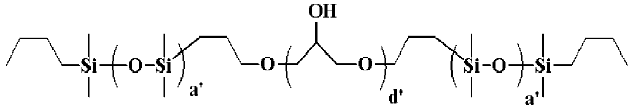

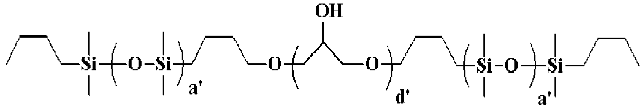

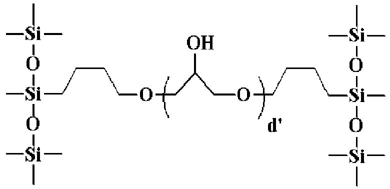

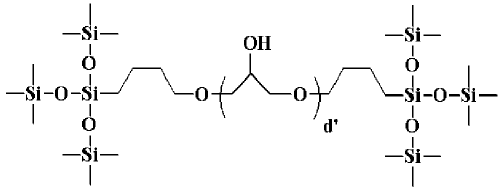

In the formulae, a', b', c' and d' are as defined above.

Incorporating such a silicone compound having a polyglycerin structure enables the bio-electrode composition to form a living body contact layer capable of exhibiting more excellent moisture-holding property and consequently more excellent sensitivity to ions released from skin. When the polymer compound (A) or the filler particles (B) has moisture-holding property, the silicone compound having a polyglycerin structure is not necessarily essential.

### [Resin Component (D)]

The inventive bio-electrode composition can further contain a resin component (D), besides the polymer compound (A) and the filler particles (B). For example, the inventive bio-electrode composition can further contain, as the resin component (D), one or more selected from the group consisting of a silicone-based resin, an acrylic-based resin, and a urethane-based resin. Incorporating one or more kinds of resin selected from the group consisting of silicone-based, acrylic-based, and urethane-based resins can provide a bio-electrode including a living body contact layer excellent in stretchability. Preferably, the silicone-based resin used as the component (D) is different from the silicone compound having a polyglycerin structure.

The resin component (D) blendable in the inventive bio-electrode composition can be, for example, a component that is compatibilized (well mixed) with the polymer compound (A) (ionic material (salt)) to prevent elution of the salt and exhibits adhesion. When the bio-electrode composition contains a metal powder, a carbon powder, a silicon powder, a lithium titanate powder, or the like as described later, the resin component (D) can hold these powders. When the polymer compound (A) has adhesion, the resin (C) is not necessarily essential. Note that the resin component (D) may be any resin other than the component (A) described above, and is preferably either or both of a thermosetting resin and a photocurable resin, particularly preferably one or more selected from the group consisting of silicone-based resin, acrylic-based resin, and urethane-based resin that are different from the silicone compound having a polyglycerin structure.

Examples of the adherent (adhesive) silicone-based resin include an addition reaction-curable (addition-curable) type and a radical crosslinking reaction-curable (radical curable) type. As the addition reaction-curable type, it is possible to use one that contains diorganosiloxane having an alkenyl group (s), an MQ resin having R₃SiO_{0.5} and SiO₂ units, organohydrogenpolysiloxane having multiple SiH groups, a platinum catalyst, an addition-reaction inhibitor, and an organic solvent, for example, described in JP 2015-193803A. As the radical crosslinking reaction-curable type, it is possible to use one that contains diorganopolysiloxane with or without an alkenyl group, an MQ resin having R₃SiO_{0.5} and SiO₂ units, organic peroxide, and an organic solvent, for example, described in JP 2015-193803A. Here, R represents a substituted or unsubstituted monovalent hydrocarbon group having 1 to 10 carbon atoms.

It is also possible to use a polysiloxane-resin integrated compound that is formed by condensation reaction of an MQ resin and polysiloxane having silanol at the terminal or the side chain of the polymer. The MQ resin contains many silanols, and improves adhesive strength by addition of it, but does not bind to the polysiloxane in molecular level because it is not crosslinkable. The adhesive strength can be increased by integrating the polysiloxane and the resin as described above.

The silicone-based resin may contain modified siloxane that has a functional group selected from the group consisting of an amino group, an oxirane group, an oxetane group, a polyether group, a hydroxy group, a carboxyl group, a mercapto group, a methacryl group, an acryl group, a phenol group, a silanol group, a carboxylic anhydride group, an aryl group, an aralkyl group, an amide group, an ester group, and a lactone ring. The addition of the modified siloxane improves dispersibility of the component (A) in the silicone resin. The modified siloxane may be modified at any part such as one terminal, both terminals, or a side chain of the siloxane.

As the adherent acrylic-based resin, it is possible to use one having hydrophilic (meth)acrylic ester and hydrophobic long chain (meth)acrylic ester as the repeating units described in JP 2016-011338A, for example. In some cases, it is also possible to copolymerize (meth)acrylic ester having a functional group or (meth)acrylic ester having a siloxane bond.

As the adherent urethane-based resin, it is possible to use one having a urethane bond with a polyether bond, a polyester bond, a polycarbonate bond, or a siloxane bond described in JP 2016-065238A, for example.

In the inventive bio-electrode composition, the resin component (D) preferably has high compatibility with the component (A) to prevent lowering of the electric conductivity due to elution of the component (A) from the resulting living body contact layer. Additionally, the resin component (D) in the inventive bio-electrode composition preferably has high adhesion to the electro-conductive base material to prevent peeling of the living body contact layer from the electro-conductive base material. In order to increase the adhesion to the electro-conductive base material and the compatibility with the salt, a use of a resin with high polarity as the resin of the component (D) is effective. Examples of such a resin include resin having one or more moieties selected from an ether bond, an ester bond, an amide bond, an imide bond, a urethane bond, a thiourethane bond, and a thiol group; polyacrylic resin, polyamide resin, polyimide resin, polyurethane resin, polythiourethane resin; etc. On the other hand, the living body contact layer is to be contacted with a living body, thereby being susceptible to perspiration. Accordingly, in the inventive bio-electrode composition, the resin component (D) preferably has high repellency and is hardly hydrolyzed. To make the resin of the component (D) be highly repellent and hardly hydrolyzed, the use of a silicon-containing resin is effective.

The silicon atom-containing polyacrylic resin includes a polymer that has a silicone main chain and a polymer that has a silicon atom(s) on the side chain, either of which can be suitably used. As the polymer that has a silicone main chain, siloxane, silsesquioxane, or the like having a (meth)acrylpropyl group can be used. In this case, an addition of a photoradical generator allows the (meth)acryl moiety to polymerize to cure.

As the silicon atom-containing polyamide resin, it is possible to suitably use polyamide silicone resins described in JP 2011-079946A and US 5981680B, for example. Such polyamide silicone resins can be synthesized by combining, for example, a silicone or non-silicone compound having amino groups at both terminals and a non-silicone or silicone compound having carboxyl groups at both terminals.

It is also possible to use polyamic acid before cyclization thereof, which is obtained by reacting carboxylic anhydride and amine. The carboxyl group of the polyamic acid may be crosslinked by using a crosslinking agent such as an epoxy type and an oxetane type. It is also possible to esterify the carboxyl group with hydroxyethyl (meth)acrylate to perform photoradical crosslinking of the (meth)acrylate moiety.

As the silicon atom-containing polyimide resin, it is possible to suitably use polyimide silicone resins described in JP 2002-332305A, for example. Although polyimide resins have very high viscosity, the viscosity can be decreased by blending a (meth)acrylic monomer as a solvent and a crosslinking agent.

Examples of the silicon atom-containing polyurethane resin include polyurethane silicone resins. Such polyurethane silicone resins can be crosslinked through urethane bond by blending a compound having isocyanate groups at both terminals and a compound having a hydroxy group(s) at the terminal(s), followed by heating. In this case, a silicon atom(s) (siloxane bond) have to be contained in either or both of the compound having isocyanate groups at both terminals and the compound having a hydroxy group(s) at the terminal(s). Alternatively, polysiloxane and a urethane (meth)acrylate monomer can be blended and photo-crosslinked as described in JP 2005-320418A. It is also possible to photo-crosslink a polymer having both of a siloxane bond(s) and a urethane bond(s), with the terminal having a (meth)acrylate group(s). Particularly, a material having a polyurethane main chain with a silicone chain on a side chain as described in JP 2018-123304A and JP 2019-70109A is preferable because of the properties of high strength and high stretchability.

The silicon atom-containing polythiourethane resin can be obtained by reaction of a compound having a thiol group(s) and a compound having an isocyanate group(s), provided that at least one of them contains a silicon atom(s). It can also be photo-cured if (meth)acrylate groups are contained at the terminals.

The silicone-based resin can be improved in compatibility with the foregoing salt by adding modified siloxane that has a functional group selected from the group consisting of an amino group, an oxirane group, an oxetane group, a polyether group, a hydroxy group, a carboxyl group, a mercapto group, a methacryl group, an acryl group, a phenol group, a silanol group, a carboxylic anhydride group, an aryl group, an aralkyl group, an amide group, an ester group, and a lactone ring, in addition to the diorganosiloxane having an alkenyl group(s), the MQ resin having R₃SiO_{0.5} and SiO₂ units, and the organohydrogenpolysiloxane having multiple SiH groups.

In the inventive bio-electrode composition, the resin component (D) is blended in an amount of preferably 0 to 2,000 parts by mass, more preferably 10 to 1,000 parts by mass, relative to 100 parts by mass of the polymer compound (A), which is an ion polymer. Moreover, one kind of the resin component (D) may be used singly, or two or more kinds thereof may be used in mixture.

As will be described later, the living body contact layer of a bio-electrode according to the present invention is a cured product of the inventive bio-electrode composition. By curing the bio-electrode composition, the resulting living body contact layer has favorable adhesion to both of skin and the electro-conductive base material. The curing means is not particularly limited, and common means can be used, including crosslinking reaction by either or both of heat and light, or with an acid catalyst or a base catalyst, for example. The crosslinking reaction can be performed, for example, by appropriately selecting methods described in "Kakyou han-nou handbook (handbook of crosslinking reaction)", Chapter 2, pages 51-371, Yasuharu Nakayama, Maruzen Publishing Co., Ltd. (2013).

The diorganosiloxane having an alkenyl group(s) and the organohydrogenpolysiloxane having multiple SiH groups can be crosslinked through an addition reaction with a platinum catalyst.

Examples of the platinum catalyst include platinum-based catalysts such as chloroplatinic acid, alcohol solution of chloroplatinic acid, reaction product of chloroplatinic acid and alcohol, reaction product of chloroplatinic acid and an olefin compound, reaction product of chloroplatinic acid and vinyl group-containing siloxane, a platinum-olefin complex, and a complex of platinum and vinyl group-containing siloxane; platinum group metal-based catalysts such as a rhodium complex and a ruthenium complex; etc. These catalysts may be used after dissolved or dispersed in alcohol solvent, hydrocarbon solvent, or siloxane solvent.

Note that the platinum catalyst is added in an amount within preferably 5 to 2,000 ppm, particularly preferably 10 to 500 ppm, relative to 100 parts by mass of the resin in the polymer compound (A) and the resin component (D).

When the addition-curable silicone resin is used, an addition-reaction inhibitor may be added. This addition-reaction inhibitor is added as a quencher to prevent the action of the platinum catalyst in the solution and under a low temperature circumstance after forming the coating film and before heat curing. Specific examples of the addition-reaction inhibitor include 3-methyl-1-butyn-3-ol, 3-methyl-1-pentyn-3-ol, 3,5-dimethyl-1-hexyn-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxycyclohexane, bis(2,2-dimethyl-3-butynoxy)dimethylsilane, 1,3,5,7-tetramethyl-1,3,5,7-tetravinylcyclotetrasiloxane, 1,1,3,3-tetramethyl-1,3-divinyldisiloxane, etc.

The addition-reaction inhibitor is added in an amount within preferably 0 to 10 parts by mass, particularly preferably 0.05 to 3 parts by mass, relative to 100 parts by mass of the resin in the polymer compound (A) and the resin component (D).

Examples of the photo-curing method include a method of adding a photoradical generator to generate radical by light, together with a resin having a (meth)acrylate terminal(s) or an olefin terminal(s), or a crosslinking agent with the terminal(s) being (meth)acrylate, olefin, or a thiol group(s); and a method of adding a photo-acid generator to generate acid by light, together with a resin or a crosslinking agent having an oxirane group(s), an oxetane group(s), or a vinyl ether group(s).

Examples of the photoradical generator include acetophenone, 4,4'-dimethoxybenzyl, benzyl, benzoin, benzophenone, 2-benzoylbenzoic acid, 4,4'-bis(dimethylamino)benzophenone, 4,4'-bis(diethylamino)benzophenone, benzoin methyl ether, benzoin ethyl ether, benzoin isopropyl ether, benzoin butyl ether, benzoin isobutyl ether, 4-benzoylbenzoic acid, 2,2'-bis(2-chlorophenyl)-4,4',5,5'-tetraphenyl-1,2'-biimidazole, methyl 2-benzoylbenzoate, 2-(1,3-benzodioxole-5-yl)-4,6-bis(trichloromethyl)-1,3,5-triazine, 2-benzyl-2-(dimethylamino)-4'-morpholinobutyrophenone, 4,4'-dichlorobenzophenone, 2,2-diethoxyacetophenone, 2,2-dimethoxy-2-phenylacetophenone, 2,4-diethylthioxanthene-9-one, diphenyl(2,4,6-trimethylbenzoyl)phosphine oxide (BAPO), 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isonitrosopropiophenone, and 2-phenyl-2-(p-toluenesulfonyloxy)acetophenone.

The curing can also be performed by adding a radical generator of a heat decomposition type. Examples of the thermal-decomposition type radical generator include 2,2'-azobis(isobutyronitrile), 2,2'-azobis(2,4-dimethylvaleronitrile), 2,2'-azobis(2-methylbutyronitrile), 2,2'-azobis(methylpropionamidine) hydrochloride, 2,2'-azobis[2-(2-imidazolin-2-yl)propane] hydrochloride, 2,2'-azobis(4-methoxy-2,4-dimethylvaleronitrile), 2,2'-azobis(cyclohexane-1-carbonitrile), 1[(1-cyano-1-methylethyl)azo]formamide, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl-2,2'-azobis(isobutylate), 4,4'-azobis(4-cyanopentanoic acid), dimethyl-2,2'-azobis(2-methylpropionate), benzoyl peroxide, tert-butyl hydroperoxide, cumene hydroperoxide, di-tert-butyl peroxide, di-tert-amyl peroxide, di-n-butyl peroxide, dicumyl peroxide, etc.

Examples of the photo-acid generator include sulfonium salt, iodonium salt, sulfonyldiazomethane, N-sulfonyloxyimide, oxime-O-sulfonate type acid generators, etc. Specific examples of the photo-acid generator include ones described in paragraphs [0122] to [0142] of JP 2008-111103A, and in JP 2009-080474A.

Note that the amount of the radical generator or photo-acid generator added is preferably in a range of 0.1 to 50 parts by mass relative to 100 parts by mass of the resin in the polymer compound (A) and the resin component (D).

Above all, the resin of the component (D) particularly preferably contains any of: a silicone resin having an SiO₂ unit and an RₓSiO_{(4-x)/2} unit, where R is a substituted or unsubstituted monovalent hydrocarbon group having 1 to 10 carbon atoms, and "x" is the number in a range of 2.5 to 3.5; diorganosiloxane having an alkenyl group; and organohydrogenpolysiloxane having an SiH group. Such resin components (D) are compatibilized with the polymer compound (A), and can prevent elution of the salt, and can impart higher adhesion to the bio-electrode composition.

### [Organic Solvent (E)]

Further, the inventive bio-electrode composition may contain an organic solvent (E). Specific examples of the organic solvent (E) include: aromatic hydrocarbon solvents, such as toluene, xylene, cumene, 1,2,3-trimethylbenzene, 1,2,4-trimethylbenzene, 1,3,5-trimethylbenzene, styrene, α-methylstyrene, butylbenzene, sec-butylbenzene, isobutylbenzene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tertamylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; aliphatic hydrocarbon solvents, such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonyne, 2-nonyne, 3-nonyne, 4-nonyne, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, α-phellandrene, pinene, terpinene, tetrahydrodicyclopentadiene, 5,6-dihydrodicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0^{2,7}]undeca-4-ene, n-dodecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffins; ketone solvents, such as cyclohexanone, cyclopentanone, 2-octanone, 2-nonanone, 2-heptanone, 3-heptanone, 4-heptanone, 2-hexanone, 3-hexanone, diisobutyl ketone, methylcyclohexanone, and methyl n-pentyl ketone; alcohol solvents, such as 3-methoxybutanol, 3-methyl-3-methoxybutanol, 1-methoxy-2-propanol, and 1-ethoxy-2-propanol; ether solvents, such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropyl ether, diisobutyl ether, diisopentyl ether, di-n-pentyl ether, methyl cyclopentyl ether, methyl cyclohexyl ether, di-n-butyl ether, di-sec-butyl ether, di-sec-pentyl ether, di-tert-amyl ether, di-n-hexyl ether, and anisole; ester solvents, such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactone solvents, such as γ-butyrolactone; water; etc.

Note that the organic solvent is added in an amount within preferably 10 to 50,000 parts by mass relative to 100 parts by mass of the resin in the polymer compound (A) and the resin component (D).

### [Electro-Conductive Powder (F)]

The inventive bio-electrode composition may further contain an electro-conductive powder (F). Specific examples of the electro-conductive powder (F) include a carbon powder, a metal powder, a silicon powder, a lithium titanate powder, etc.

### [Metal Powder]

The inventive bio-electrode composition may contain a metal powder in order to improve electron conductivity. The metal powder is a powder of a metal selected from the group consisting of gold, silver, platinum, copper, tin, titanium, nickel, aluminum, tungsten, molybdenum, ruthenium, chromium, and indium. The metal powder is added in an amount within preferably 1 to 50 parts by mass relative to 100 parts by mass of the resin in the polymer compound (A) and the resin component (D).

As the kind of the metal powder, gold, silver, and platinum are preferable from the viewpoint of electric conductivity; and silver, copper, tin, titanium, nickel, aluminum, tungsten, molybdenum, ruthenium, and chromium are preferable from the viewpoint of cost. From the viewpoint of biocompatibility, noble metals are preferable. From comprehensive viewpoint including the above, silver is most preferable.

The metal powder may have any shape, such as a spherical shape, a disk shape, a flaky shape, and a needle shape. The addition of flaky powder brings highest electric conductivity and is preferable. The metal powder is preferably a flake having relatively lower density and large specific surface area with a size of 100 µm or less, a tapped density of not more than 5 g/cm³, and a specific surface area of not less than 0.5 m²/g.

### [Carbon Powder]

A carbon powder can be added as an electric conductivity improver. Examples of the carbon powder (carbon material) include carbon black, graphite, carbon nanotube, carbon fiber, etc. The carbon nanotube may be either single layer or multilayer, and the surface may be modified with an organic group(s). The carbon material is added in an amount within preferably 1 to 50 parts by mass relative to 100 parts by mass of the resin in the polymer compound (A) and the resin component (D). The carbon powder is preferably one or both of carbon black and carbon nanotube.

### [Silicon Powder]

A silicon powder can be added as an electric conductivity improver. Examples of the silicon powder include powders of elemental silicon (Si), silicon monoxide (SiO), silicon carbide (SiC), silicon oxycarbide, silicate, or the like. Particularly, a powder of silicon selected from Si, SiO, and SiC is preferable. The particle diameter of the powder is preferably smaller than 100 pm, more preferably 1 µm or less. The silicon powder is added in an amount within preferably 1 to 50 parts by mass relative to 100 parts by mass of the resin in the polymer compound (A) and the resin component (D).

### [Lithium Titanate Powder]

The inventive bio-electrode composition may contain a lithium titanate powder to enhance ion reception sensitivity. Examples of the lithium titanate powder include powders containing a compound shown by molecular formulae Li₂TiO₃, LiTiO₂, or Li₄Ti₅O₁₂ with a spinel structure. The lithium titanate powder preferably has a spinel structure. It is also possible to use carbon-incorporated lithium titanate particles. The particle diameter of the powder is preferably smaller than 100 pm, more preferably 1 µm or less. Since finer particles have a larger surface area, the bio-electrode can receive a larger amount of ions, and has higher sensitivity. The aforementioned powders may be composite powders with carbon. The lithium titanate powder is added in an amount within preferably 1 to 50 parts by mass relative to 100 parts by mass of the resin in the polymer compound (A) and the resin component (D).

Above all, it is preferable to further incorporate as the component (F) a carbon powder, a silver powder, and/or a lithium titanate powder.

### [Tackifier]

The inventive bio-electrode composition may also contain a tackifier in order to have adhesion to a living body. Examples of such a tackifier include silicone resin, non-crosslinkable siloxane, non-crosslinkable poly(meth)acrylate, non-crosslinkable polyether, etc.

### [Crosslinking Agent]

The inventive bio-electrode composition may contain an epoxy-type crosslinking agent. This crosslinking agent is a compound having multiple epoxy groups or oxetane groups in one molecule. The amount of the crosslinking agent added can be 1 to 30 parts by mass relative to 100 parts by mass of the resin in the polymer compound (A) and the resin component (D).

### [Crosslinking Catalyst]

The inventive bio-electrode composition may also contain a catalyst for crosslinking the epoxy groups or the oxetane groups. As this catalyst, for example, ones described in paragraphs 0027 to 0029 of JP 2019-503406A can be used. The amount of the catalyst added can be 0.01 to 10 parts by mass relative to 100 parts by mass of the resin in the polymer compound (A) and the resin component (D).

### [Ionic Additive]

The inventive bio-electrode composition may contain an ionic additive to enhance the ionic conductivity. In consideration of biocompatibility, examples of the ionic additive include sodium chloride, potassium chloride, calcium chloride, saccharin, acesulfame K, and salts disclosed in JP 2018-44147A, JP 2018-59050A, JP 2018-59052A, and JP 2018-130534A.

As has been described above, the inventive bio-electrode composition makes it possible to form a living body contact layer for a bio-electrode which is excellent in electric conductivity and biocompatibility, light-weight, manufacturable at low cost, and free from significant reduction in the electric conductivity even when the bio-electrode is wetted with water or dried, and which enables quick signal collection after attachment to skin, does not leave residue on the skin, and is transparent and high in aesthetic feature. The living body contact layer for a bio-electrode formed using the inventive bio-electrode composition is capable of exhibiting excellent electric conductivity, and thus capable of transmitting electric signals from a living body, for example, skin, to a device efficiently. Moreover, this living body contact layer satisfactorily exhibits excellent biocompatibility, thereby preventing allergy even when the bio-electrode is worn on skin for a long time. It is also possible to further improve the electric conductivity by adding an electric conductivity improver such as a carbon material. In addition, it is possible to manufacture a bio-electrode with particularly high adhesive strength and stretchability by combining the inventive bio-electrode composition with a resin having adhesion and stretchability. Further, the stretchability and adhesion to skin can be enhanced by adding an additive and so forth to the inventive bio-electrode composition. Furthermore, the stretchability and adhesion can also be controlled by appropriately adjusting the composition of the polymer compound (A) in the inventive bio-electrode composition, the amount of the filler particles (B), or the thickness of the living body contact layer.

### <Bio-Electrode>

The present invention also provides a bio-electrode including an electro-conductive base material and a living body contact layer formed on the electro-conductive base material, the living body contact layer containing a cured product of the inventive bio-electrode composition described above.

Hereinafter, the inventive bio-electrode will be described in detail with reference to the drawings, but the present invention is not limited thereto.

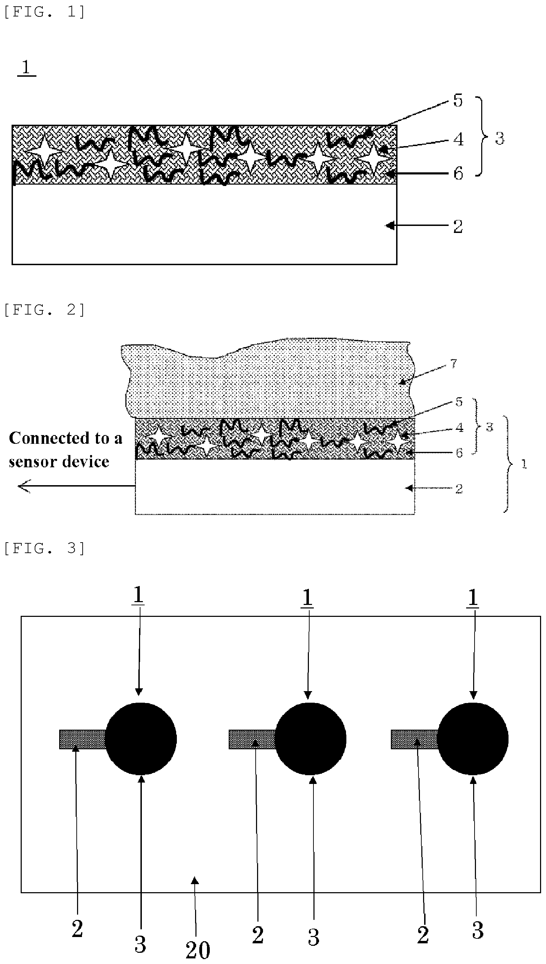

FIG. 1 is a schematic sectional view showing an example of the inventive bio-electrode. In FIG. 1, a bio-electrode 1 has an electro-conductive base material 2 and a living body contact layer 3 formed on the electro-conductive base material 2. The living body contact layer 3 is a layer in which an ionic polymer (the polymer compound (A)) 5 and filler particles 4 are dispersed in a resin 6. The ionic polymer 5 is an example of the polymer compound (A) described above. The resin 6 is the above-described compound (C) having a polyglycerin structure and resin component (D). This living body contact layer 3 is a cured product of the bio-electrode composition, which is an example of the present invention.

When the bio-electrode 1 as shown in FIG. 1 is used, the living body contact layer 3 (i.e., the layer in which the ionic polymer 5 and the filler particles 4 are dispersed in the resin 6) is brought into contact with a living body 7 as shown in FIG. 2. Electric signals are picked from the living body 7 through the ionic polymer 5 and the filler particles 4, and then conducted to a sensor device or the like (not shown) via the electro-conductive base material 2. As described above, the inventive bio-electrode is capable of coping with both electric conductivity and biocompatibility by using the ionic polymer (ionic material) described above, and obtaining electric signals from skin stably in high sensitivity because the contact area with the skin is kept constant due to the adhesion thereof. Particularly, as has been described above, the inventive bio-electrode composition makes it possible to form a living body contact layer capable of quickly collecting signals after attachment to skin. Accordingly, the bio-electrode 1 in FIG. 1 can quickly collect signals after attached to the living body 7.

Hereinafter, each component of the inventive bio-electrode will be described more specifically.

### [Electro-Conductive Base Material]

The inventive bio-electrode has an electro-conductive base material. This electro-conductive base material is usually connected electrically with a sensor device etc., and conducts electrical signals picked from a living body through the living body contact layer to the sensor device etc.

The electro-conductive base material is not particularly limited, as long as it has electric conductivity. The electro-conductive base material preferably contains one or more selected from the group consisting of gold, silver, silver chloride, platinum, aluminum, magnesium, tin, tungsten, iron, copper, nickel, stainless steel, chromium, titanium, and carbon, for example.

The electro-conductive base material is not particularly limited, and may be a hard electro-conductive substrate, an electro-conductive film having flexibility, a substrate having a stretchable film coated with electro-conductive paste, a cloth with the surface being coated with electro-conductive paste, a cloth into which electro-conductive polymer is kneaded, etc. The electro-conductive base material may be flat, uneven, or mesh-form of woven metal wires, which can be appropriately selected in accordance with the use of the bio-electrode, and so forth. Among these, in consideration of the usage after attachment on skin, preferable are substrates including stretchable film or cloth coated with electro-conductive paste. Examples of the stretchable film include polyurethane and polyester. The electro-conductive paste to be used can be obtained by mixing a solvent and an electro-conductive powder of carbon, silver, gold, copper, or the like in a stretchable resin such as polyurethane, polyester, silicone, or nitrile resin.

### [Living Body Contact Layer]

The inventive bio-electrode has a living body contact layer formed on the electro-conductive base material. This living body contact layer is a part to be actually in contact with a living body when the bio-electrode is used. The living body contact layer has electric conductivity and adhesion. The living body contact layer is a cured product of the inventive bio-electrode composition described above; that is, an adherent resin layer containing: the polymer compound (A) (ionic material (salt)); one or more kinds of the filler particles (B) selected from the group consisting of silica particles, alumina particles, titania particles, and zirconia particles; and optionally the compound (C) having a polyglycerin structure, the resin component (D), and other additive(s).

The living body contact layer preferably has an adhesive strength in a range of 0.5 N/25mm or more and 20 N/25mm or less. The adhesive strength is commonly measured by the method described in JIS Z 0237, in which a metal substrate such as a stainless steel (SUS) substrate or a polyethylene terephthalate (PET) substrate can be used as a base material. Alternatively, human skin can be used for measuring. Human skin has lower surface energy than metals and various plastics, and the energy is as low as that of Teflon (registered trademark). Thus, human skin is hard to adhere.

The living body contact layer of the bio-electrode has a thickness of preferably 1 µm or more and 5 mm or less, more preferably 2 µm or more and 3 mm or less. As the living body contact layer is thinner, the adhesive strength lowers, but the flexibility is improved, the weight decreases and the compatibility with skin is improved. The thickness of the living body contact layer can be selected based on the balance of adhesion and texture to the skin.

The living body contact layer of the inventive bio-electrode is preferably humidified. The bio-electrode including such a living body contact layer can more quickly collect signals when attached to skin.

The inventive bio-electrode may be additionally provided with an adherent film on the living body contact layer as in conventional bio-electrodes (e.g., the bio-electrode described in JP 2004-033468A) in order to prevent peeling off of the bio-electrode from a living body during the use. When the adherent film is provided separately, the adherent film may be formed by using a raw material for the adherent film such as an acrylic type, a urethane type, and a silicone type. Particularly, the silicone type is suitable because of: the high oxygen permeability, which enables dermal respiration while the electrode is attached to the skin; the high water repellency, which suppresses lowering of adhesion due to perspiration; and the low irritation to skin. It is to be noted that the inventive bio-electrode does not necessarily require this adherent film that is provided separately, because peeling off from a living body can be prevented by adding a tackifier to the bio-electrode composition or using a resin having good adhesion to a living body as described above.

When the inventive bio-electrode is used as a wearable device, wiring between the bio-electrode and a sensor device, and other components are not limited to particular ones. For example, it is possible to employ ones described in JP 2004-033468A.

As described above, since the inventive bio-electrode include the living body contact layer formed from the cured product of the inventive bio-electrode composition, the bio-electrode is excellent in electric conductivity and biocompatibility, light-weight, manufacturable at low cost, free from significant reduction in the electric conductivity even when wetted with water or dried, enables quick signal collection after attached to skin, does not leave residue on the skin, and is transparent and high in aesthetic feature. The living body contact layer of the inventive bio-electrode is capable of exhibiting excellent electric conductivity and thus transmitting electric signals from a living body, for example, skin, to a device efficiently. Moreover, this living body contact layer satisfactorily exhibits excellent biocompatibility, thereby preventing allergy even when the bio-electrode is worn on skin for a long time. It is also possible to further improve the electric conductivity of the living body contact layer in the inventive bio-electrode by adding a metal powder. In addition, a bio-electrode with particularly high adhesive strength and stretchability can be manufactured by combining the inventive bio-electrode composition with a resin having adhesion and stretchability. Further, the stretchability and adhesion of the living body contact layer to skin can be enhanced by adding an additive and so forth to the living body contact layer. Furthermore, the stretchability and adhesion can also be controlled by appropriately adjusting the composition of the polymer compound (A) and/or the compound (C) having a polyglycerin structure in the inventive bio-electrode composition, the amount of one or more kinds of the filler particles (B) selected from the group consisting of silica particles, alumina particles, titania particles, and zirconia particles, or the thickness of the living body contact layer. Accordingly, the inventive bio-electrode as described above is particularly suitable as a bio-electrode used for a medical wearable device.

### <Method for Manufacturing Bio-Electrode>

The present invention also provides a method for manufacturing a bio-electrode having an electro-conductive base material and a living body contact layer formed on the electro-conductive base material, the method including:
applying the above-described bio-electrode composition onto the electro-conductive base material; and
curing the bio-electrode composition to form the living body contact layer.

Note that the electro-conductive base material, bio-electrode composition, etc. used in the inventive method for manufacturing a bio-electrode may be the same as those described above.

The method for applying the bio-electrode composition onto the electro-conductive base material is not particularly limited. Examples of the suitable method include dip coating, spray coating, spin coating, roll coating, flow coating, doctor coating, screen printing, flexographic printing, gravure printing, stencil printing, inkjet printing, etc.

The method for curing the resin is not particularly limited and can be appropriately selected based on the kinds of the compound (C) having a polyglycerin structure and the resin (D) used for the bio-electrode composition. For example, the bio-electrode composition is preferably cured by either or both of heat and light. The bio-electrode composition can also be cured by adding in advance a catalyst for generating acid or base to the bio-electrode composition, which causes a crosslinking reaction.

The heating temperature is not particularly limited and may be appropriately selected based on the kinds of the compound (C) having a polyglycerin structure and the resin (D) used for the bio-electrode composition, but is preferably about 50 to 250°C, for example.

When the heating and light irradiation are combined, it is possible to perform the heating and the light irradiation simultaneously, to perform the light irradiation and then the heating, or to perform the heating and then the light irradiation. It is also possible to perform air-drying to evaporate the solvent before heating the coating film.

The cured film, that is, the resulting living body contact layer, may be immersed in water; alternatively, the film surface may be wiped with a water-containing absorbent cotton, gauze, or nonwoven fabric, or sprayed with water droplets, water vapor, or mist. In other words, performing humidification treatment improves the compatibility with skin, and can obtain biological signals quickly. Water mixed with alcohol can be used to reduce size of water droplets in water vapor or mist.

Before the bio-electrode is attached to skin, the sensitivity to biological signals can be improved by wiping the skin with a gauze, absorbent cotton, nonwoven fabric, or the like containing water or alcohol to remove the oily substance on the skin and moisturize the skin. If the skin is dry, this suppresses ion release from the skin. Moisturizing the skin and the bio-electrode promotes the ion release from the skin, and improves the sensitivity to biological signals. The absorbent cotton, nonwoven fabric, gauze, and so forth preferably contain water, or water-containing water-soluble alcohols such as ethanol, glycerin, ethylene glycol, and diethylene glycol.

As has been described above, the inventive method for manufacturing a bio-electrode makes it possible to manufacture the inventive bio-electrode easily and at low cost, with the bio-electrode being excellent in electric conductivity and biocompatibility, light-weight, and capable of preventing significant reduction in the electric conductivity even when wetted with water or dried, capable of quick signal collection after attachment to skin, leaving no residue on the skin, and being transparent and high in aesthetic feature.

### EXAMPLE

Hereinafter, the present invention will be specifically described with reference to Examples and Comparative Examples, but the present invention is not limited thereto. Incidentally, "Me" represents a methyl group, and "Vi" represents a vinyl group.

### (Ionic Monomer)

### [Polymer Compound (A)]

Ionic polymers 1 to 19, which were blended as the polymer compound (A) (ionic material (electroconductivity material)) to bio-electrode composition solutions of Examples, and Comparative ionic polymer 1, which was blended to bio-electrode composition solutions of Comparative Examples, were synthesized as follows. First, 30 mass% solutions of corresponding monomers in cyclopentanone were introduced into a reaction vessel and mixed. The inside of the reaction vessel was cooled to -70°C under a nitrogen atmosphere, and subjected to vacuum degassing and nitrogen blowing, which were repeated three times. After the temperature was raised to room temperature, azobisisobutyronitrile (AIBN) was added as a polymerization initiator in an amount of 0.01 moles per 1 mole of the whole monomers. The resultant was warmed to 60°C and then allowed to react for 15 hours. After the solvent was dried, the composition of the resulting polymer was identified by ¹H-NMR. Moreover, the molecular weight (Mw) and dispersity (Mw/Mn) of the obtained polymer were determined by gel permeation chromatography (GPC) using tetrahydrofuran (THF) as a solvent. Ionic polymers 1 to 19 and Comparative ionic polymer 1 synthesized in this manner are shown below.
Ionic polymer 1
Mw=38,100
Mw/Mn=1.91

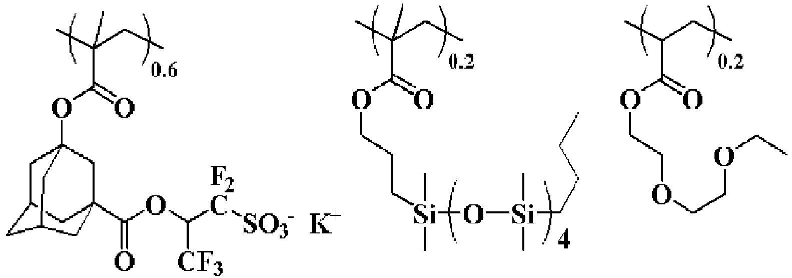

The repeating number in each formula shows the average value.
Ionic polymer 2
Mw=36,100
Mw/Mn=1.93

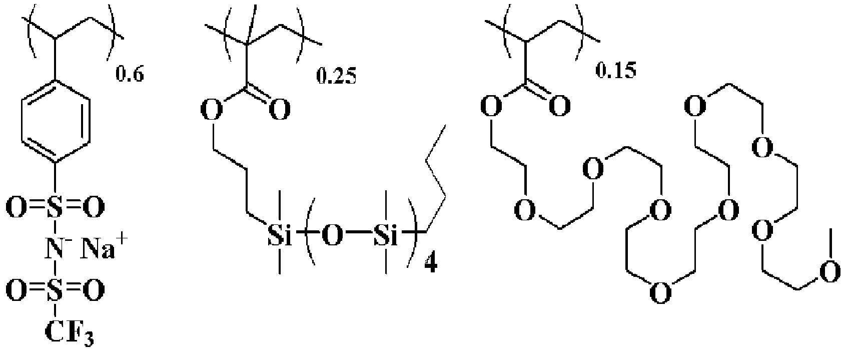

The repeating number in each formula shows the average value.
Ionic polymer 3
Mw=150,600
Mw/Mn=1.85

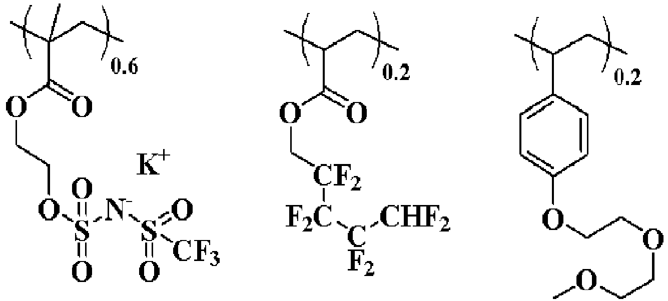

Ionic polymer 4
Mw=44,400
Mw/Mn=1.94

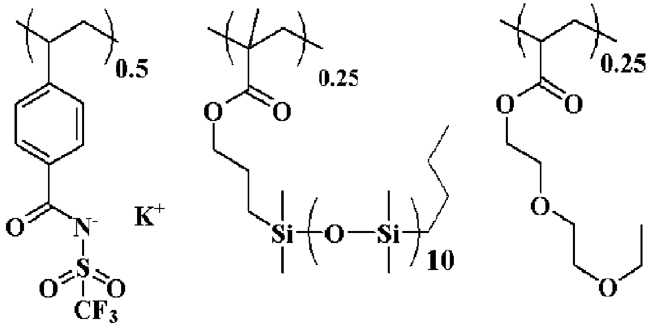

The repeating number in each formula shows the average value.
Ionic polymer 5
Mw=43,100
Mw/Mn=1.88

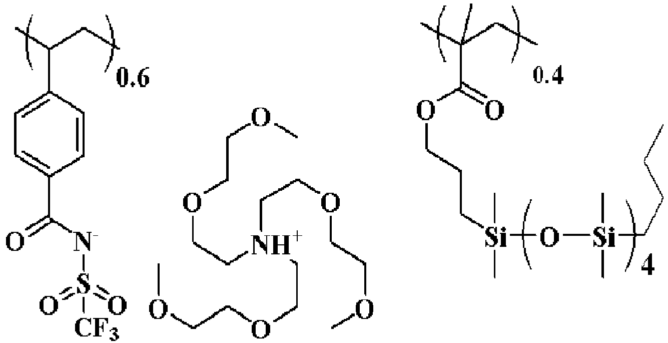

The repeating number in each formula shows the average value.
Ionic polymer 6
Mw=41,200
Mw/Mn=1.72

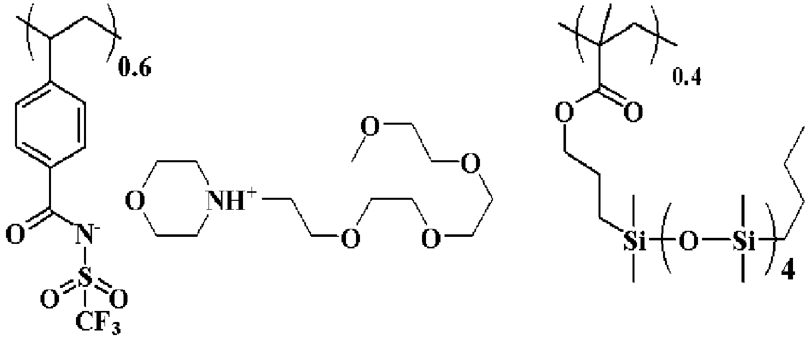

The repeating number in each formula shows the average value.
Ionic polymer 7
Mw=43,600
Mw/Mn=1.93

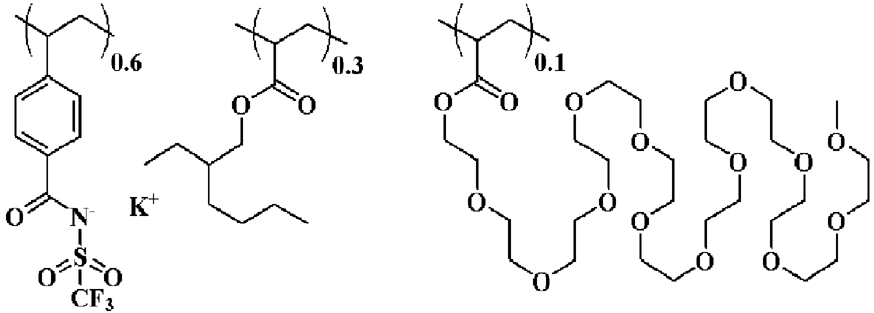

Ionic polymer 8
Mw=31,600
Mw/Mn=2.10

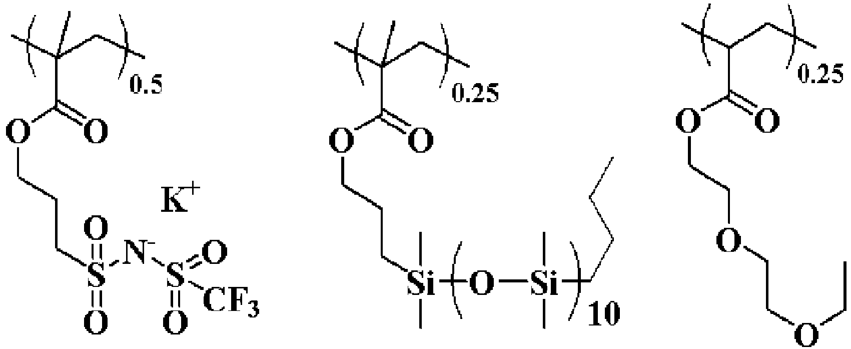

The repeating number in each formula shows the average value.
Ionic polymer 9
Mw=55,100
Mw/Mn=2.02

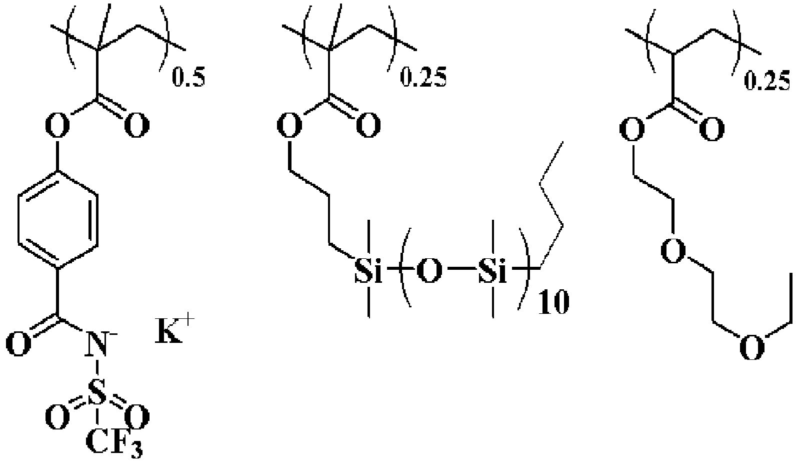

The repeating number in each formula shows the average value.
Ionic polymer 10
Mw=87,500
Mw/Mn=2.01

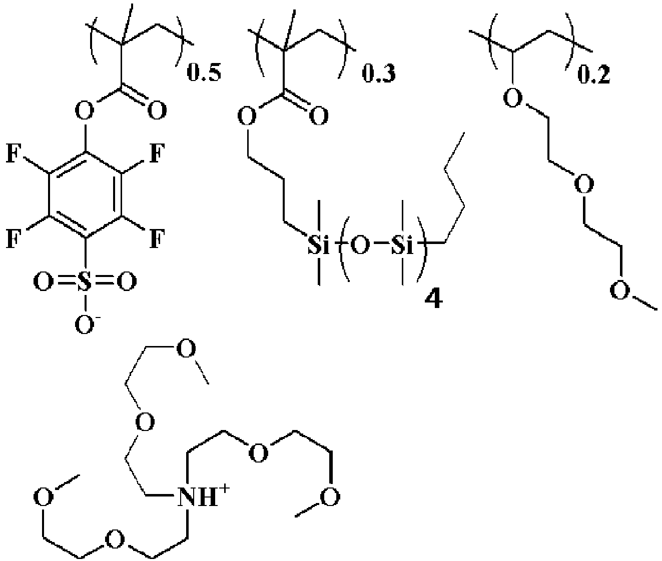

The repeating number in each formula shows the average value.
Ionic polymer 11
Mw=43,600
Mw/Mn=1.91

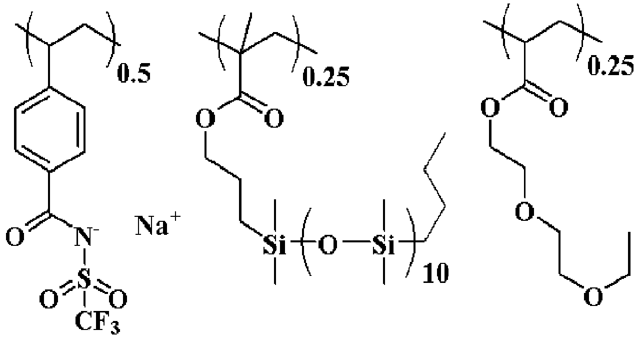

The repeating number in each formula shows the average value.
Ionic polymer 12
Mw=97,100
Mw/Mn=2.20

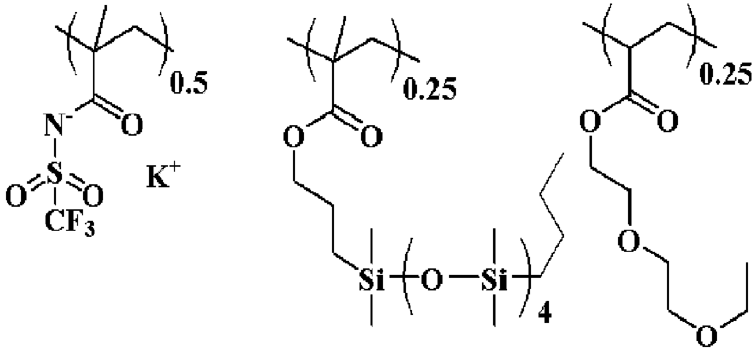

The repeating number in each formula shows the average value.
Ionic polymer 13
Mw=98,300
Mw/Mn=2.05

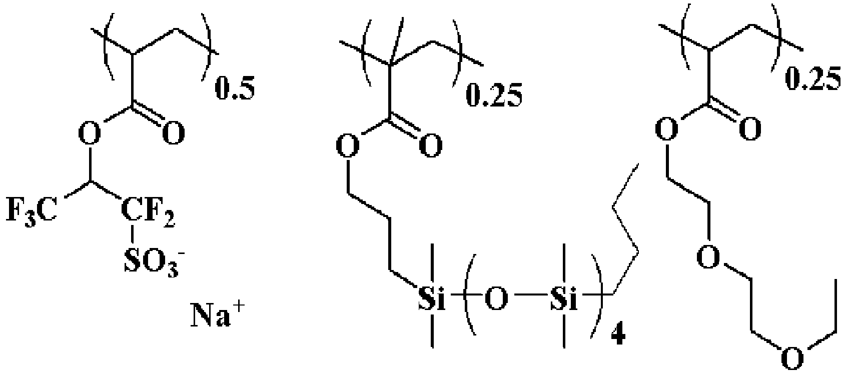

The repeating number in each formula shows the average value.
Ionic polymer 14
Mw=68,900
Mw/Mn=2.26

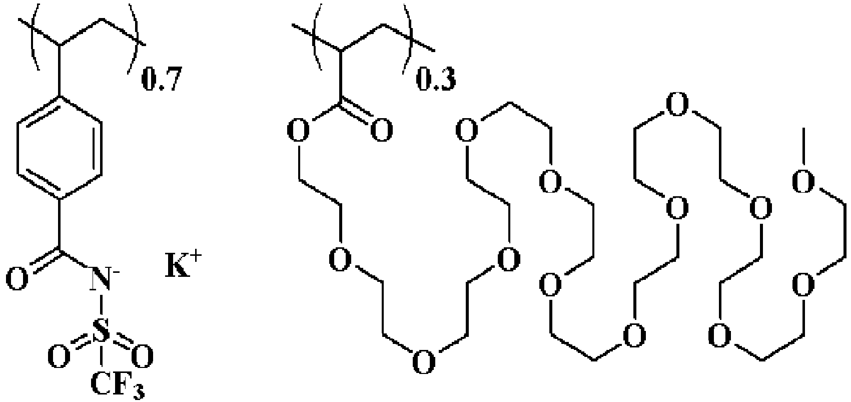

Ionic polymer 15
Mw=67,100
Mw/Mn=1.89

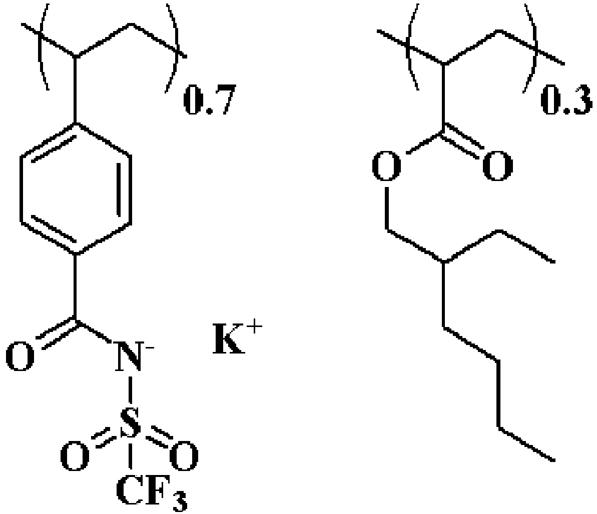

Ionic polymer 16
Mw=23,400
Mw/Mn=1.77

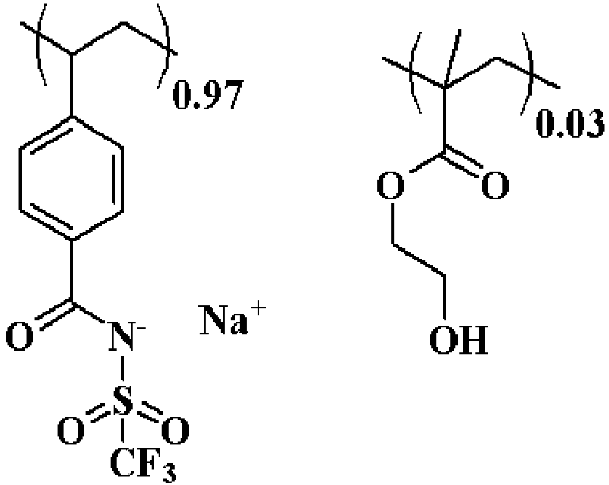

Ionic polymer 17
Mw=34,300
Mw/Mn=1.75

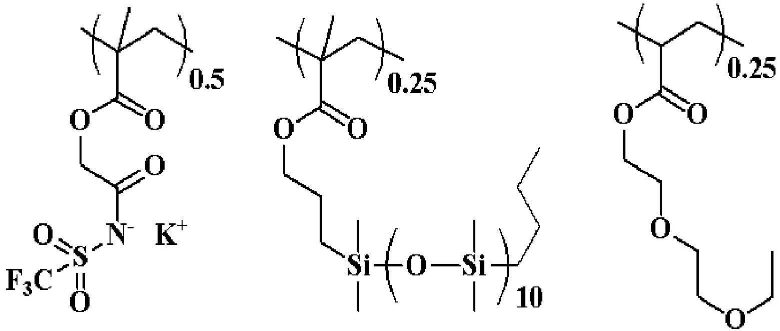

The repeating number in each formula shows the average value.
Ionic polymer 18
Mw=37,700
Mw/Mn=1.79

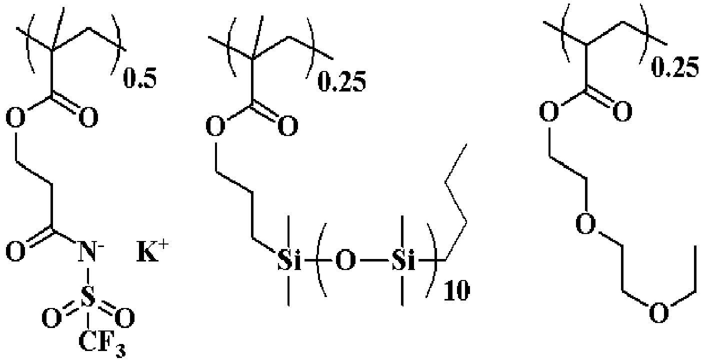

The repeating number in each formula shows the average value.
Ionic polymer 19
Mw=46,300
Mw/Mn=2.21

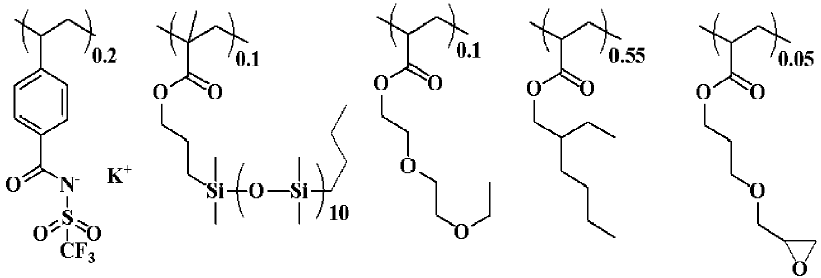

The repeating number in each formula shows the average value.
Comparative ionic polymer 1
Mw=46,700
Mw/Mn=2.25

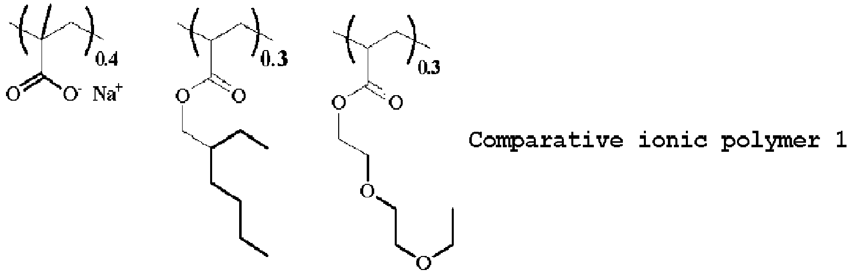

### [Filler Particles (B)]

The filler particles (B) blended in the bio-electrode composition solutions of Examples and Comparative Examples were as follows:
fumed silica with an average particle diameter of 0.007 µm manufactured by Sigma-Aldrich Co., LLC.
aluminum oxide with an average particle diameter of less than 0.050 µm manufactured by Sigma-Aldrich Co., LLC.
titanium oxide with an average particle diameter of less than 0.025 µm manufactured by Sigma-Aldrich Co., LLC.
zirconium oxide with an average particle diameter of less than 0.100 µm manufactured by Sigma-Aldrich Co., LLC.
AEROSIL 200 with an average particle diameter of 0.012 µm manufactured by Nippon Aerosil Co., Ltd.
AEROSIL RX200 with an average particle diameter of 0.012 µm manufactured by Nippon Aerosil Co., Ltd.
AEROSIL RX300 with an average particle diameter of 0.007 µm manufactured by Nippon Aerosil Co., Ltd.
AEROSIL RY300 with an average particle diameter of 0.007 µm manufactured by Nippon Aerosil Co., Ltd.

### [Compound (C) having Polyglycerin Structure]

Polyglycerin-silicone compounds 1 to 22 and Polyether-silicone compound 1, which were blended as the compound (C) having a polyglycerin structure to the bio-electrode composition solutions of Examples are shown below. As the synthesis method, these compounds were synthesized through hydrosilylation reaction of a silicone compound having a SiH group, a polyglycerin compound having a double bond, an alkylene group compound, and a polyether compound having a double bond in the presence of a platinum catalyst as described in JP 2019-99469A.

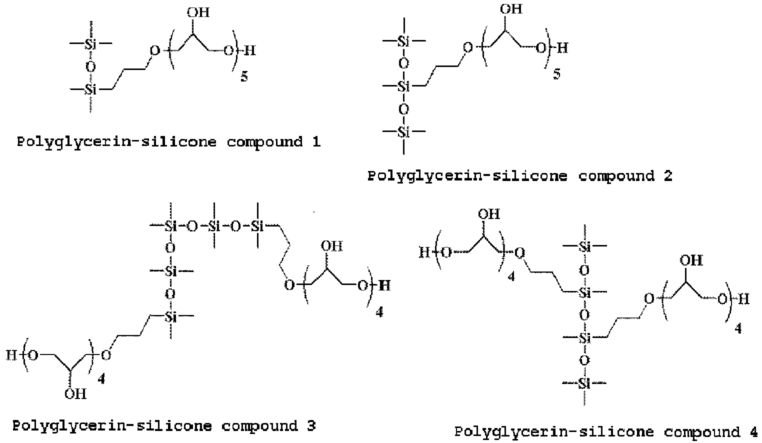

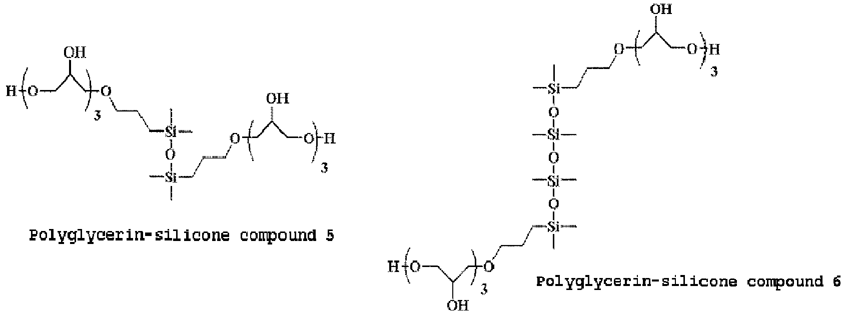

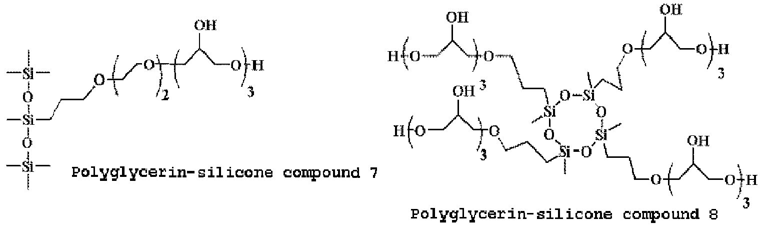

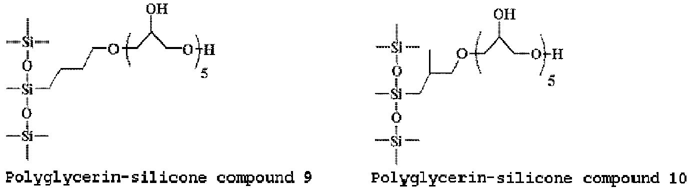

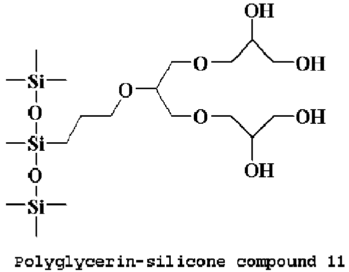

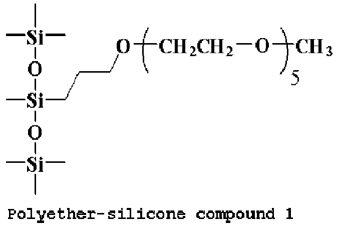

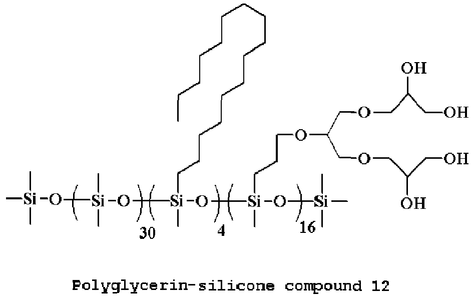

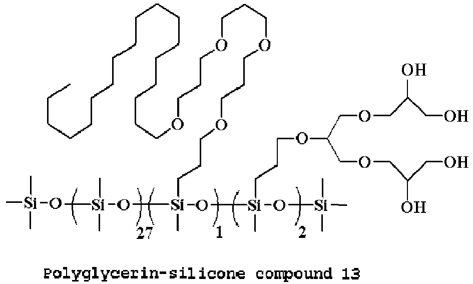

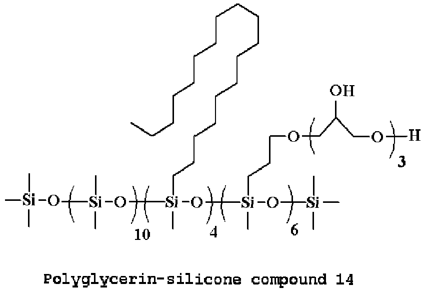

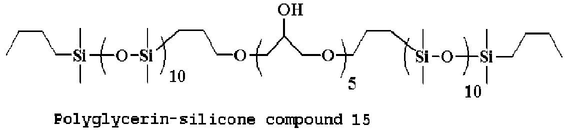

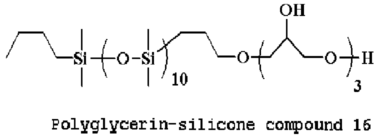

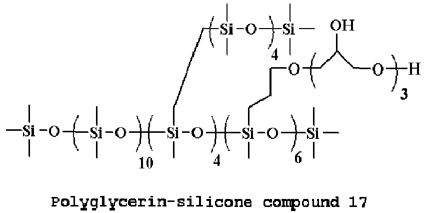

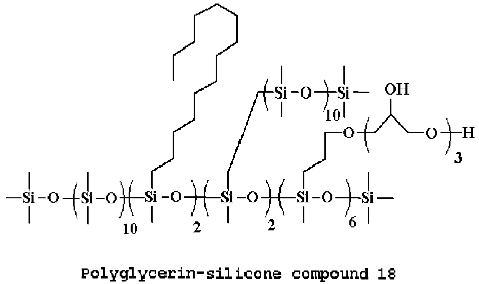

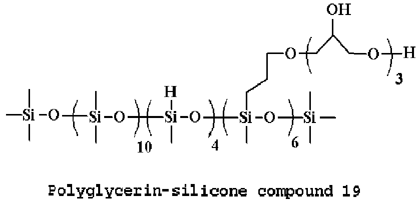

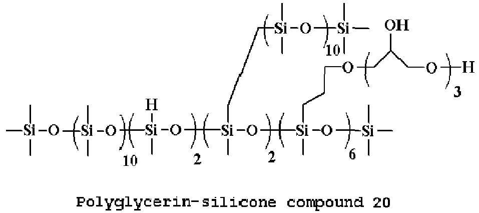

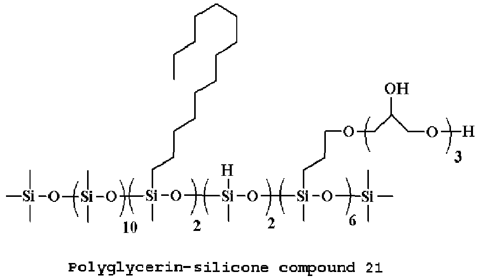

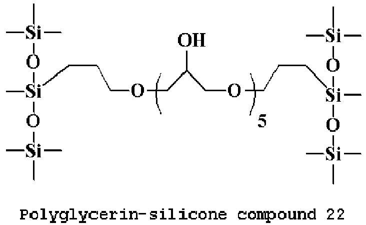

### [Resin Component (D)]

Siloxane compounds 1 to 4, which were blended as silicone-based resin to the bio-electrode composition solutions of Examples and Comparative Examples, were as follows.

### (Siloxane compound 1)

Siloxane compound 1 was vinyl group-containing polydimethylsiloxane having an alkenyl group-content of 0.007 mol/100 g in which the terminals of molecular chain were capped with SiMe₂Vi groups, with the 30% solution in toluene having a viscosity of 27,000 mPa·s.

### (Siloxane compound 2)

Siloxane compound 2 was a 60% solution of polysiloxane of MQ resin composed of an Me₃SiO_{0.5} unit and an SiO₂ unit (Me₃SiO_{0.5} unit/SiO₂ unit = 0.8) in toluene.

### (Siloxane compound 3)

Siloxane compound 3 was polydimethylsiloxanebonded MQ resin obtained by heating a solution with refluxing for 4 hours, followed by cooling. The solution was composed of 40 parts by mass of vinyl group-containing polydimethylsiloxane having an alkenyl group-content of 0.007 mol/100 g in which the terminals of molecular chain were capped with OH groups, with the 30% solution in toluene having a viscosity of 42,000 mPa·s; 100 parts by mass of 60% solution of polysiloxane of MQ resin composed of an Me₃SiO_{0.5} unit and an SiO₂ unit (Me₃SiO_{0.5} unit/SiO₂ unit = 0.8) in toluene; and 26.7 parts by mass of toluene.

### (Siloxane compound 4)

As methylhydrogensilicone oil, KF-99 manufactured by Shin-Etsu Chemical Co., Ltd. was used.

Acrylic polymer blended as acrylic-based resin to the bio-electrode composition solutions is shown below.
Acrylic polymer 1
Mw=108,000
Mw/Mn=2.32

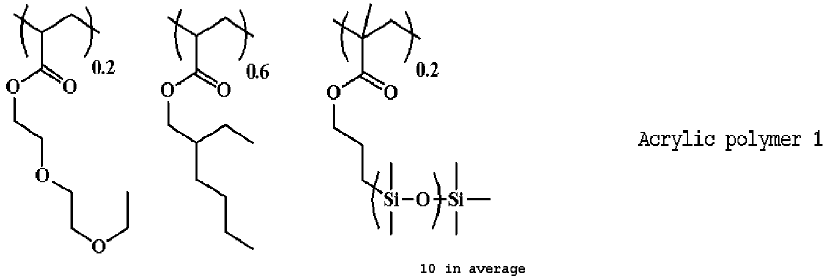

The repeating number in each formula shows the average value.

Silicone pendant urethane (meth)acrylates 1 to 3 and Urethane (meth)acrylate 4, which were blended as silicone-based, acrylic-based, or urethane-based resins to the bio-electrode composition solutions of Examples and Comparative Examples, were as follows.

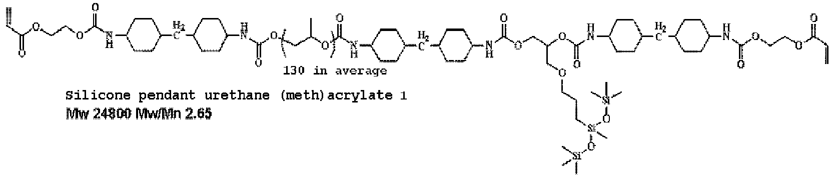

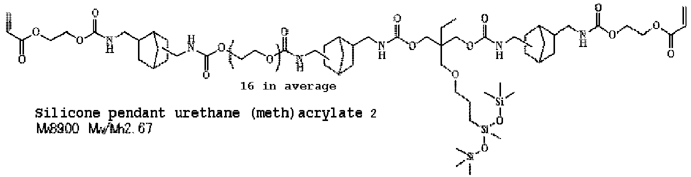

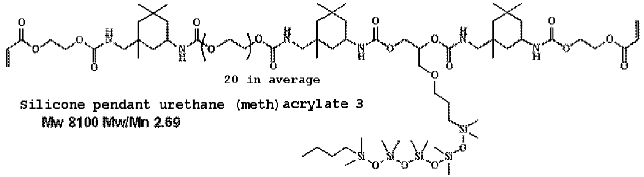

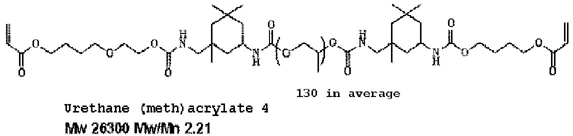

The repeating number in each formula shows the average value.

### [Crosslinking Agent]

A crosslinking agent blended to the bio-electrode composition solutions of Examples and Comparative Examples was as follows.

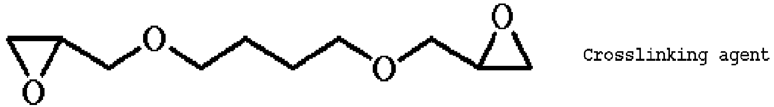

### [Organic Solvent (E)]

Organic solvents (E) blended to the bio-electrode composition solutions of Examples and Comparative Examples were as follows.
EDE: diethylene glycol diethyl ether
BE: diethylene glycol butyl ether
ISOPAR G (manufactured by Exxon Mobile Corporation): isoparaffin

### [Others]

Lithium titanate powder, silver flake, radical generator, platinum catalyst, electric conductivity improver (carbon black, multilayer carbon nanotube), which were blended as additives to the bio-electrode composition solutions of Examples and Comparative Examples, were as follows.
Lithium titanate powder (spinel): with the size of 200 nm or less manufactured by Sigma-Aldrich Co., LLC.
Silver flake: with the average size of 10 µm manufactured by Sigma-Aldrich Co., LLC.
Radical generator: IRGACURE TPO manufactured by BASF SE
Platinum catalyst: CAT-PL-56 manufactured by Shin-Etsu Chemical Co., Ltd.
Carbon black: DENKA BLACK Li-400 manufactured by Denka Co., Ltd.
Multilayer carbon nanotube: with the diameter of 110 to 170 nm and length of 5 to 9 pm manufactured by Sigma-Aldrich Co., LLC.

### [Examples 1 to 42, Comparative Examples 1 to 2]

According to the compositions shown in Tables 1 to 5, the ionic materials (salts), filler particles, polyglycerin-silicone compounds, resins, organic solvents, additives (radical generator, platinum catalyst, electric conductivity improver, a lithium titanate powder, ionic additive, platinum regulator, etc.), and crosslinking agent were blended to prepare bio-electrode composition solutions (Bio-electrode composition solutions 1 to 42, Comparative bio-electrode composition solutions 1 to 2).

**[Table 1]**

| Bio-electrode composition solution | Ionic material (parts by mass) | Filler particles (parts by mass) | Polyglycerin -silicone compound (parts by mass) | Resin (parts by mass) | Organic solvent (parts by mass) | Additive (parts by mass) |
|---|---|---|---|---|---|---|
| Bio-electrode composition solution 1 | Ionic polymer 1 (30) | fumed silica (14) | Polyglycerin -silicone compound 1(50) | Siloxane compound 1(40) Siloxane compound 2(100) Siloxane compound 4(3) | ISOPAR G(135) cyclopentanone (60) | CAT-PL-56(5) |
| Bio-electrode composition solution 2 | Ionic polymer 2 (30) | fumed silica (14) | Polyglycerin -silicone compound 2(50) | Siloxane compound 3(126) Siloxane compound 4(3) | ISOPAR G(135) cyclopentanone (60) | CAT-PL-56(5) |
| Bio-electrode composition solution 3 | Ionic polymer 3 (25) | fumed silica (14) | Polyglycerin -silicone compound 3(50) | Siloxane compound 1(40) Siloxane compound 2(100) Siloxane compound 4(3) | ISOPAR G(135) cyclopentanone (60) | CAT-PL-56(5) |
| Bio-electrode composition solution 4 | Ionic polymer 4 (30) | fumed silica (14) | Polyglycerin -silicone compound 4(50) | Siloxane compound 1(40) Siloxane compound 2(100) Siloxane compound 4(3) | ISOPAR G(60) cyclopentanone (70) | CAT-PL-56(5) lithium titanate powder (12) silver flake (8) |
| Bio-electrode composition solution 5 | Ionic polymer 5 (30) | fumed silica (14) | Polyglycerin -silicone compound 5(50) | Siloxane compound 3(126) siloxane compound 4(3) | n-octane (135) cyclopentanone (60) | CAT-PL-56(5) |
| Bio-electrode composition solution 6 | Ionic polymer 6 (30) | fumed silica (14) | Polyglycerin -silicone compound 6(50) | Siloxane compound 3(126) Siloxane compound 4(3) | n-nonane(135) 2-heptanone (60) | CAT-PL-56(5) lithium titanate powder (5) carbon black (5) |
| Bio-electrode composition solution 7 | Ionic polymer 7 (30) | fumed silica (14) | Polyglycerin -silicone compound 7(50) | Siloxane compound 3(126) Siloxane compound 4(3) | ISOPAR G(135) 2-heptanone (60) | CAT-PL-56(5) carbon black (14) |
| Bio-electrode composition solution 8 | Ionic polymer 8 (30) | fumed silica (14) | Polyglycerin -silicone compound 8(50) | Siloxane compound 3(126) Siloxane compound 4(3) | n-decane(70) n-octane(65) 2-heptanone (60) | CAT-PL-56(5) lithium titanate powder(5) carbon black(5) |
| Bio-electrode composition solution 9 | Ionic polymer 9 (30) | fumed silica (14) | Polyglycerin -silicone compound 9(50) | Siloxane compound 3(126) Siloxane compound 4(3) | ISOPAR G(135) cyclopentanone (60) | CAT-PL-56(5) lithium titanate powder (5) multilayer carbon nanotube (3) |

**[Table 2]**

| Bio-electrode composition solution | Ionic material (parts by mass) | Filler particles (parts by mass) | Polyglycerin -silicone compound (parts by mass) | Resin (parts by mass) | Organic solvent (parts by mass) | Additive (parts by mass) |
|---|---|---|---|---|---|---|
| Bio-electrode composition solution 10 | Ionic polymer 10 (40) | fumed silica (14) | Polyglycerin -silicone compound 10(50) | Silicone pendant urethane (meth)acrylate 1(80) | EDE(60) cyclopentanone (70) | IRGACURE TPO(1) |
| Bio-electrode composition solution 11 | Ionic polymer 11 (20) | fumed silica (14) | Polyglycerin -silicone compound 11(50) | Acrylic polymer 1(55) Silicone pendant urethane (meth)acrylate 1(25) | EDE(60) cyclopentanone (70) | IRGACURE TPO(1) |
| Bio-electrode composition solution 12 | Ionic polymer 12 (20) | fumed silica (14) | Polyglycerin -silicone compound 1(50) | Acrylic polymer 1(20) Silicone pendant urethane (meth) acrylate 2(60) | EDE(60) cyclopentanone (70) | IRGACURE TPO(1) |
| Bio-electrode composition solution 13 | Ionic polymer 13 (25) | fumed silica (10) | Polyglycerin -silicone compound 1(50) | Acrylic polymer 1(20) Silicone pendant urethane (meth)acrylate 3(60) | EDE(60) cyclopentanone (70) | IRGACURE TPO(1) |
| Bio-electrode composition solution 14 | Ionic polymer 14 (25) | fumed silica (10) | Polyglycerin -silicone compound 5(50) | Silicone pendant urethane (meth)acrylate 1(80) | BE(120) water (10) | IRGACURE TPO(1) sodium chloride (2) |
| Bio-electrode composition solution 15 | Ionic polymer 15 (26) | fumed silica (18) | Polyglycerin -silicone compound 5(50) | Silicone pendant urethane (meth)acrylate 1(80) | BE(120) water (10) | IRGACURE TPO(1) potassium chloride (2) |
| Bio-electrode composition solution 16 | Ionic polymer 16 (16) | fumed silica (14) | Polyglycerin -silicone compound 5(50) | Silicone pendant urethane (meth)acrylate 1(80) | BE(120) water (10) | IRGACURE TPO(1) crosslinking agent(2) |
| Bio-electrode composition solution 17 | Ionic polymer 11 (20) | fumed silica (20) | Polyglycerin -silicone compound 11(50) | Urethane (meth) acrylate 4(80) | EDE(60) cyclopentanone (70) | IRGACURE TPO(1) |

**[Table 3]**

| Bioelectrode composition solution | Ionic material (parts by mass) | Filler particles (parts by mass) | Polyglycerin -silicone compound (parts by mass) | Resin (parts by mass) | Organic solvent (parts by mass) | Additive (parts by mass) |
|---|---|---|---|---|---|---|
| Bio-electrode composition solution 18 | Ionic polymer 1 (30) | fumed silica (10) | Polyglycerin -silicone compound 12(40) | Siloxane compound 1(40) Siloxane compound 2(100) Siloxane compound 4(3) | ISOPAR G(135) cyclopentanone (60) | CAT-PL-56(5) |
| Bio-electrode composition solution 19 | Ionic polymer 1 (30) | fumed silica (10) | Polyglycerin -silicone compound 13(40) | Siloxane compound 1(40) Siloxane compound 2(100) Siloxane compound 4(3) | ISOPAR G(135) cyclopentanone (60) | CAT-PL-56(5) ethynylcyc lohexanol (5) |
| Bio-electrode composition solution 20 | Ionic polymer 1 (30) | fumed silica (18) | Polyglycerin -silicone compound 14(40) | Siloxane compound 1(40) Siloxane compound 2(100) Siloxane compound 4(3) | ISOPAR G(135) cyclopentanone (60) | CAT-PL-56(5) carbon black (14) ethynylcyc lohexanol (5) |
| Bio-electrode composition solution 21 | Ionic polymer 1 (30) | fumed silica (20) | Polyglycerin -silicone compound 15(50) | Siloxane compound 1(40) Siloxane compound 2(100) Siloxane compound 4(3) | ISOPAR G(135) cyclopentanone (60) | CAT-PL-56(5) ethynylcyc lohexanol (5) |
| Bio-electrode composition solution 22 | Ionic polymer 1 (30) | fumed silica (20) | Polyglycerin -silicone compound 16(50) | Siloxane compound 1(40) Siloxane compound 2(100) Siloxane compound 4(3) | ISOPAR G(135) cyclopentanone (60) | CAT-PL-56(5) |
| Bio-electrode composition solution 23 | Ionic polymer 1 (30) | AEROSIL 200 (14) | Polyglycerin -silicone compound 17(50) | Siloxane compound 1(40) Siloxane compound 2(100) Siloxane compound 4(3) | ISOPAR G(135) cyclopentanone (60) | CAT-PL-56(5) |
| Bio-electrode composition solution 24 | Ionic polymer 1 (30) | aluminum oxide (14) | Polyglycerin -silicone compound 18(50) | Siloxane compound 1(40) Siloxane compound 2(100) Siloxane compound 4(3) | ISOPAR G(135) cyclopentanone (60) | CAT-PL-56(5) |
| Bio-electrode composition solution 25 | Ionic polymer 17 (30) | titanium oxide (14) | Polyglycerin -silicone compound 19(50) | Siloxane compound 1(40) Siloxane compound 2(100) Siloxane compound 4(3) | ISOPAR G(135) cyclopentanone (60) | CAT-PL-56(5) |
| Bio-electrode composition solution 26 | Ionic polymer 18 (30) | zirconium oxide (14) | Polyglycerin -silicone compound 20(50) | Siloxane compound 1(40) Siloxane compound 2(100) Siloxane compound 4(3) | ISOPAR G(135) cyclopentanone (60) | CAT-PL-56(5) |
| Bio-electrode composition solution 27 | Ionic polymer 19 (30) | fumed silica (14) | Polyglycerin -silicone compound 19(50) | - | cyclopentanone (150) | |
| Bio-electrode composition solution 28 | Ionic polymer 1 (30) | fumed silica (20) | Polyglycerin -silicone compound 20(50) | Siloxane compound 1(40) Siloxane compound 2(100) | ISOPAR G(135) cyclopentanone (60) | CAT-PL-56(5) |
| Bio-electrode composition solution 29 | Ionic polymer 1 (30) | fumed silica (10) | Polyglycerin -silicone compound 21(50) | Siloxane compound 1(40) Siloxane compound 2(100) | ISOPAR G(135) cyclopentanone (60) | CAT-PL-56(5) ethynylcyc lohexanol (5) |
| Bio-electrode composition solution 30 | Ionic polymer 1 (30) | fumed silica (20) | Polyglycerin -silicone compound 22 (50) | Siloxane compound 1(40) Siloxane compound 2(100) Siloxane compound 4(2) | ISOPAR G(135) cyclopentanone (60) | CAT-PL-56(5) ethynylcyc lohexanol (5) |

**[Table 4]**

| Bio-electrode composition solution | Ionic material (parts by mass) | Filler particles (parts by mass) | Polyglycerin-silicone compound (parts by mass) | Resin (parts by mass) | Organic solvent (parts by mass) | Additive (parts by mass) |
|---|---|---|---|---|---|---|
| Bio-electrode composition solution 31 | Ionic polymer 1 (30) | fumed silica (10) | - | Siloxane compound 1(40) Siloxane compound 2(100) Siloxane compound 4(3) | ISOPAR G(135) cyclopentanone (60) | CAT-PL-56(5) |
| Bio-electrode composition solution 32 | Ionic polymer 2 (30) | fumed silica (16) | - | Siloxane compound 3(126) Siloxane compound 4(3) | ISOPAR G(135) cyclopentanone (60) | CAT-PL-56(5) ethynylcyclohex anol(5) |
| Bio-electrode composition solution 33 | Ionic polymer 3 (30) | fumed silica (10) | - | Siloxane compound 1(40) Siloxane compound 2(100) Siloxane compound 4(3) | ISOPAR G(60) cyclopentanone (70) | CAT-PL-56(5) lithium titanate powder (12) silver flake(8) |
| Bio-electrode composition solution 34 | Ionic polymer 4 (30) | fumed silica (14) | - | Siloxane compound 3(126) Siloxane compound 4(3) | n-octane (135) cyclopentanone (60) | CAT-PL-56(5) |
| Bio-electrode composition solution 35 | Ionic polymer 5 (30) | fumed silica (18) | - | Siloxane compound 3(126) Siloxane compound 4(3) | n-nonane (135) 2-heptanone (60) | CAT-PL-56(5) lithium titanate powder (5) carbon black (5) |
| Bio-electrode composition solution 36 | Ionic polymer 6 (30) | fumed silica (20) | - | Siloxane compound 3(126) Siloxane compound 4(3) | ISOPAR G(135) 2-heptanone (60) | CAT-PL-56(5) carbon black (14) |
| Bio-electrode composition solution 37 | Ionic polymer 7 (30) | fumed silica (14) | - | Siloxane compound 3(126) Siloxane compound 4(3) | n-decane (70) n-octane (65) 2-heptanone (60) | CAT-PL-56(5) lithium titanate powder (5) carbon black(5) ethynylcyclohex anol (5) |
| Bio-electrode composition solution 38 | Ionic polymer 8 (30) | fumed silica (14) | - | Siloxane compound 3(126) Siloxane compound 4(3) | ISOPAR G(135) cyclopentanone (60) | CAT-PL-56(5) lithium titanate powder (5) multilayer carbon nanotube(3) |
| Bio-electrode composition solution 39 | Ionic polymer 1 (30) | fumed silica (14) | Polyether-silicone compound 1(50) | Siloxane compound 1(40) Siloxane compound 2(100) Siloxane compound 4(3) | ISOPAR G(135) cyclopentanone (60) | CAT-PL-56(5) |
| Bio-electrode composition solution 40 | Ionic polymer 1 (30) | AEROSIL RX200 (14) | Polyglycerin-silicone compound 17(50) | Siloxane compound 1(40) Siloxane compound 2(100) Siloxane compound 4(3) | ISOPAR G(135) cyclopentanone (60) | CAT-PL-56(5) |
| Bio-electrode composition solution 41 | Ionic polymer 1 (30) | AEROSIL RX300 (14) | Polyglycerin-silicone compound 17(50) | Siloxane compound 1(40) Siloxane compound 2(100) Siloxane compound 4(3) | ISOPAR G(135) cyclopentanone (60) | CAT-PL-56(5) |
| Bio-electrode composition solution 42 | Ionic polymer 1 (30) | AEROSIL RY300 (14) | Polyglycerin-silicone compound 17(50) | Siloxane compound 1(40) Siloxane compound 2(100) Siloxane compound 4(3) | ISOPAR G(135) cyclopentanone (60) | CAT-PL-56(5) |

**[Table 5]**

| Bio-electrode composition solution | Ionic material (parts by mass) | Filler particles (parts by mass) | Polyglycerin -silicone compound (parts by mass) | Resin (parts by mass) | Organic solvent (parts by mass) | Additive (parts by mass) |
|---|---|---|---|---|---|---|
| Comparative bio-electrode composition solution 1 | Ionic polymer 1 (30) | | Polyglycerin -silicone compound 1(50) | Siloxane compound 1(40) Siloxane compound 2(100) Siloxane compound 4(3) | ISOPAR G(135) cyclopentanone (60) | CAT-PL-56(5) carbon black (14) |
| Comparative bio-electrode composition solution 2 | Comparative ionic polymer 1 (30) | fumed silica (14) | Polyglycerin -silicone compound 1(50) | Siloxane compound 1(40) Siloxane compound 2(100) Siloxane compound 4(3) | ISOPAR G(135) cyclopentanone (60) | CAT-PL-56(5) |

### (Preparation of Samples for Biological Signal Evaluation)

A thermoplastic urethane (TPU) film ST-604 (manufactured by Bemis Associates Inc.) was coated with an electro-conductive paste DOTITE FA-333 (manufactured by Fujikura Kasei Co., Ltd.) by screen printing. The coating film was baked in an oven at 120°C for 10 minutes to print a keyhole-shaped electro-conductive pattern including a circular portion with a diameter of 2 cm and a rectangular portion. Then, one of the bio-electrode composition solutions shown in Tables 1 to 5 was applied onto the circular portion of the printed electro-conductive pattern by screen printing. After air-dried at room temperature for 10 minutes, the coating film was baked using an oven at 125°C for 10 minutes to evaporate the solvent. In Examples 10 to 17, the curing was further continued by irradiation with a xenon lamp at a radiant exposure level of 200 mJ/cm² under a nitrogen atmosphere. By the curing, cured products of the bio-electrode compositions were obtained as living body contact layers (Bio-electrodes 1 to 42, Comparative bio-electrodes 1 to 2).

FIG. 3 is a schematic view of the printed bio-electrodes prepared in each Example. As shown in FIG. 3, multiple bio-electrodes 1 were prepared on the thermoplastic urethane film 20. The bio-electrodes 1 each include a keyhole-shaped electro-conductive pattern 2 as the electro-conductive base material, and a living body contact layer 3 formed to cover the circular portion of the electro-conductive pattern 2.

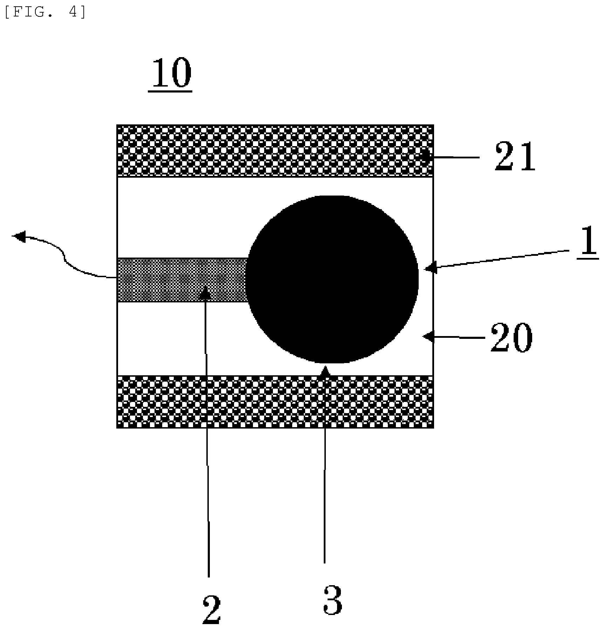

Next, as shown in FIG. 4, the thermoplastic urethane film 20 having the bio-electrode 1 printed thereon was cut out and pasted on a double-sided tape 21. In this manner, three bio-electrode samples 10 (samples for biological signal evaluation) were prepared for each of the composition solutions.

### (Thickness Measurement of Living Body Contact Layer)

The thickness of the living body contact layer of each bio-electrode sample prepared as described above was measured with a micrometer. Table 6 shows the result.

### (Biological Signal Measurement)

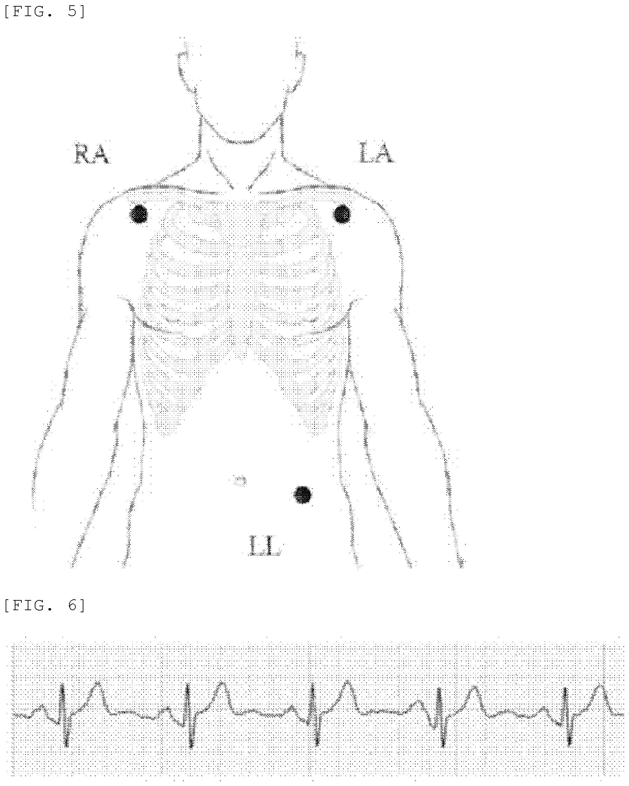

The electro-conductive wiring pattern formed from the electro-conductive paste of each bio-electrode sample was connected to a portable electrocardiograph HCG-901 (manufactured by OMRON HEALTHCARE Co., Ltd.) through an electro-conductive wire. A positive electrode of the electrocardiograph was attached to the location LA in FIG. 5 on a human body, a negative electrode was attached to the location LL, and an earth was attached to the location RA. Immediately after the attachments, the electrocardiogram measurement was started to measure the time until an electrocardiogram waveform including P, Q, R, S, and T waves appeared as shown in FIG. 6. Table 6 shows the result. Additionally, immediately before the attachments, the electrode surface or the skin may be wiped with a gauze impregnated with a solution containing 70% ethanol and 30% water.

### (Observation of Residue on Skin)

After the electrocardiogram waveform measurement, the attached electrodes were continuously left on the skin. One hour later, the electrodes were peeled from the skin, and whether or not a residue remained on the skin was checked. Table 6 shows the result.

**[Table 6]**

| Example | Bio-electrode | Resin thickness (µm) | Time (min.) until ECG signal appeared | Presence/absence of residue on skin after ECG measurement |
|---|---|---|---|---|
| Example 1 | Bio-electrode 1 | 20 | 0 | absent |
| Example 2 | Bio-electrode 2 | 18 | 0 | absent |
| Example 3 | Bio-electrode 3 | 22 | 0 | absent |
| Example 4 | Bio-electrode 4 | 28 | 2 | absent |
| Example 5 | Bio-electrode 5 | 27 | 0 | absent |
| Example 6 | Bio-electrode 6 | 31 | 1.5 | absent |
| Example 7 | Bio-electrode 7 | 33 | 2 | absent |
| Example 8 | Bio-electrode 8 | 29 | 1.5 | absent |
| Example 9 | Bio-electrode 9 | 28 | 1.5 | absent |
| Example 10 | Bio-electrode 10 | 31 | 0 | absent |
| Example 11 | Bio-electrode 11 | 41 | 0 | absent |
| Example 12 | Bio-electrode 12 | 33 | 0 | absent |
| Example 13 | Bio-electrode 13 | 25 | 0 | absent |
| Example 14 | Bio-electrode 14 | 21 | 0.5 | absent |
| Example 15 | Bio-electrode 15 | 26 | 1 | absent |
| Example 16 | Bio-electrode 16 | 20 | 1 | absent |
| Example 17 | Bio-electrode 17 | 23 | 0.5 | absent |
| Example 18 | Bio-electrode 18 | 26 | 0 | absent |
| Example 19 | Bio-electrode 19 | 20 | 0 | absent |
| Example 20 | Bio-electrode 20 | 18 | 0 | absent |
| Example 21 | Bio-electrode 21 | 22 | 0.5 | absent |
| Example 22 | Bio-electrode 22 | 28 | 0.5 | absent |
| Example 23 | Bio-electrode 23 | 27 | 0 | absent |
| Example 24 | Bio-electrode 24 | 31 | 0 | absent |
| Example 25 | Bio-electrode 25 | 33 | 0 | absent |
| Example 26 | Bio-electrode 26 | 29 | 0 | absent |
| Example 27 | Bio-electrode 27 | 28 | 0 | absent |
| Example 28 | Bio-electrode 28 | 31 | 0 | absent |
| Example 29 | Bio-electrode 29 | 41 | 0 | absent |
| Example 30 | Bio-electrode 30 | 33 | 0 | absent |
| Example 31 | Bio-electrode 31 | 25 | 30 | absent |
| Example 32 | Bio-electrode 32 | 21 | 30 | absent |
| Example 33 | Bio-electrode 33 | 26 | 30 | absent |
| Example 34 | Bio-electrode 34 | 20 | 30 | absent |
| Example 35 | Bio-electrode 35 | 23 | 30 | absent |
| Example 36 | Bio-electrode 36 | 26 | 30 | absent |
| Example 37 | Bio-electrode 37 | 25 | 30 | absent |
| Example 38 | Bio-electrode 38 | 21 | 30 | absent |
| Example 39 | Bio-electrode 39 | 22 | 20 | absent |
| Example 40 | Bio-electrode 40 | 25 | 0 | absent |
| Example 41 | Bio-electrode 41 | 23 | 0 | absent |
| Example 42 | Bio-electrode 42 | 29 | 0 | absent |
| Comparative Example 1 | Comparative bio-electrode 1 | 20 | 0.5 | present |
| Comparative Example 2 | Comparative bio-electrode 2 | 21 | 60 | absent |

As shown in Table 6, biological signals (ECG signals) were detected immediately after the attachment to the body, and no residue remained on the skin in Examples 1 to 30 and Examples 40 to 42, in each of which the living body contact layer was formed using the inventive bio-electrode composition containing: the polymer compound (A) (salt (ionic material)) having a particular structure described above, one or more kinds of the filler particles (B) selected from the group consisting of silica particles, alumina particles, titania particles, and zirconia particles, and the compound (C) having a polyglycerin structure. Meanwhile, it took longer time to detect biological signals (ECG signals), but no residue remained on the skin in Examples 31 to 38, in each of which the living body contact layer was formed using the inventive bio-electrode composition containing: the polymer compound (A) (salt (ionic material)) having a particular structure and one or more kinds of the filler particles (B) selected from the group consisting of silica particles, alumina particles, titania particles, and zirconia particles. The living body contact layer of Example 39 contained the polymer compound (A), the filler particles (B), and the compound having a polyether structure. As a result, adding the compound a having polyether structure slightly shortened the time to detect a biological signal, but the effect as high as those obtained by adding the compound (C) having a polyglycerin structure was not observed. This indicates that although both of polyether structure and polyglycerin structure have hydrophilicity, polyglycerin having hydroxyl groups are more effective in the system of the present invention. On the other hand, the living body contact layer of Comparative Example 1 contained the polymer compound (A) and the compound (C) having a polyglycerin structure, but did not contain the filler particles (B). As a result, it took short time for the living body contact layer of Comparative Example 1 to detect a biological signal, but the living body contact layer caused a residue on the skin when peeled from the skin. This is conceivably due to poor affinity among the polymer compound (A), the compound (C) having a polyglycerin structure, and the carbon black because the filler particles (B) were not incorporated. Meanwhile, the living body contact layer of Comparative Example 2 contained the filler particles (B), but did not contain the polymer compound (A). As a result, it took longer time to detect a biological signal conceivably because the living body contact layer of Comparative Example 2 was not able to exhibit sufficient ion sensitivity.

The above results revealed that the bio-electrode including the living body contact layer formed from the inventive bio-electrode composition is capable of quickly detecting biological signals. More specifically, since the inventive bio-electrode composition contains the polymer compound (A) and one or more kinds of the filler particles (B) selected from the group consisting of silica particles, alumina particles, titania particles, and zirconia particles as described above, the inventive bio-electrode composition makes it possible to provides a bio-electrode which is capable of quickly detecting biological signals after attachment to a body, and which leaves no residue on the skin.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A bio-electrode composition comprising:
a polymer compound (A), which comprises a repeating unit-a having a structure selected from the group consisting of salts of ammonium, sodium, potassium, and silver formed with any of fluorosulfonic acid, fluorosulfonimide, and N-carbonyl-fluorosulfonamide; and
filler particles (B), which are one or more selected from the group consisting of silica particles, alumina particles, titania particles, and zirconia particles, optionally
wherein the repeating unit-a has a structure shown by any of the following general formulae (1)-1 to (1)-4, 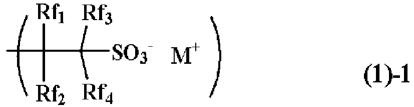 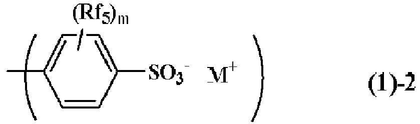 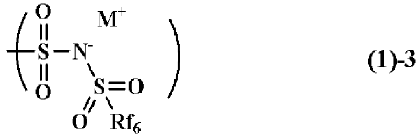 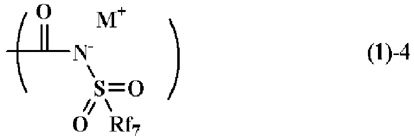
wherein Rf₁ and Rf₂ each represent a hydrogen atom, a fluorine atom, an oxygen atom, a methyl group, or a trifluoromethyl group, provided that when Rf₁ and Rf₂ represent an oxygen atom, the single oxygen atom represented by Rf₁ and Rf₂ bonds to a single carbon atom to form a carbonyl group; Rf₃ and Rf₄ each represent a hydrogen atom, a fluorine atom, or a trifluoromethyl group, provided that at least one of Rf₁ to Rf₄ is a fluorine atom or a trifluoromethyl group; Rf₅, Rf₆, and Rf₇ each represent a fluorine atom, or a linear or branched alkyl group having 1 to 4 carbon atoms, and have at least one fluorine atom; M⁺ represents an ion selected from the group consisting of an ammonium ion, a sodium ion, a potassium ion, and a silver ion; and "m" represents an integer of 1 to 4.

2. The bio-electrode composition according to claim 1, wherein the repeating unit-a comprises at least one repeating unit selected from the group consisting of repeating units-al to -a7 shown by the following general formula (2), 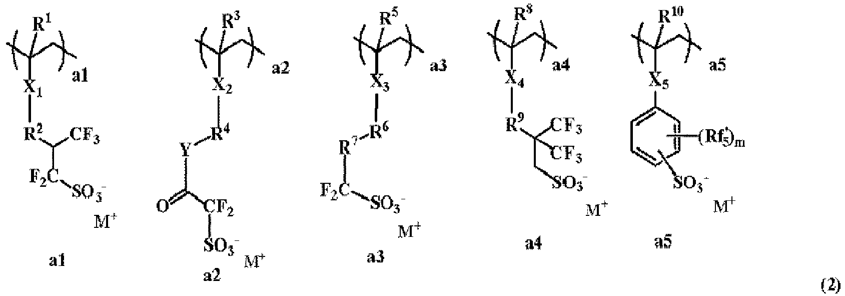 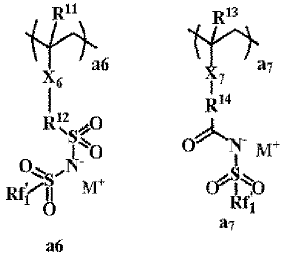 wherein R¹, R³, R⁵, R⁸, R¹⁰, R¹¹, and R¹³ each independently represent a hydrogen atom or a methyl group; R², R⁴, R⁶, R⁹, R¹², and R¹⁴ each independently represent a single bond, or a linear, branched, or cyclic hydrocarbon group having 1 to 13 carbon atoms, the hydrocarbon group optionally having either or both of an ester group and an ether group; R⁷ represents a linear or branched alkylene group having 1 to 4 carbon atoms, and one or two hydrogen atoms in R⁷ are optionally substituted with a fluorine atom; X₁, X₂, X₃, X₄, X₆, and X₇ each independently represent any of a single bond, a phenylene group, a naphthylene group, an ether group, an ester group, and an amide group; X₅ represents any of a single bond, an ether group, and an ester group; Y represents an oxygen atom or a -NR¹⁹- group; R¹⁹ represents a hydrogen atom, or a linear or branched alkyl group having 1 to 4 carbon atoms, and optionally forms a ring together with R⁴; Rf₁' and Rf₅' each represent a fluorine atom, a trifluoromethyl group, or a linear or branched alkyl group having 1 to 4 carbon atoms, and have at least one fluorine atom; "m" represents an integer of 1 to 4; a1, a2, a3, a4, a5, a6, and a7 satisfy 0≤a1≤1.0, 0≤a2≤1.0, 0≤a3≤1.0, 0≤a4≤1.0, 0≤a5≤1.0, 0≤a6≤1.0, 0≤a7≤1.0, and 0<a1+a2+a3+a4+a5+a6+a7≤1.0; and M⁺ represents an ion selected from the group consisting of an ammonium ion, a sodium ion, a potassium ion, and a silver ion.

3. The bio-electrode composition according to claim 1 or 2, wherein the polymer compound (A) comprises an ammonium ion shown by the following general formula (3) as an ammonium ion for forming the ammonium salts, 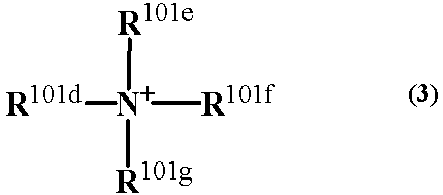 wherein R^{101d}, R^{101e}, R^{101f}, and R^{101g} each represent a hydrogen atom, a linear, branched, or cyclic alkyl group having 1 to 14 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and R^{101d} and R^{101e}, or R^{101d}, R^{101e}, and R^{101f}, are optionally bonded to each other together with a nitrogen atom bonded therewith to form a ring in which R^{101d} and R^{101e}, or R^{101d}, R^{101e}, and R^{101f}, represent an alkylene group having 3 to 10 carbon atoms, or to form a heteroaromatic ring having the nitrogen atom in the general formula (3) within the ring.

4. The bio-electrode composition according to any one of claims 1 to 3, further comprising a compound (C) having a polyglycerin structure.

5. The bio-electrode composition according to claim 4, wherein the compound (C) having a polyglycerin structure is a silicone compound having a polyglycerin structure.

6. The bio-electrode composition according to claim 5, wherein the silicone compound having a polyglycerin structure is shown by any of the following general formulae (4) and (5), 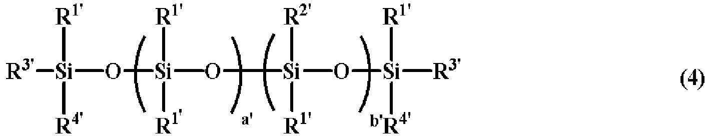 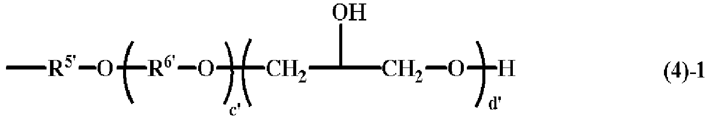 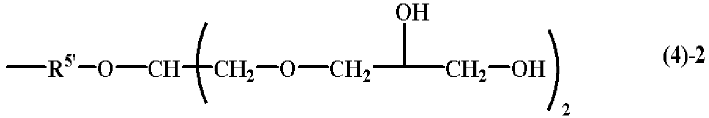 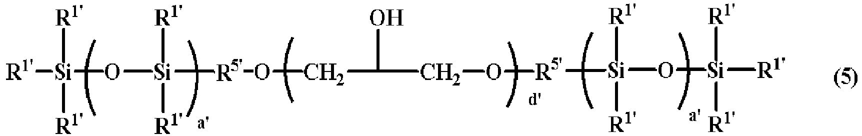 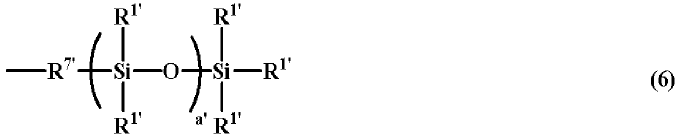 wherein each R^{1'} is identical to or different from one another, and independently represents a hydrogen atom, a phenyl group, a linear or branched alkyl group having 1 to 50 carbon atoms, or a silicone chain shown by a general formula (6), and optionally contains an ether group; R^{2'} represents a group having a polyglycerin group structure shown by a general formula (4)-1 or (4)-2; each R^{3'} is identical to or different from the other, and independently represents the R^{1'} group or the R^{2'} group; each R^{4'} is identical to or different from the other, and independently represents the R^{1'} group, the R^{2'} group, or an oxygen atom, provided that when R^{4'} represents an oxygen atom, the two R^{4'} moieties bond to each other and optionally constitute an ether group to form a ring together with silicon atoms; each a' is identical to or different from one another and represents 0 to 100, b' represents 0 to 100, and a'+b' is 0 to 200, provided that when b' is 0, at least one R^{3'} is the R^{2'} group; R^{5'} represents an alkylene group having 2 to 10 carbon atoms or an aralkylene group having 7 to 10 carbon atoms; R^{6'} represents an alkylene group having 2 to 6 carbon atoms; R^{7'} represents an alkylene group having 2 to 6 carbon atoms or an ether group; c' represents 0 to 20; and d' represents 1 to 20.

7. The bio-electrode composition according to any one of claims 1 to 6, further comprising a resin component (D), which is one or more selected from the group consisting of a silicone-based resin, an acrylic-based resin, and a urethane-based resin, optionally
wherein
the resin component (D) comprises any of:
a silicone resin having an SiO₂ unit and an RₓSiO_{(4-x)/2} unit, wherein R represents a substituted or unsubstituted monovalent hydrocarbon group having 1 to 10 carbon atoms, and "x" represents a number in a range of 2.5 to 3.5;
diorganosiloxane having an alkenyl group; and
organohydrogenpolysiloxane having an SiH group.

8. The bio-electrode composition according to any one of claims 1 to 7, further comprising an organic solvent (E).

9. The bio-electrode composition according to any one of claims 1 to 8, further comprising a component (F), which is a carbon powder, a silver powder, a silicon powder, and/or a lithium titanate powder.

10. The bio-electrode composition according to claim 9, wherein the carbon powder is one or both of carbon black and carbon nanotube.

11. A bio-electrode comprising an electro-conductive base material and a living body contact layer formed on the electro-conductive base material, wherein
the living body contact layer comprises a cured product of the bio-electrode composition according to any one of claims 1 to 10, optionally
wherein the electro-conductive base material comprises one or more selected from the group consisting of gold, silver, silver chloride, platinum, aluminum, magnesium, tin, tungsten, iron, copper, nickel, stainless steel, chromium, titanium, and carbon.

12. The bio-electrode according to claim 11, wherein the living body contact layer is humidified.

13. A method for manufacturing a bio-electrode having an electro-conductive base material and a living body contact layer formed on the electro-conductive base material, comprising:
applying the bio-electrode composition according to any one of claims 1 to 10 onto the electro-conductive base material; and
curing the bio-electrode composition to form the living body contact layer.

14. The method for manufacturing a bio-electrode according to claim 13, wherein the electro-conductive base material comprises one or more selected from the group consisting of gold, silver, silver chloride, platinum, aluminum, magnesium, tin, tungsten, iron, copper, nickel, stainless steel, chromium, titanium, and carbon.

15. The method for manufacturing a bio-electrode according to claim 13 or 14, wherein after the living body contact layer is formed, the living body contact layer is immersed in water, or the living body contact layer is humidified.
